# EUROPEAN PATENT SPECIFICATION

(11) **EP 3 841 876 B1**
(45) Date of publication and mention of the grant of the patent: **14.10.2026**
(21) Application number: 20196265.1
(22) Date of filing: 14.03.2014
(51) Int. Cl.: A01K 67/0278, C12N 15/85, C07K 16/00

(54) **TRANSGENIC MOUSE FOR ANTIBODY PRODUCTION**
TRANSGENE MAUS FÜR DIE PRODUKTION VON ANTIKÖRPERN
SOURIS TRANSGÉNIQUE DESTINÉ À LA PRODUCTION D'ANTICORPS

(30) Priority: 14.03.2013 US 201361851974 P
(43) Date of publication of application: 30.06.2021
(62) Document number(s) of the earlier application(s) in accordance with Art. 76 EPC: 14715432.2 / 2 967 012
(73) Proprietor: Erasmus University Medical Center Rotterdam, 3000 CA Rotterdam (NL); Craig, Roger Kingdon, Jubilee House Farm, Spen Moss, Smallwood Sandbach, Cheshire CW11 2XB (GB)
(72) Inventor: CRAIG, Roger Kingdon, Sandbach, Cheshire CW11 2XB (GB); GROSVELD, Franklin Gerardus, 3000 CA Rotterdam (NL); JANSSENS, Richard Wilhelm, 3000 CA Rotterdam (NL); VAN HAPEREN, Marinus Johannes, 3000 CA Rotterdam (NL); DE BOER, Ernie, 3000 CA Rotterdam (NL)
(74) Representative: Carpmaels & Ransford LLP

(56) References cited:
- WO-A1-2010/070263
- WO-A1-2011/158009
- WO-A1-2012/122512
- WO-A1-2012/141798
- M. J. OSBORN ET AL: "High-Affinity IgG Antibodies Develop Naturally in Ig-Knockout Rats Carrying Germline Human IgH/Ig /Ig Loci Bearing the Rat CH Region", THE JOURNAL OF IMMUNOLOGY, vol. 190, no. 4, 15 February 2013 (2013-02-15), pages 1481 - 1490, XP055063917, ISSN: 0022-1767, DOI: 10.4049/jimmunol.1203041

## Description

### FIELD OF THE INVENTION

The present invention relates to improved methods for the derivation and selection using transgenic non-human mammals of a diverse repertoire of functional, affinitymatured tetrameric immunoglobulins comprising heavy and light chains in response to antigen challenge and uses thereof.

In the following description, all amino acid residue position numbers are given according to the numbering system devised by Kabat et al. (1991) US Public Health Services publication No 91-3242.

### BACKGROUND OF THE INVENTION

### Antibodies

The structure of antibodies is well known in the art. Most natural antibodies are tetrameric, comprising two heavy chains and two light chains. The heavy chains are joined to each other via disulphide bonds between hinge domains located approximately halfway along each heavy chain. A light chain is associated with each heavy chain on the N-terminal side of the hinge domain. Each light chain is normally bound to its respective heavy chain by a disulphide bond close to the hinge domain.

When an antibody molecule is correctly folded, each chain folds into a number of distinct globular domains joined by more linear polypeptide sequences. For example, the light chain folds into a variable (V_{L}) and a constant (C_{κ} or C_{λ}) domain. Heavy chains have a single variable domain V_{H}, a first constant domain (C_{H}1), a hinge domain and two or three further constant domains. The heavy chain constant domains and the hinge domain together form what is generally known as the constant region of an antibody heavy chain. Interaction of the heavy (V_{L}) and light (V_{L}) chain variable domains results in the formation of an antigen binding region (Fv). Interaction of the heavy and light chains is facilitated by the C_{H}1 domain of the heavy chain and the C_{κ} or Cλ domain of the light chain. Generally, both V_{H} and V_{L} are required for antigen binding, although heavy chain dimers and amino-terminal fragments have been shown to retain activity in the absence of light chain (Jaton et al. (1968) Biochemistry, 7, 4185-4195). Generally the proportion of circulating λ light chain is low, representing perhaps 2-5% of the total light chain complexed as a tetrameric immunoglobulin in plasma (Goldsby et al. (2003) Immunology, 5th edition, W.H. Freeman & Co NY).

The *in vitro* manipulation of heavy chain immunoglobulin genes to construct novel antibodies was first described in the 1980s. Much of the early antibody engineering work used a rearranged mouse immunoglobulin µ gene (IgM) raised against a wellcharacterised antigen. A feature of this antibody was that antigen binding specificity was known to reside in the V_{H} domains, since assembly and secretion with an irrelevant light chain showed retention of antigen binding (Neuberger and Williams (1986) Phil. Trans. R. Soc. Lond., A317, 425-432). Using this system, it was shown that a mouse antigen-specific V_{H} binding domain could be used to derive a novel antibody comprising a human ε constant effector region fused to a mouse antigen-specific V_{H} domain. The resulting hybrid IgE retained antigen specificity and showed effector activity expected of an IgE (Neuberger et al. (1985) Nature, 314, 268-270). Other literature examples of heavy chain engineering include: hybrid mouse-human antibody genes encoding mouse V_{H} human / IgA or IgG antibody fusions which retain anti-phosphocholine activity (Morrison et al. (1984) PNAS, 81, 6851-6855); an anticarcinoma-associated antigen 17-1A antibody comprising mouse V_{H} and human IgG (y3) constant region (Sun et al. (1987) PNAS, 84, 214-218); and an anti-human T-cell antibody (anti CD7) comprising human IgG (γ1) constant region and mouse V_{H} domains (see Heinrich et al. (1989) J. Immunol., 143, 3589-97).

Normal human B cells contain a single immunoglobulin heavy chain locus on chromosome 14 from which the gene encoding a heavy chain is produced by rearrangement. In the mouse, the heavy chain locus is located on chromosome 12. A normal heavy chain locus comprises a plurality of V gene segments, a number of D gene segments and a number of J gene segments. Most of a V_{H} domain is encoded by a V gene segment, but the C terminal end of each V_{H} domain is encoded by a D gene segment and a J gene segment. VDJ rearrangement in B-cells, followed by affinity maturation, provides each V_{H} domain with its antigen-binding specificity. Sequence analysis of normal H₂L₂ tetramers derived from a heavy chain immunoglobulin comprising a single V segment demonstrates that diversity in response to antigen challenge results primarily from a combination of VDJ rearrangement and somatic hypermutation (Xu and Davies (2000) Immunity, 13, 37-45). There are over 50 human V gene segments present in the human genome of which only 39 are functional. In normal diploid antibody-producing B-cells, each cell produces an antibody tetramer (H₂L₂) from a single set of heavy and light chain antibody loci. The other set of loci are not used productively as the result of a process called allelic exclusion (Singh et al. (2003) J. Exp. Med., 197, 743-750 and references therein).

Fully human antibodies (H₂L₂) can now be derived from transgenic mice in response to antigen challenge. Such transgenic mice generally comprise a single human heavy chain immunoglobulin locus and a separate human light chain immunoglobulin locus. The corresponding endogenous mouse heavy chain, kappa light chain and, optionally, lambda light chain loci coding sequences are deleted or partially deleted. Thus, only human antibodies comprising a kappa light chain are produced in a low background of mouse/human antibodies comprising a human heavy chain and a mouse lambda light chain (WO90/04036; WO93/12227; WO98/24893; US5877397, US5814318 and US6162963). The deletion of segments of all endogenous murine heavy and light chain immunoglobulin genes to eliminate endogenous heavy and light chain gene expression completely has been achieved but remains technically demanding, particularly if the elimination of all lambda light chain coding sequence is deemed necessary. Elimination of the murine lambda light chain coding sequence has been achieved through the complete deletion of all functional V and J gene segments and the C1, C2 and C3 constant regions of the lambda locus, resulting in a mouse with a silenced lambda light chain locus (see EP1399559).

A different approach is to limit mouse B-cell development and immunoglobulin secretion by disruption of membrane exons of the gene encoding the murine heavy chain gene. Thus, whilst the endogenous murine heavy chain gene is functional, in that it is transcribed and undergoes VDJ rearrangement in response to antigen challenge, since the IgM is never expressed on the cell surface of pre-B cells, further development is arrested, resulting in a non-productive response to antigen challenge (Kitamura et al. (1991) Nature, 350, 423-426), even though both endogenous mouse kappa and lambda light chain genes remain structurally intact and functional (Tuaillon (2000) Molecular Immunology, 37, 221-231).

Where endogenous mouse heavy chain and light chain gene loci remain functional, any additional introduced immunoglobulin heavy chain transgene is also regulated by allelic exclusion, so that some B-cells functionally express mouse heavy and light chain loci only and others human heavy chain loci only and mouse light chain loci (Nussenzweig et al. (1987) Science, 236, 816-819). In any single non-human transgenic animal, there is a highly diverse population of B-cells expressing antibodies derived from potentially all immunoglobulin loci in response to disparate antigen challenge. The subsequent selection of antigen-specific antibodies using established hybridoma technology using HAT selection (Davis et al. (1982) J. Immunol. Methods, 50, 161-171) does not distinguish between hybridomas expressing one as opposed to another heavy chain immunoglobulin locus.

Regulatory elements present in immunoglobulin heavy chain transgenes comprise essential tissue-specific enhancer elements to ensure B-cell specific expression in a copy number dependent manner. The presence of a 5' intronic enhancer and the 3' Locus Control Region ("LCR") ensures that transgenes are active at all stages of B-cell maturation (Guglielmi et al. (2003) Biochim. Biophys. Acta, 1642, 181-190). The inclusion of heavy and light chain specific LCRs in the transgene loci ensures not only that expression is B-cell specific, but that expression occurs irrespective of the site of integration into the genome (WO90/10077, Mills et al. (1997) J. Exp. Med., 186, 845-858 and Pettersson et al. (1997) Immunobiol., 198, 236-248)). Thus, provided an LCR is present, every transgene is functional irrespective of its position in the genome. In the event that the LCR present on the transgene is partially deleted, the chromatin surrounding the transgene is only partially open to transcription at any point in time, leading to positional effect mosaic expression, and so limited levels of expression of the transgene across the target tissue (Festenstein et al. (1996) Science, 23, 271 (5252):1123-5; Milot et al. (1996) Cell, 87(1), 105-14)

An alternative approach for the production of human immunoglobulins in a mouse background is to replace murine immunoglobulin gene segments with the homologous gene segments from humans. Thus, if only the mouse V, D and J gene segments are replaced by human homologues, a functional mouse/human hybrid antibody comprising human V_{H} and V_{L} domains and mouse constant (effector) regions will result following antigen challenge (WO94/04667). If all murine gene segments are replaced by human homologues, then an entirely human immunoglobulin will result following antigen challenge (US6596541). One perceived advantage of this approach is that, provided only coding regions are exchanged, then the resultant transgene retains all mouse regulatory elements, so ensuring maximal response to antigen challenge. This approach provides high serum titres of high affinity human antibodies or mouse/human hybrid antibodies depending on the final configuration of the transgenes. In reality, however, the replacement of all the individual V, D and J segments in the mouse genome by homologous recombination is a long and arduous task. Similarly, the construction of a heavy chain transgene comprising all 39 functional human V, D and J segments with constant (effector) regions is technically very demanding.

WO 2010/070263 describes non-human transgenic animals expressing humanised antibodies and use thereof. Osborn et al. (2013) J. Immunol. 190(4):1481-1490 reports that high-affinity IgG antibodies develop naturally in Ig-knockout rats carrying germline human IgH/Igκ/Igλ loci bearing the rat C_{H} region. WO 2012/122512 describes recombinant production of mixtures of single chain antibodies. WO 2012/141798 describes ADAM6 mice. WO 2011/158009 describes animal models and therapeutic molecules.

Therefore, there remains a need in the art for methods not dependent on the deletion of large segments of genomic DNA, or multiple deletions, which allow for (i) simplified and reproducible methods for the construction and B-cell-specific expression, of transgenic immunoglobulin loci, and whose functional expression is antigen dependent and ultimately determined by allelic exclusion; and (ii) the ability to select for hybridomas or B-cells and their derivatives (Babcook JS et al PNAS, 1996 Jul 23;93(15):7843-8.) expressing and secreting assembled immunoglobulin tetramers comprising the full V segment repertoire present on the heavy chain transgenic loci, or alternatively to select for cells which express a subset of V gene segments present on one as opposed to another heavy chain transgene locus.

### SUMMARY OF THE INVENTION

The invention provides a transgenic mouse comprising:
(a) an immunoglobulin heavy chain locus that comprises the sequence of SEQ ID NO:1 and a cognate Locus Control Region (LCR), and
(b) a heterologous immunoglobulin kappa light chain locus comprising human Vκ gene segments, human J gene segments, and a rat constant region gene segment, and a cognate LCR, wherein:
   the human Vκ gene segments consist of, in the following order 5' to 3', Vκ2 30, Vκ2 28, Vκ1-5, Vκ1-9, Vκ1-27, Vκ1-33, Vκ1-39, Vκ3-20, Vκ3-15, Vκ3-11, and Vκ41,
   the human Jκ gene segments comprise all five human Jκ gene segments,
   and the constant region gene segment Cκ, and/or
   (b)(ii) a heterologous immunoglobulin lambda light chain locus comprising four human Vλ gene segments, two human J gene segments, and two rat constant region gene segments, and a cognate LCR, wherein:
      the human Vλ gene segments comprise, in the following order 5' to 3', Vλ3-19, Vλ3-1, Vλ2-8, and Vλ1-51,
      the human J gene segments comprise Jλ1 and Jλ3,
      and the constant region gene segments comprise Cλ2 and Cλ3,
   and wherein the endogenous mouse heavy chain locus and the endogenous mouse kappa light chain locus have been disabled.

In some embodiments, the transgenic mouse comprises a transgene that comprises a heterologous light chain gene locus that further comprises a dominant selective marker gene.

If desired, the transgenic mouse may comprise two or more transgenes comprising two or more different heterologous heavy chain gene loci and associated B-cell specific regulatory elements.

In the transgenic mouse the or each transgene may comprise a heterologous heavy chain gene locus which comprises a dominant selective marker gene.

Preferably, the transgenic mouse comprises a transgene comprising a heterologous kappa light chain gene locus and a transgene comprising one or more heterologous heavy chain loci.

Alternatively, the transgenic mouse may comprise a transgene comprising a heterologous lambda light chain gene locus and a transgene comprising one or more heterologous heavy chain loci.

In a further alternative, the transgenic mouse may comprise a transgene comprising a heterologous kappa light chain gene locus, a transgene comprising a lambda light chain gene locus and a transgene comprising one or more heterologous heavy chain gene loci.

The invention thus provides a transgenic mouse comprising:
i) an immunoglobulin heavy chain locus that comprises the sequence of SEQ ID NO:1 and a cognate Locus Control Region (LCR); and
ii) a heterologous immunoglobulin kappa light chain locus comprising human Vκ gene segments, human Jκ gene segments, and a rat constant region gene segment, and a cognate LCR, wherein the human Vκ gene segments comprise, in the following order 5' to 3', Vκ2-30, Vκ2-28, V_{κ}1-5, Vκ1-9, Vκ1-27, Vκ1-33, V_{κ}1-39, V_{κ}3-20, Vκ3-15, Vκ3-11, and Vκ4-1, the human Jκ gene segments comprise all five human Jκ gene segments, and the constant region gene segment comprises Cκ; and/or a heterologous immunoglobulin lambda light chain locus comprising four human Vλ gene segments, two human J gene segments, and two rat constant region gene segments, and a cognate LCR, wherein the human Vλ gene segments comprise, in the following order 5' to 3', Vλ3-1, Vλ3-19, Vλ2-8, and Vλ1-51, the human J gene segments comprise Jλ1 and Jλ3, and the constant region gene segments comprise Cλ2 and Cλ3.

Each heterologous locus is preferably a human locus.

However, each heterologous locus may be a hybrid locus comprising variable regions and constant regions derived from more than one species, such as a hybrid locus comprising human variable regions and rat or murine constant regions.

The transgenic mouse may comprise groups of transgenes comprising different groups of different heterologous heavy chain gene loci, wherein each group of transgenes comprises a different dominant selective marker gene.

Alternatively, the transgenic mouse may comprise transgenes comprising heterologous light chain loci and transgenes comprising heterologous heavy chain loci, wherein transgenes comprising heterologous light chain loci and transgenes comprising heterologous heavy chain loci each comprise a different dominant selective marker gene.

In some embodiments, the transgenic mouse comprises:
i) an immunoglobulin heavy chain locus comprising the sequence of SEQ ID NO: 1, and
ii) an immunoglobulin lambda light chain locus comprising the sequence of SEQ ID NO:3.

In the invention, the endogenous mouse heavy chain locus and the endogenous mouse kappa light chain locus have been disabled.

According to a second aspect, the present invention provides a method of producing an antigen-specific heterologous hybrid monoclonal antibody comprising:
(a) immunising a transgenic mouse as defined in any of claims 1-4 with the antigen;
(b) preparing hybridomas or B cells, plasmablasts, memory B-cells or plasma cells each of which produces a monoclonal antibody from the B-cells of the immunised transgenic mouse;
(c) optionally selecting at least one hybridoma or B cell, plasmablast, memory B-cell or plasma cell expressing the heterologous antibody by use of the dominant selective marker genes present in the transgenes comprising the heterologous immunoglobulin light chain and heavy chain loci; and
(d) selecting at least one hybridoma or B cell, plasmablast, memory B-cell or plasma cell which produces an antibody which binds specifically to the antigen.

According to a further aspect of the present invention, there is provided a method of deriving a human antibody from a hybrid antibody comprising:
(a) carrying out the method as described above;
(b) selecting at least one hybridoma or B cell, plasmablast, memory B-cell or plasma cell which produces an antibody which binds specifically to the antigen and comprises V_{H} and V_{L} binding domains of the species of choice;
(c) cloning and sequencing the V_{H} and V_{L} domains;
(d) recloning selected sequences comprising the V_{H} and V_{L} binding domain coding sequences with constant effectors domains of choice from the same species; and
(e) co-expressing the recloned sequences encoding heavy and light chain polypeptides of the desired species using an expression vector in a cell type of choice.

Other methods for obtaining monoclonal antibodies can be employed. See, for example, Cheung et al., Nat Biotechnol., 2012 Mar 25;30(5):447-52, doi: 10.1038/nbt.2167; Pasqualini, R. & Arap, W. Hybridoma-free generation of monoclonal antibodies. Proc. Natl. Acad. Sci. USA 101, 257-259 (2004); Reddy, S.T. et al. Monoclonal antibodies isolated without screening by analyzing the variablegene repertoire of plasma cells. Nat. Biotechnol. 28, 965-969 (2010); and Steenbakkers PG, van Meel FC, Olijve W.J Immunol Methods. 1992 Jul 31;152(1):69-77.

### BRIEF DESCRIPTION OF THE DRAWINGS

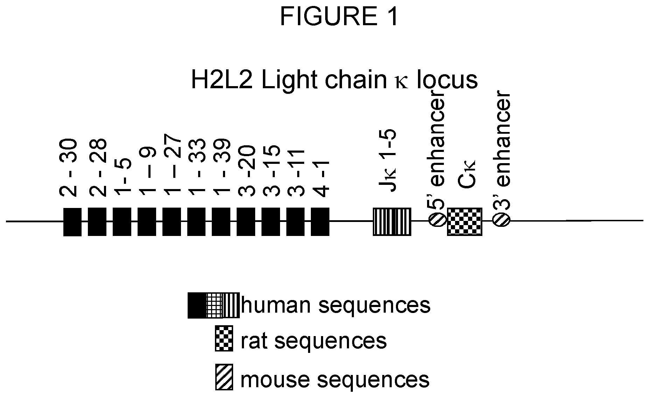

**Figure 1** depicts the composition of a hybrid human/rat Ig_{κ} locus.
   The 3' end of the locus was obtained from the mouse (diagonal stripes pattern) containing the mouse κ 3' enhancer (diagonal stripes pattern). The mouse constant coding sequences were replaced with those of the rat, including its 5' enhancer obtained by long range polymerase chain reaction ("PCR") from rat genomic DNA (checkerboard pattern). The human J_{κ} segments were obtained from a plasmid artificial chromosome ("PAC") covering this part of the human locus (vertical stripes pattern). The black boxes are V_{κ} segments added individually or as a block. The resulting locus contains all of the frequently and moderately frequently used human V_{κ} segments (black boxes).

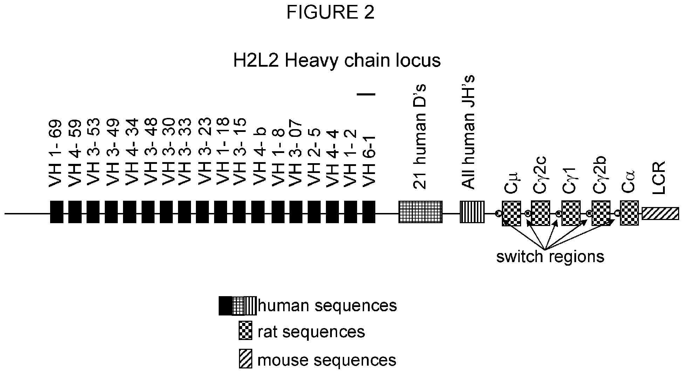

**Figure 2** depicts the composition of a hybrid human/rat IgH locus.
   The locus was generated by ligating V_{H} regions together to a concatemer of 17 consecutive human V_{H} regions (black boxes) cloned between SceI sites. A mouse spacer region was added to a human 40 kb fragment containing 21 human D_{H} (grid pattern boxes) and all six J_{H} segments (vertical stripes pattern boxes) to keep the appropriate distance between the D_{H} and V_{H} segments. This was followed by the addition of a V_{H}6-1 segment containing an SceI site. The concatemer was then added onto the V_{H}6-1. Finally, the various rat constant regions (checkerboard pattern boxes), including switch regions, and the murine LCR (diagonal stripes pattern box) were added at the 3'side. The resulting locus contains all of the frequently and moderately frequently used human V_{H} segments.

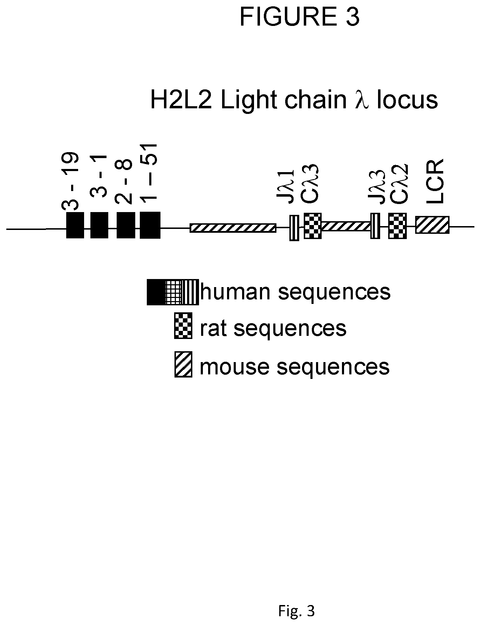

**Figure 3** depicts the composition of a hybrid human/rat Igλ locus.
   The 3' end of the locus was obtained from the mouse (diagonal stripes pattern) containing the mouse λ 3' LCR (diagonal stripes pattern). The mouse constant coding sequences were replaced with those of the rat by long range PCR from rat genomic DNA (checkerboard pattern). The segment downstream from Vλ1-51 through to the human Jλ sequences were obtained from the mouse (diagonal stripes pattern) to maintain the proper spacing between the V and J regions. The human J_{λ} segments were obtained by long range PCR of a human PAC covering this part of the human locus (vertical stripes pattern boxes). The black boxes are Vλ segments added individually or as a block.

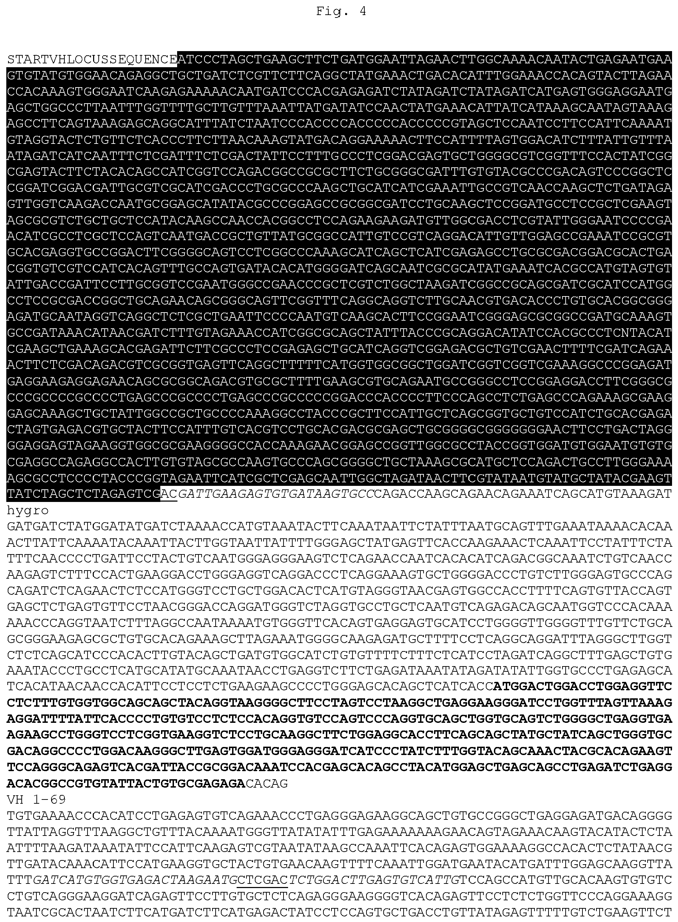

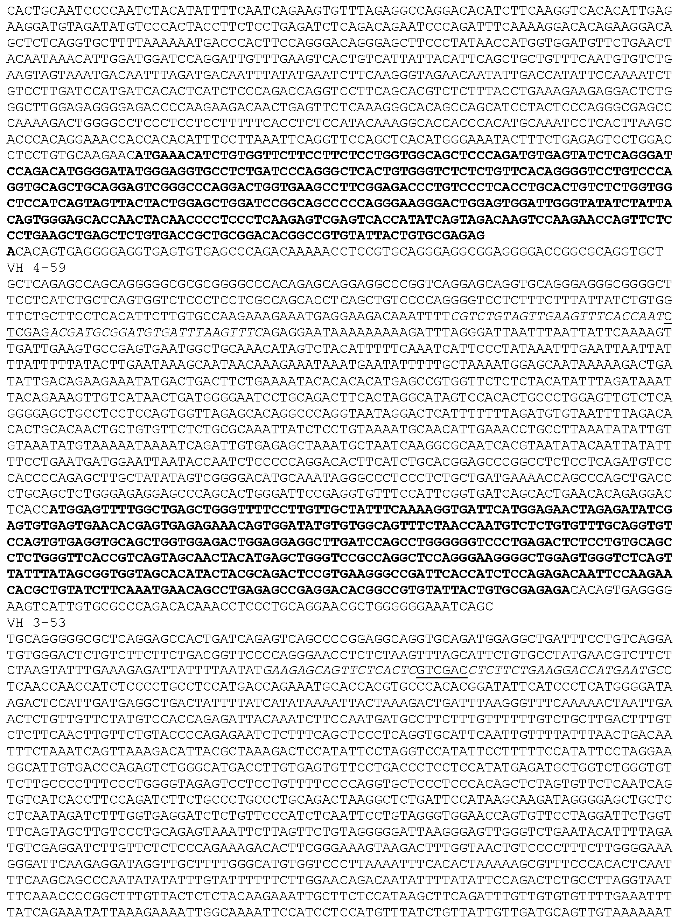

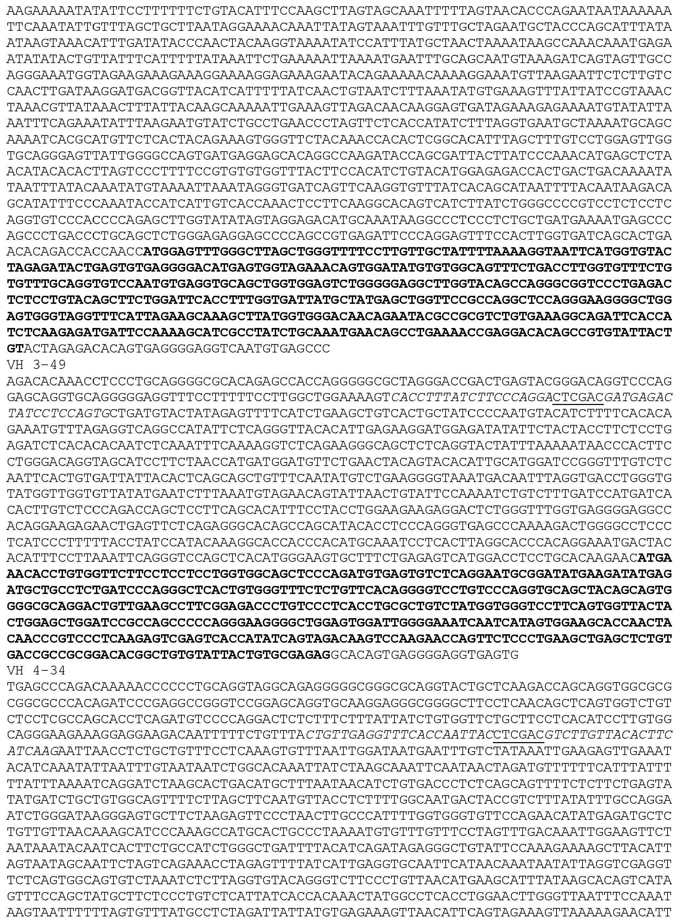

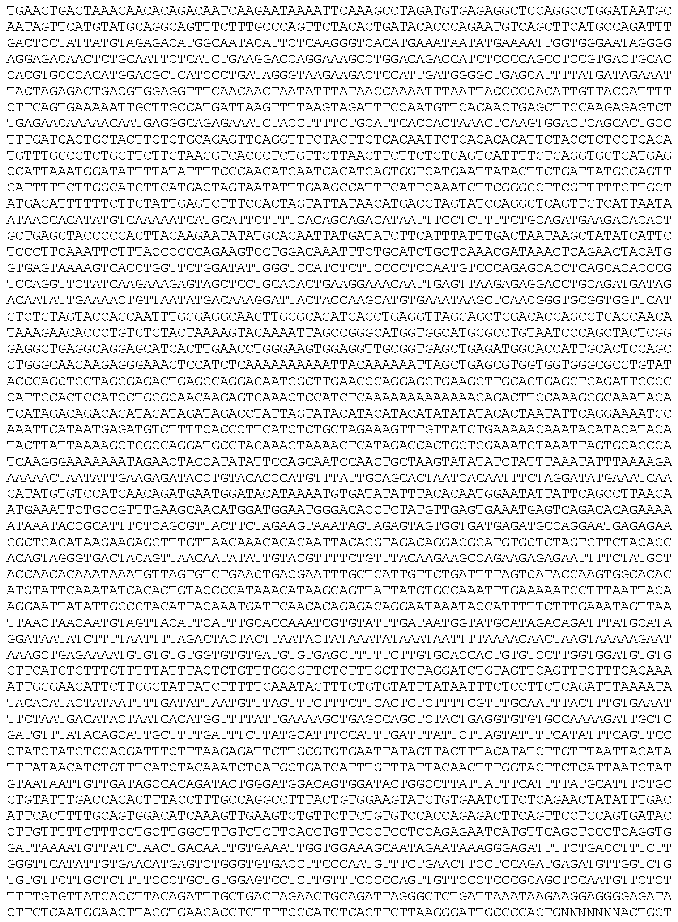

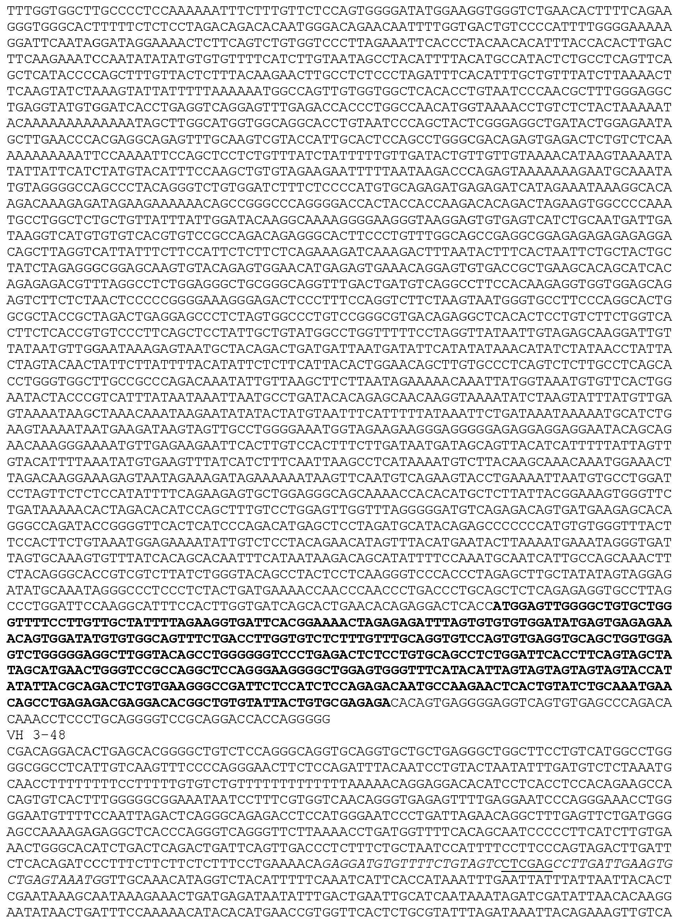

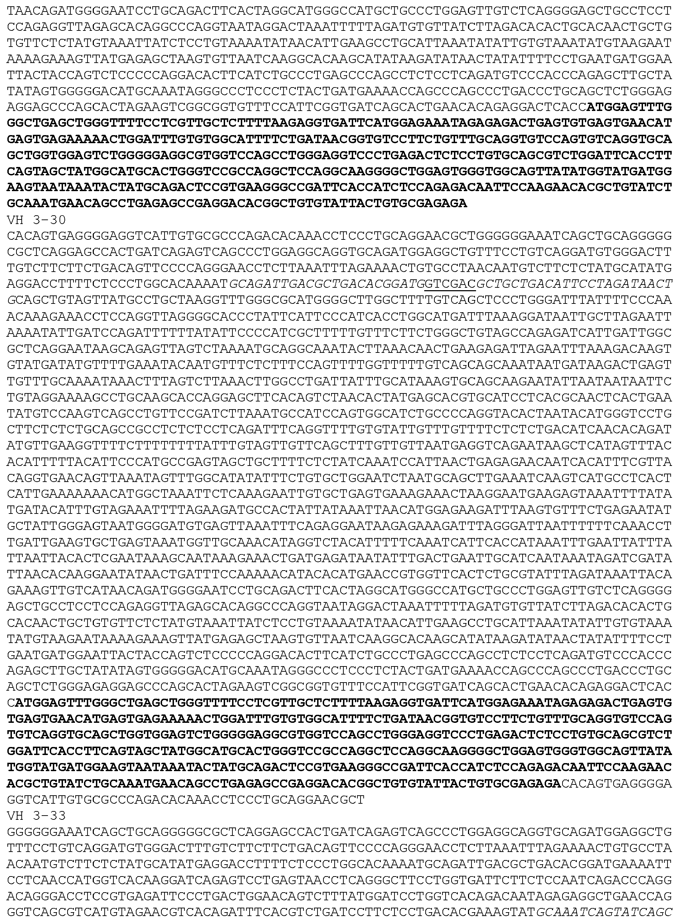

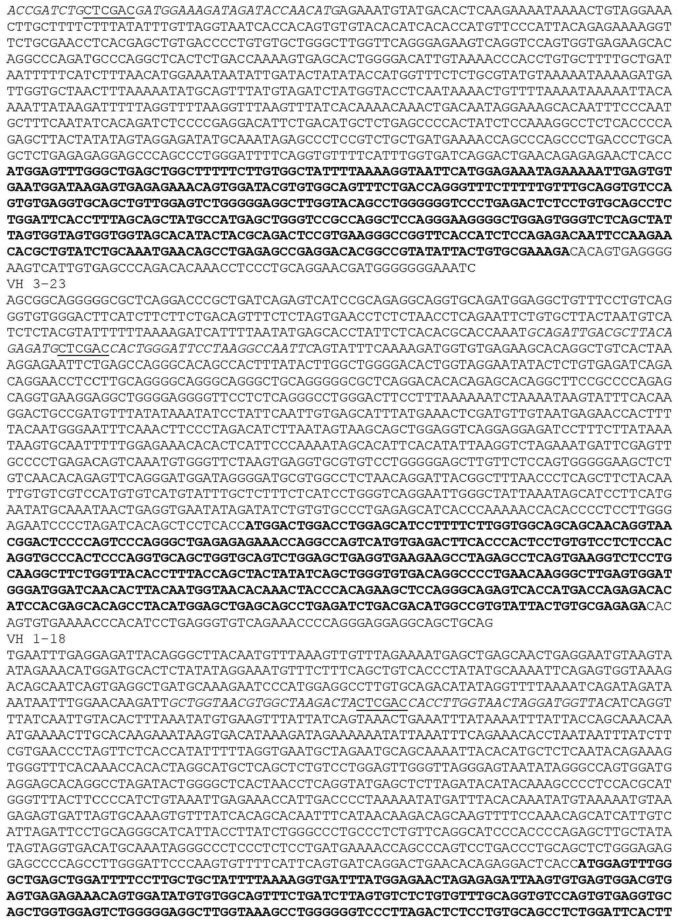

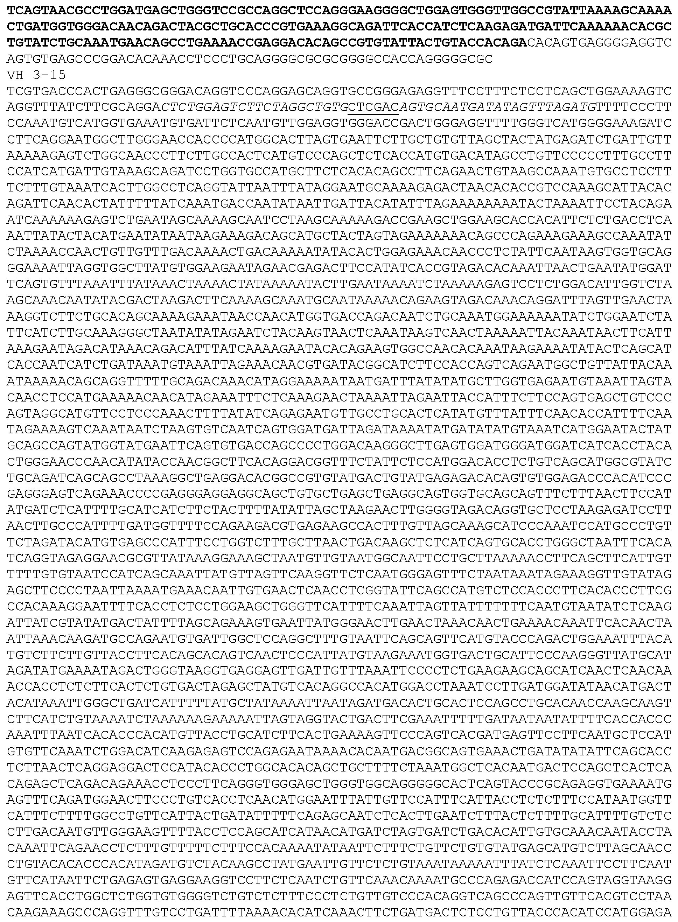

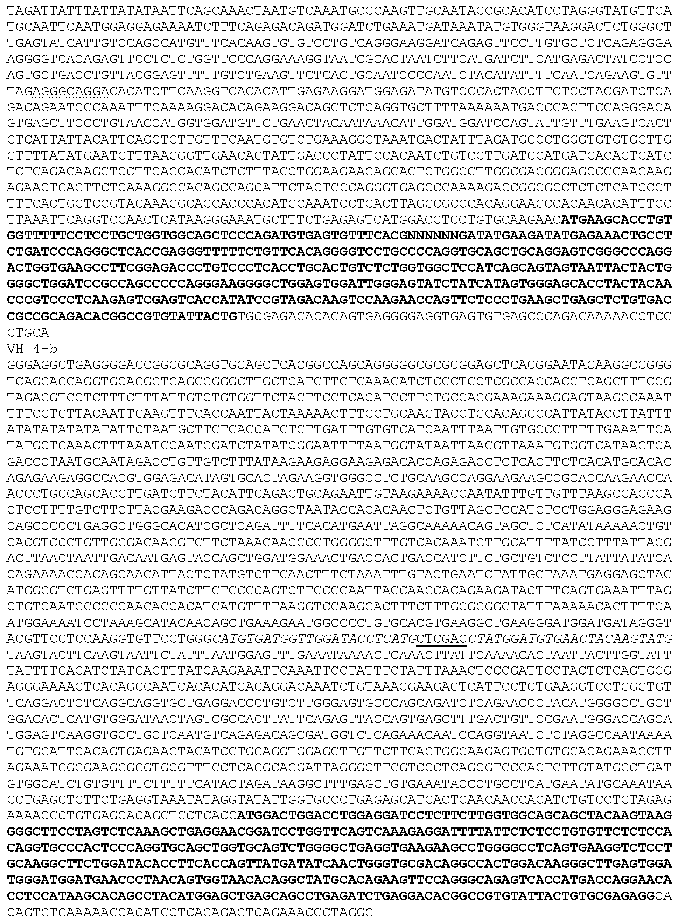

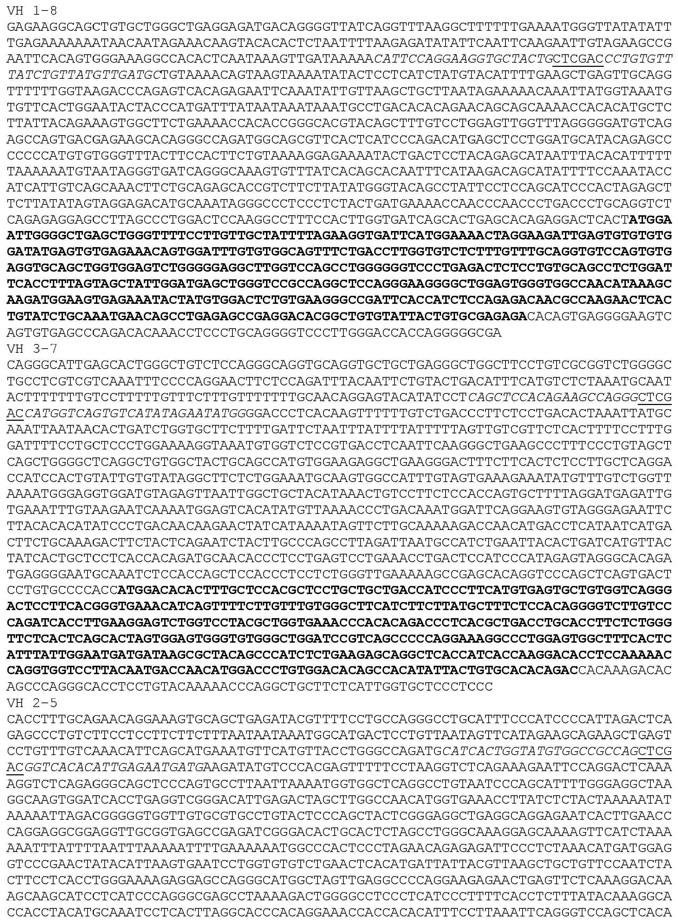

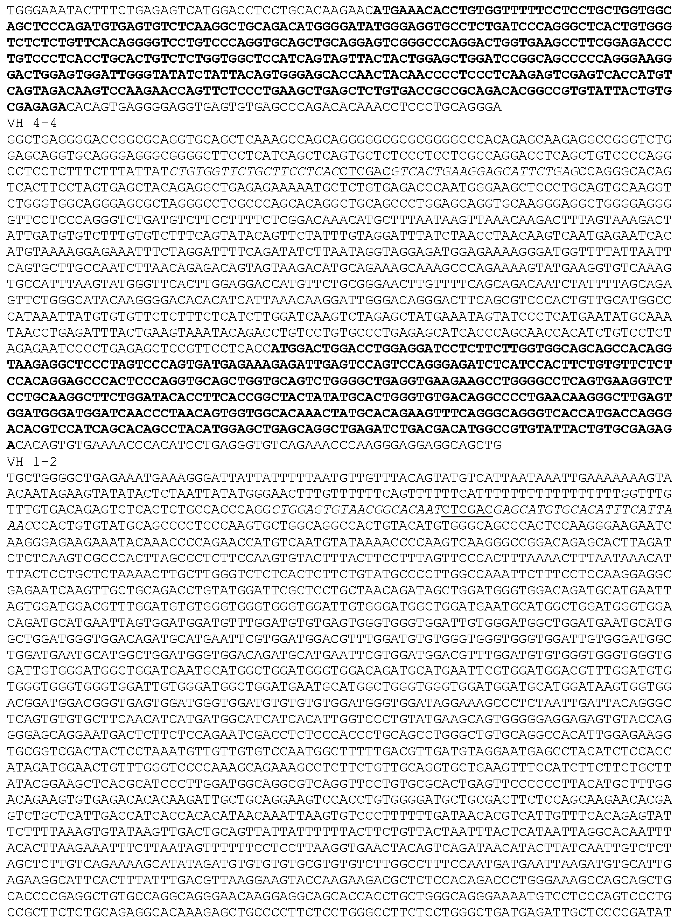

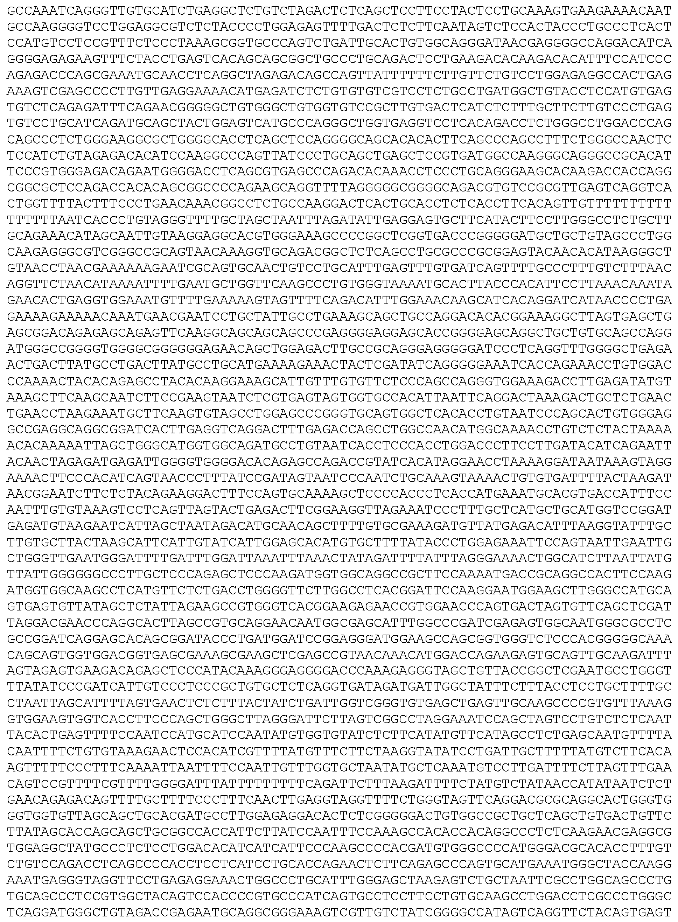

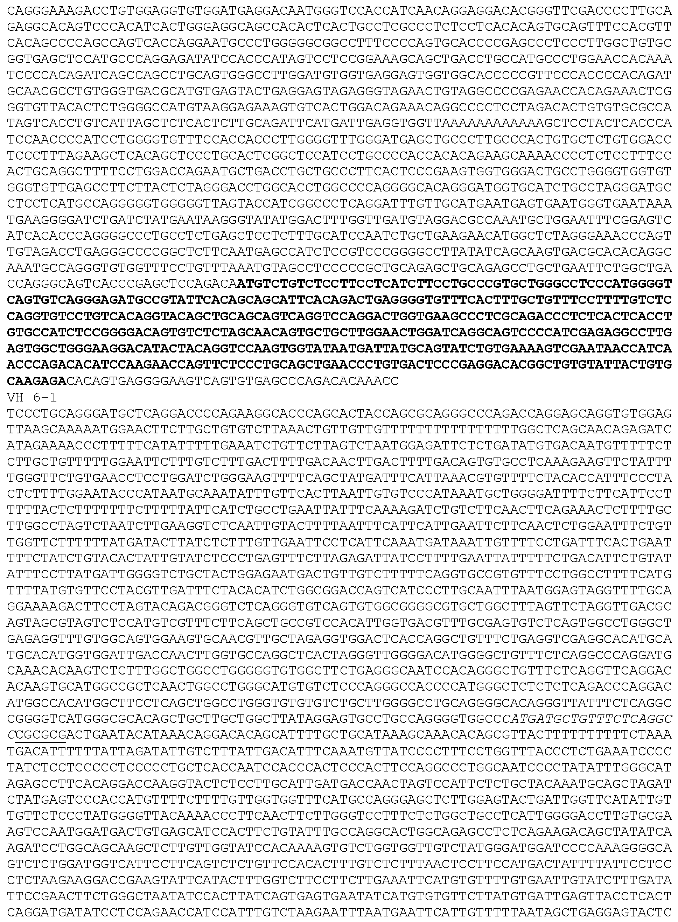

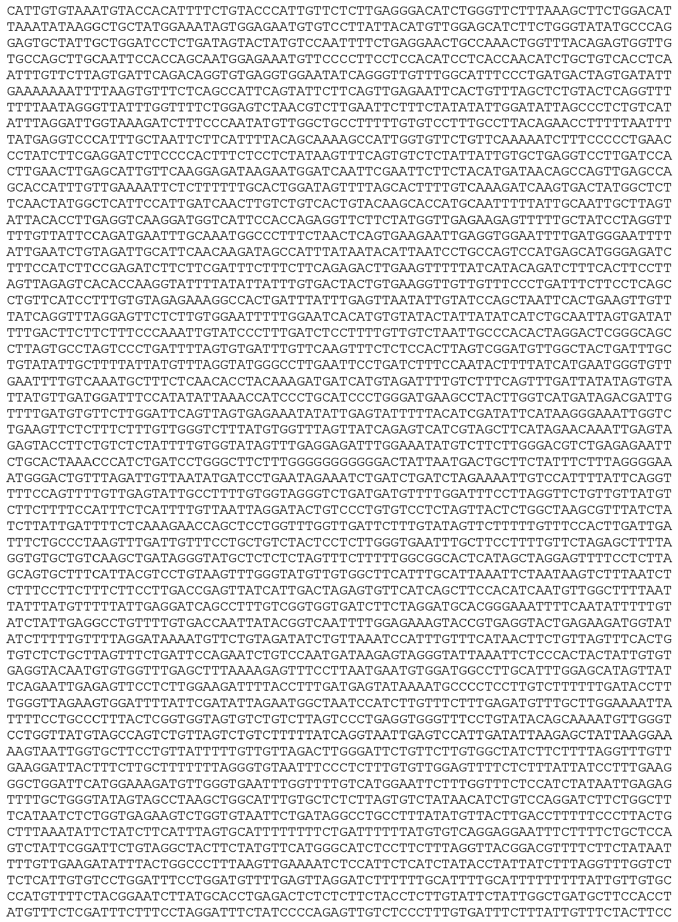

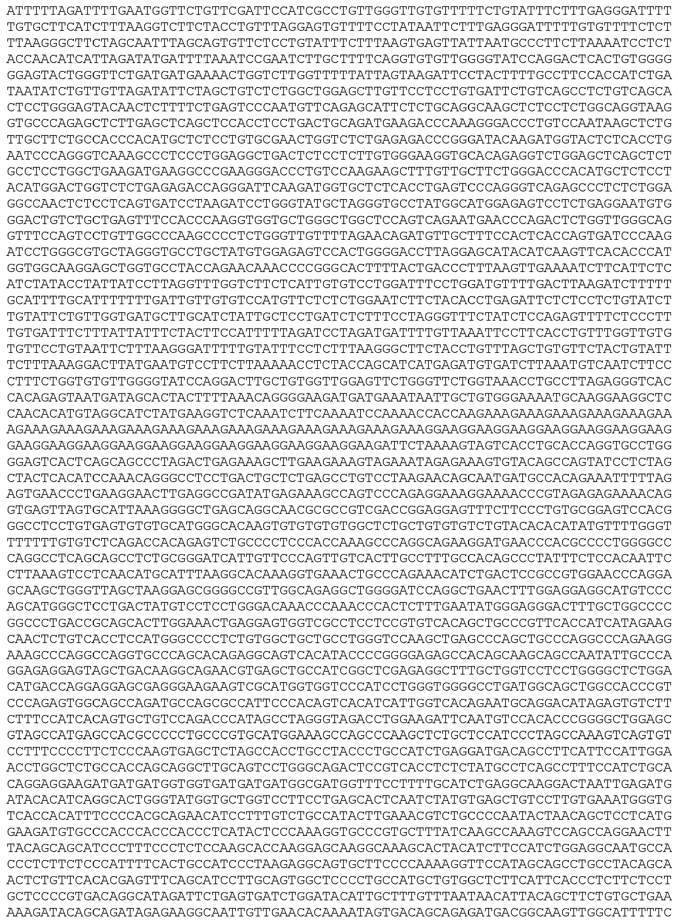

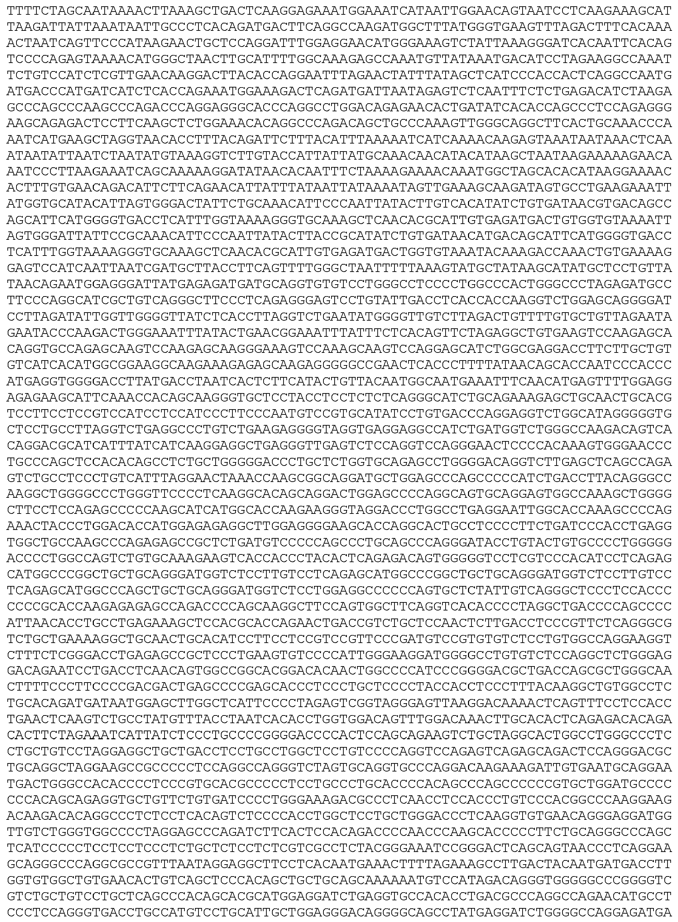

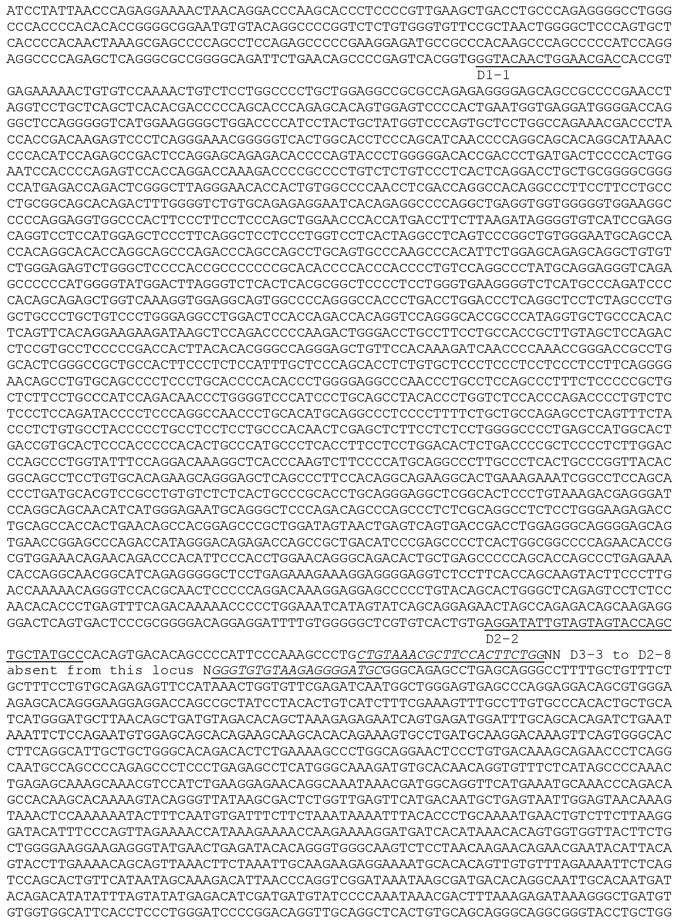

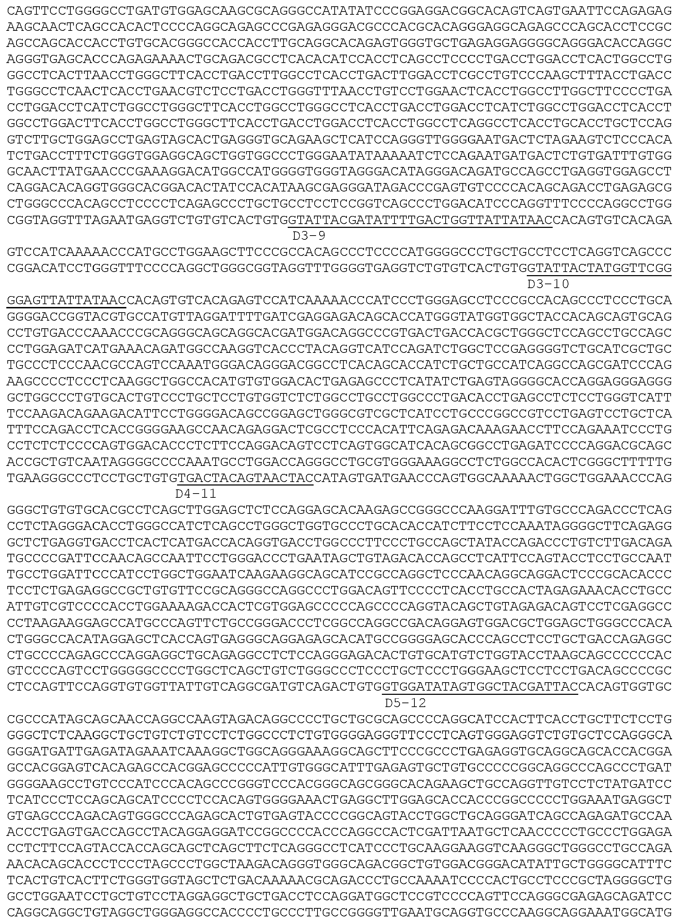

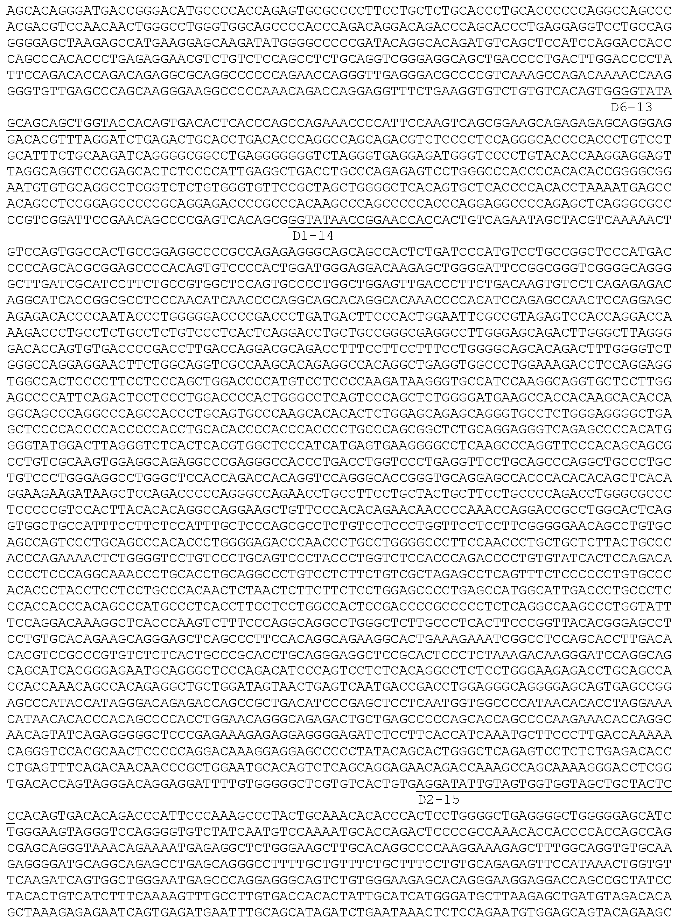

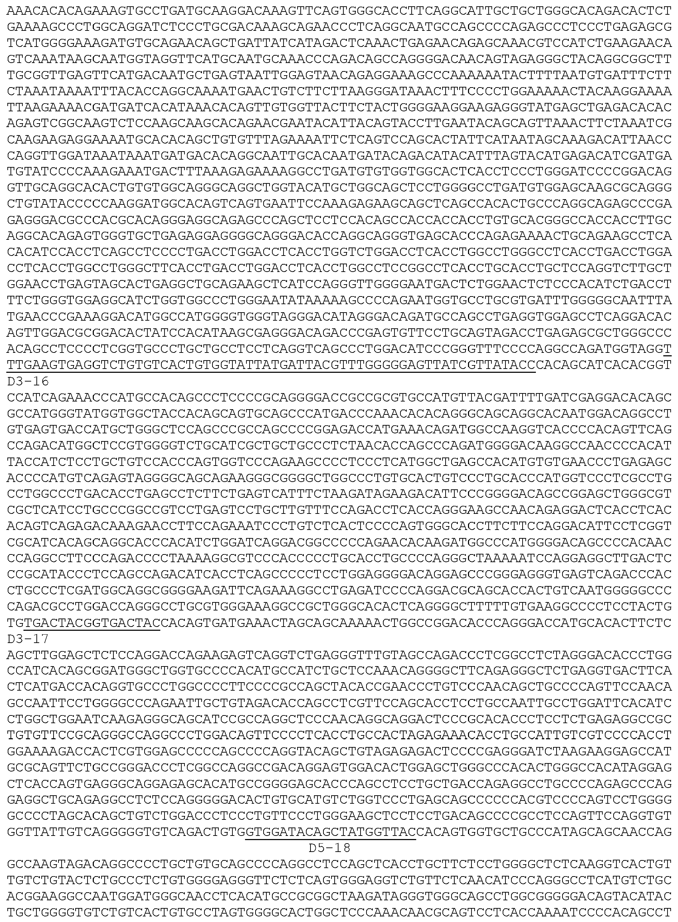

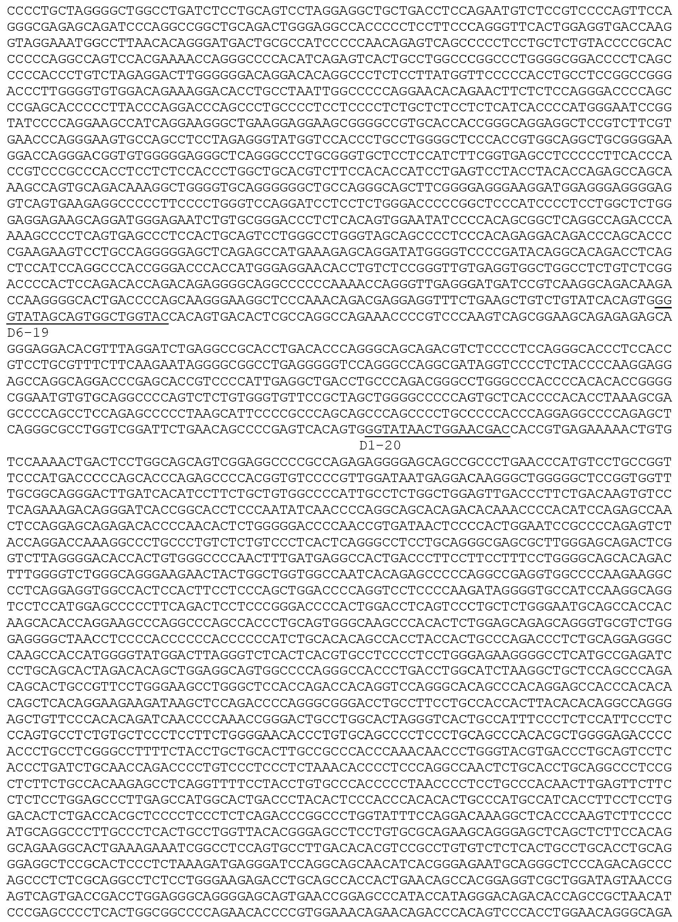

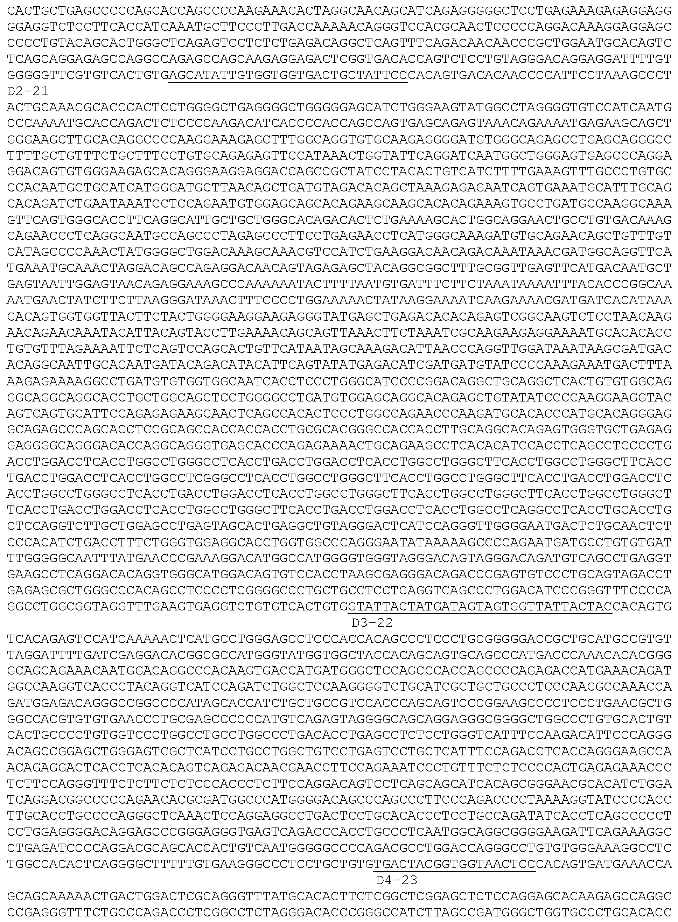

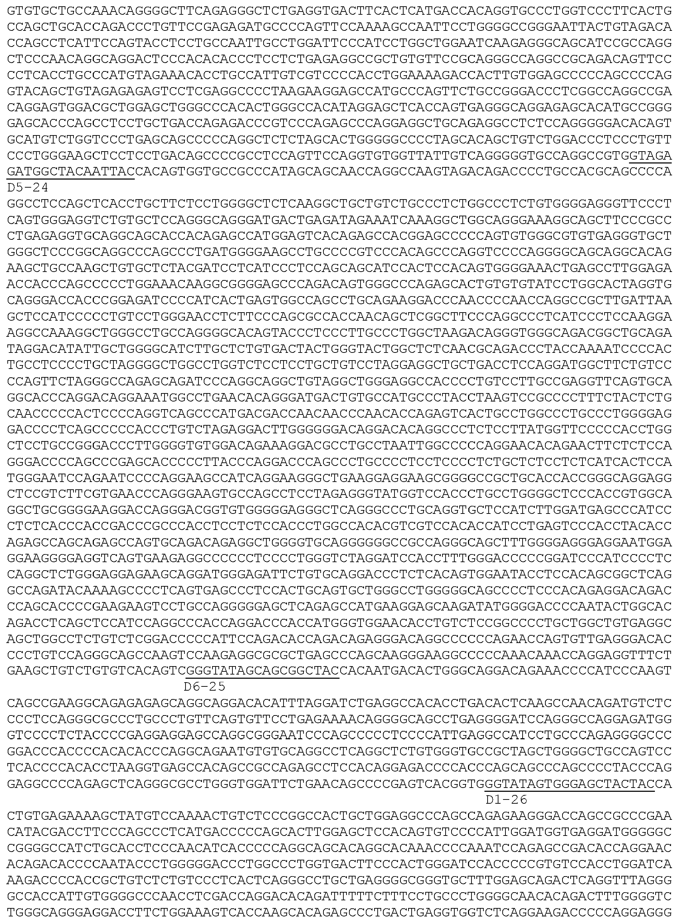

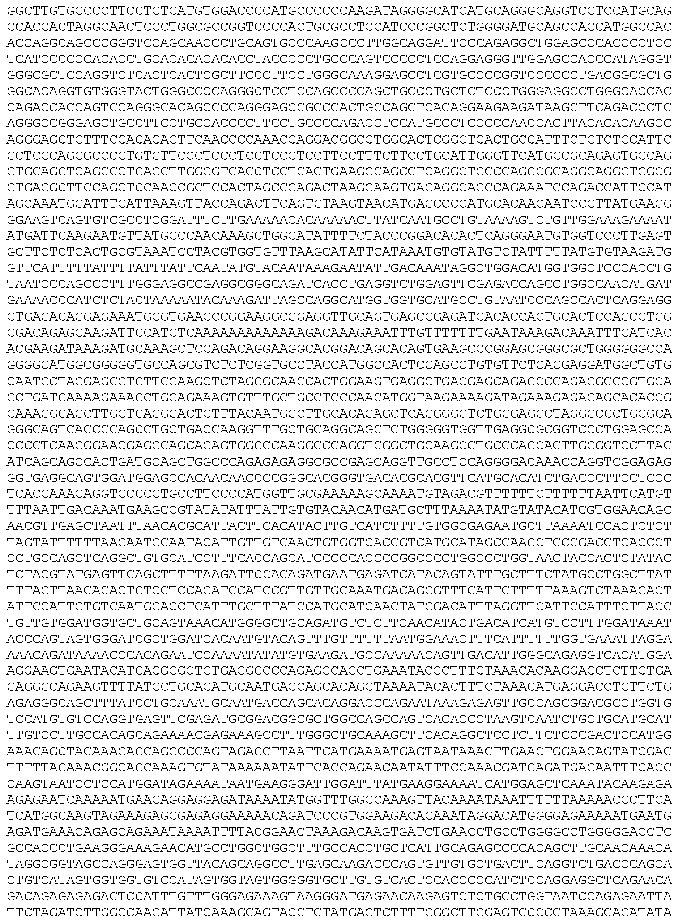

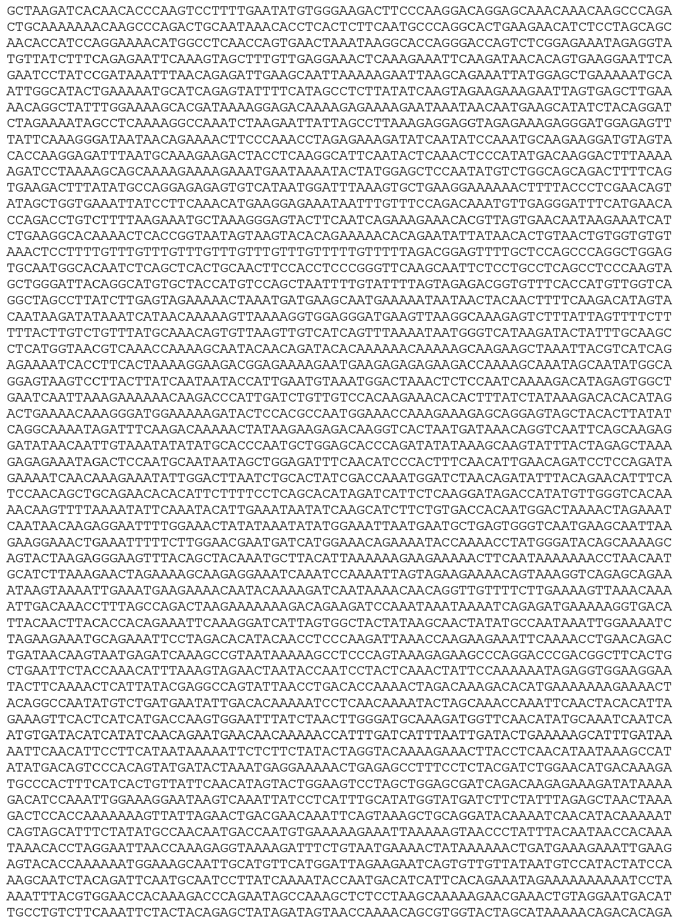

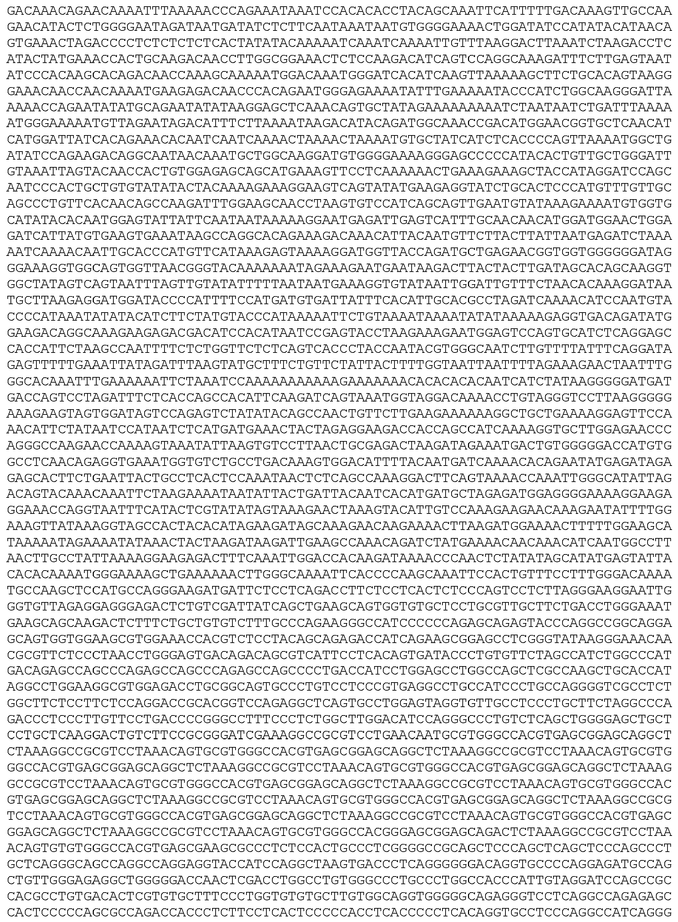

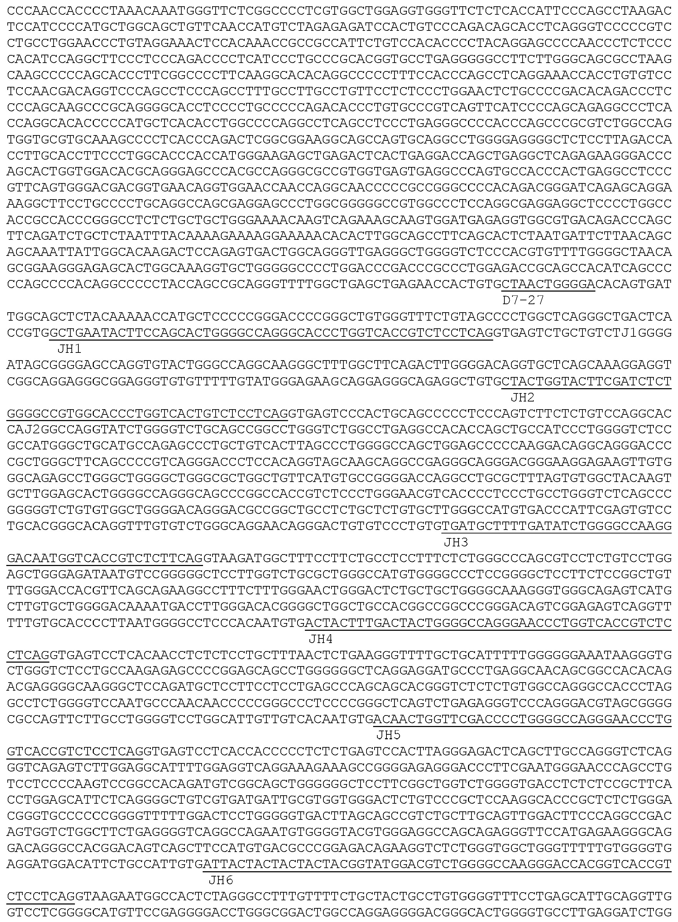

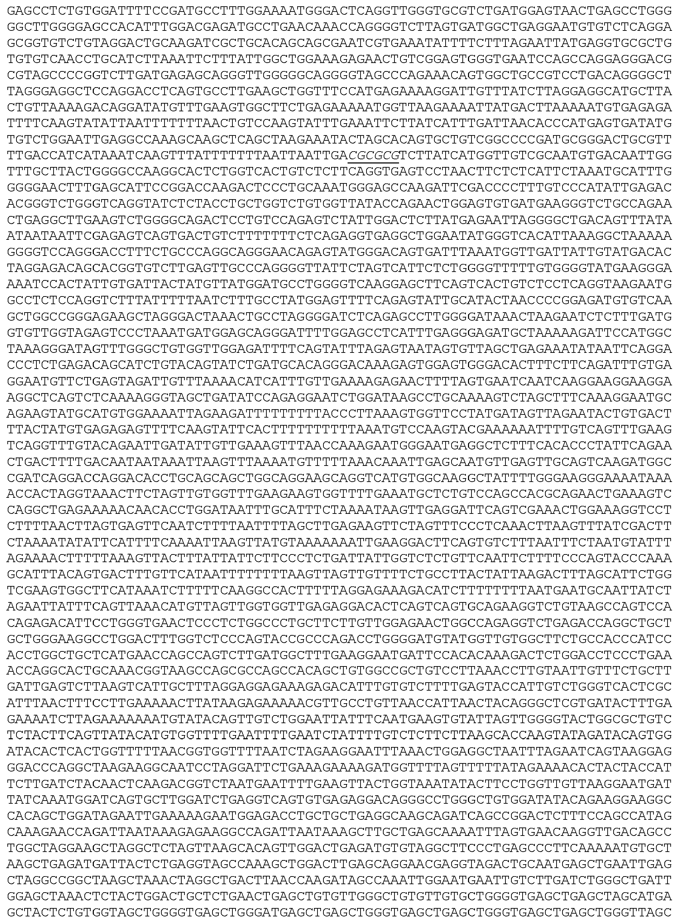

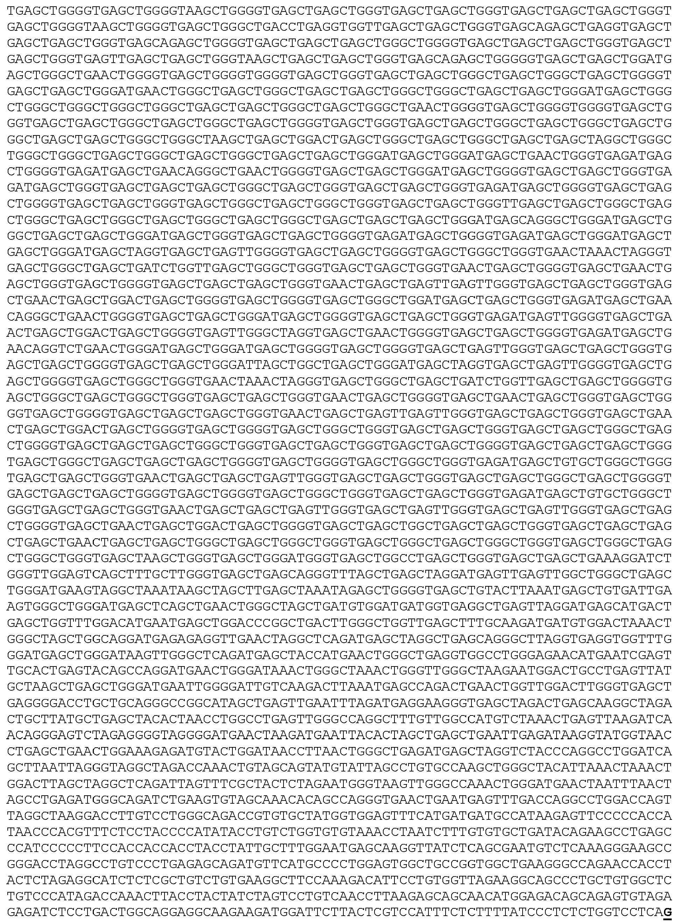

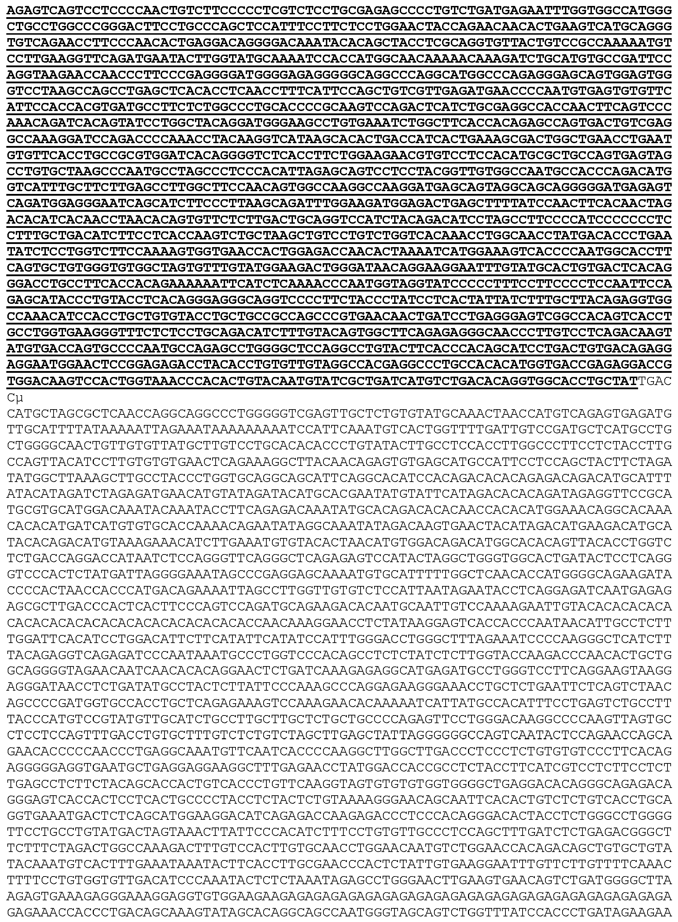

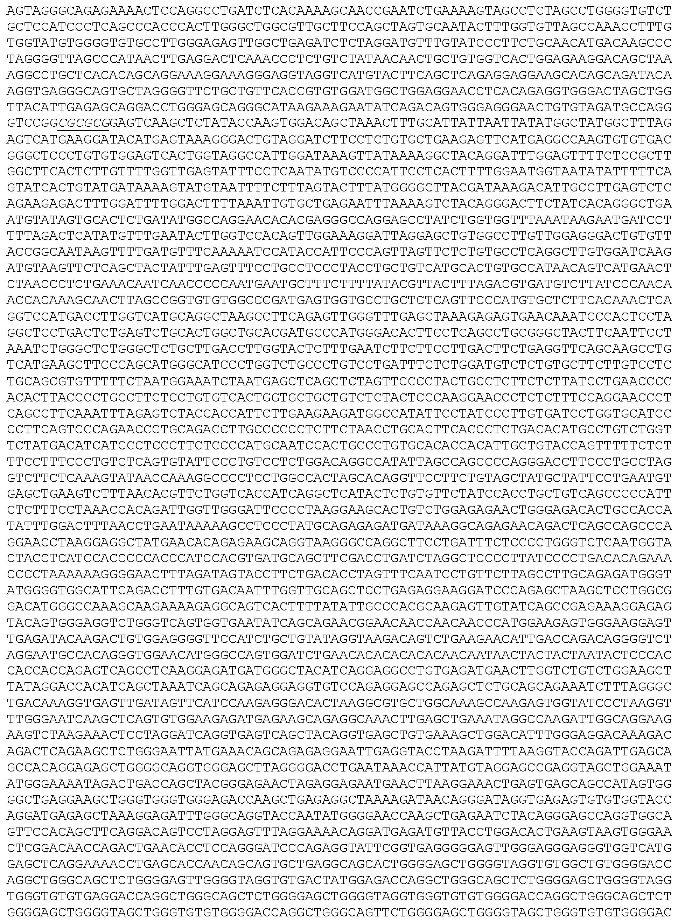

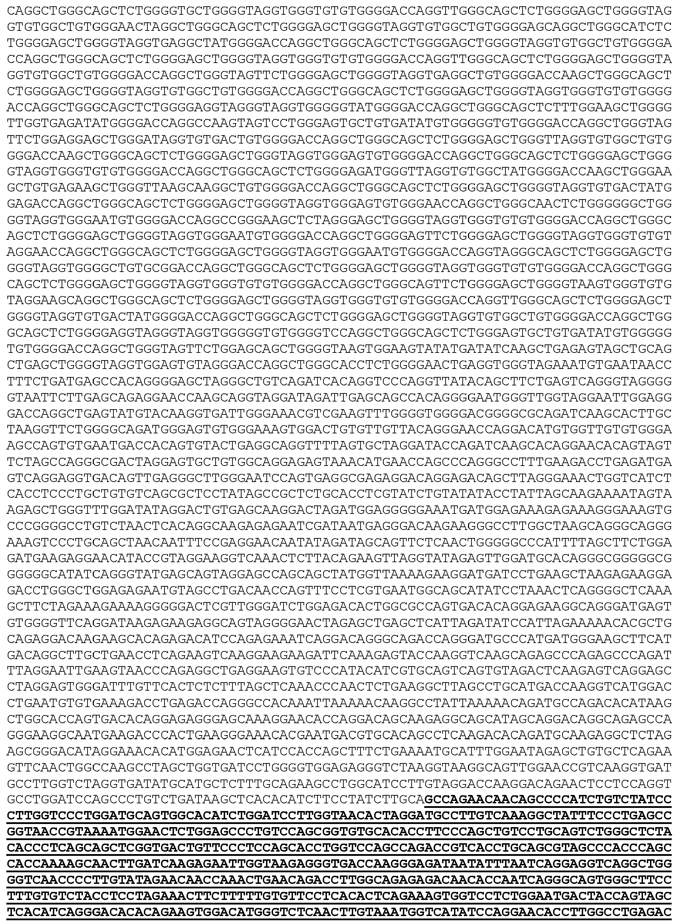

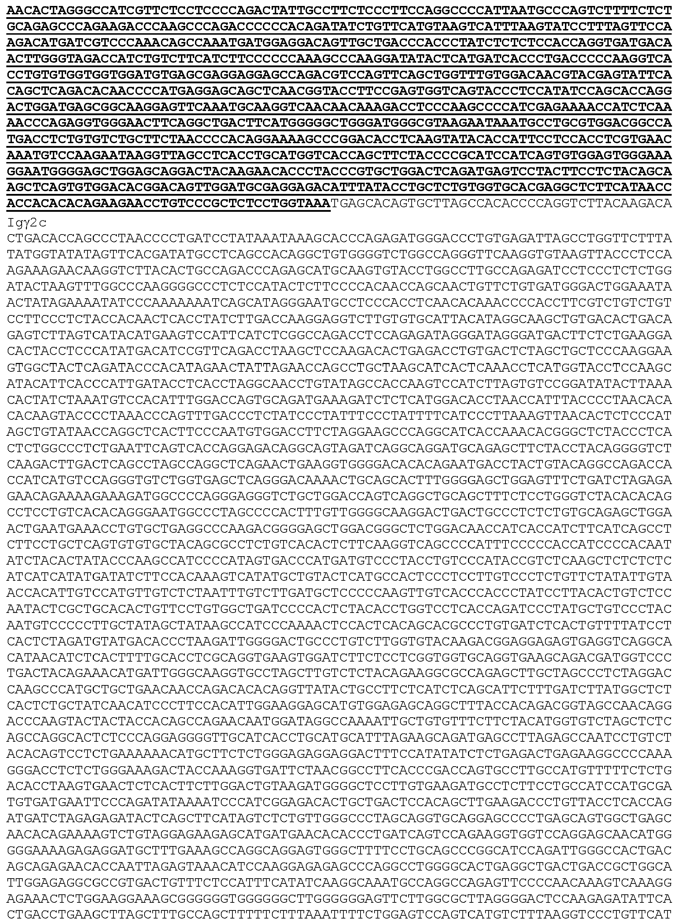

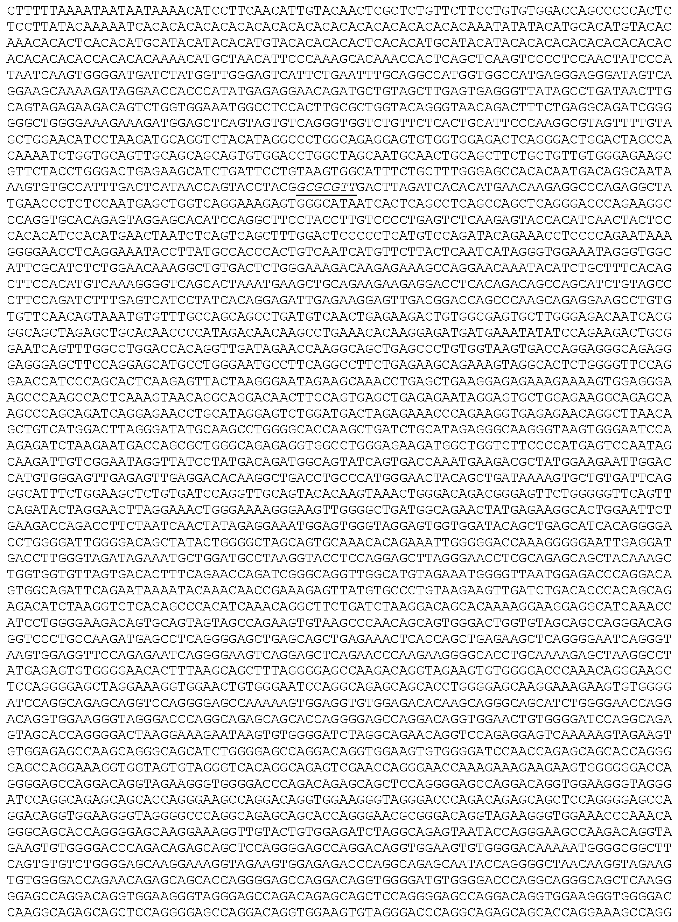

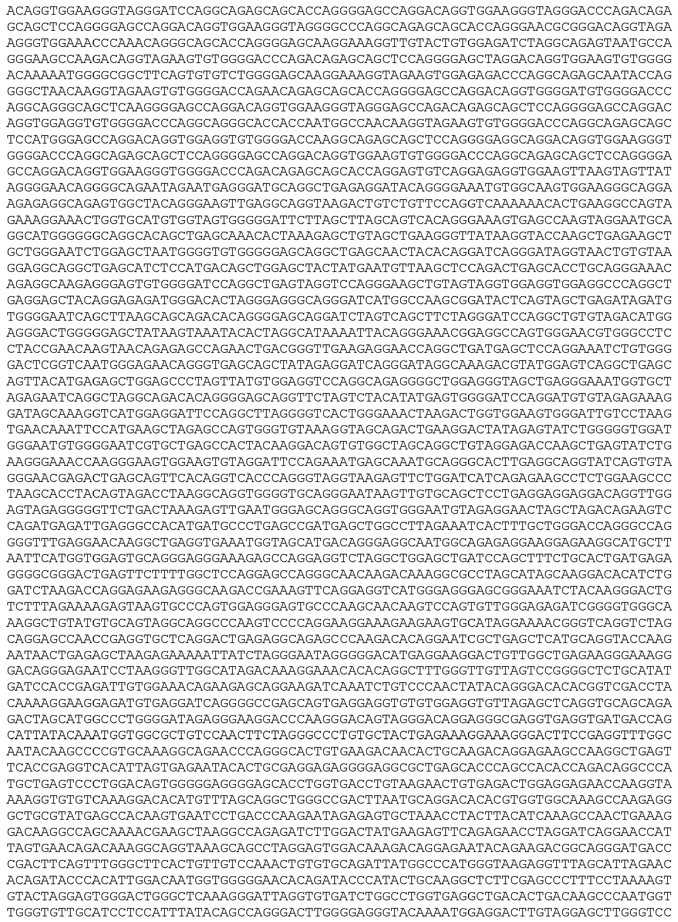

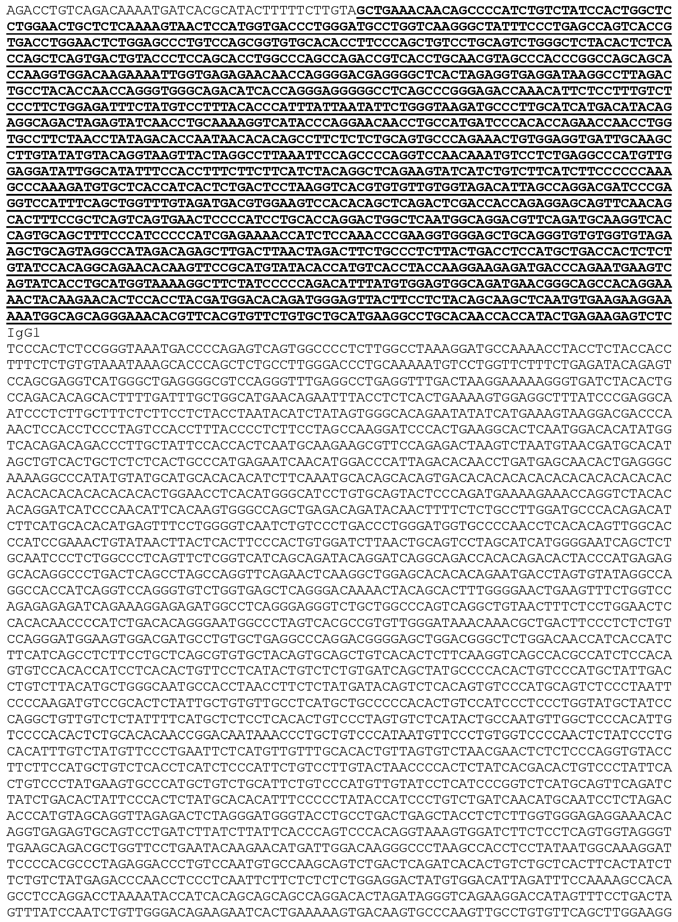

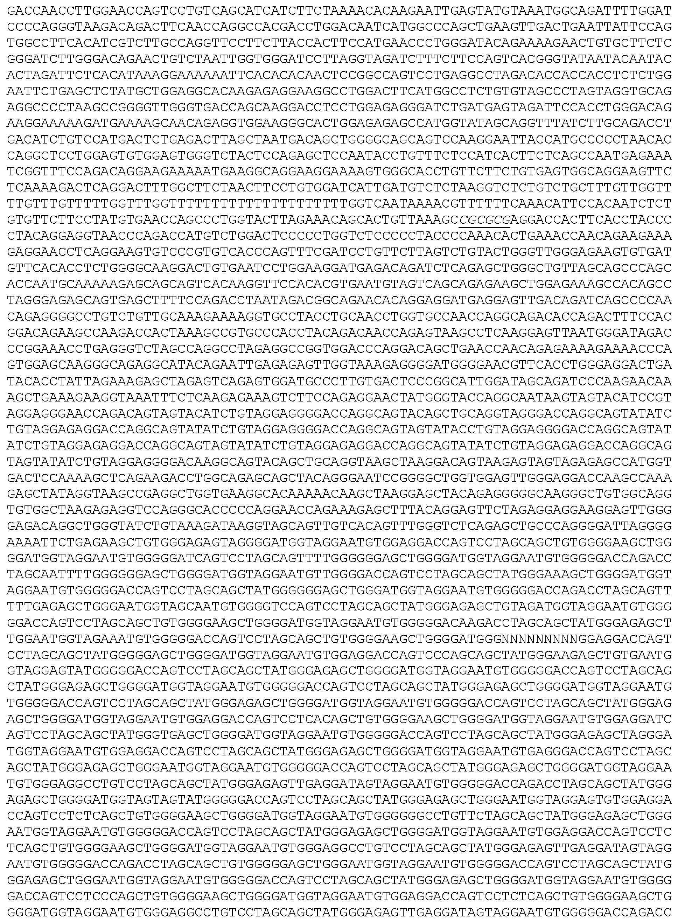

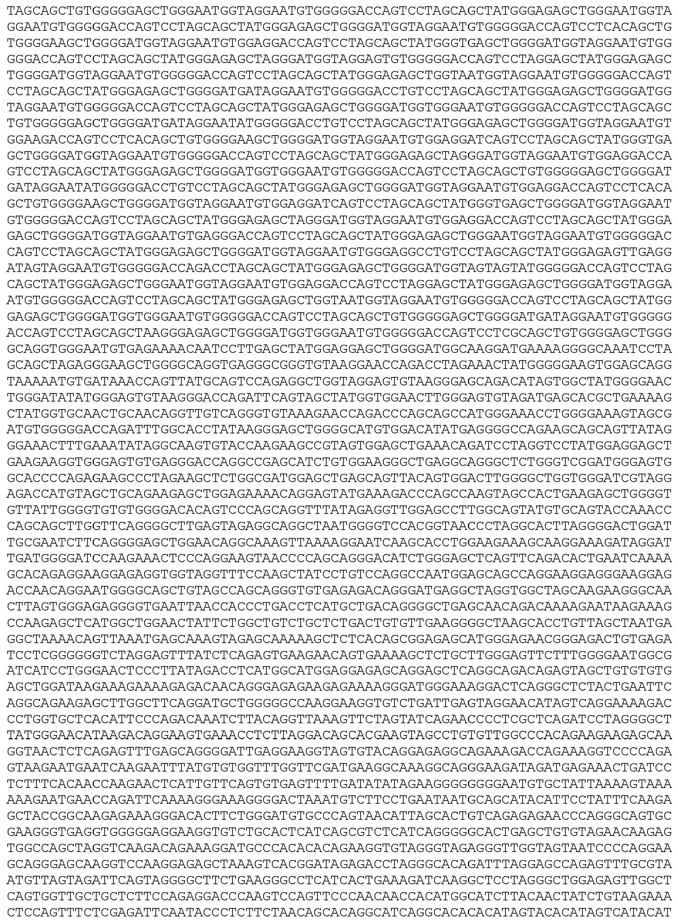

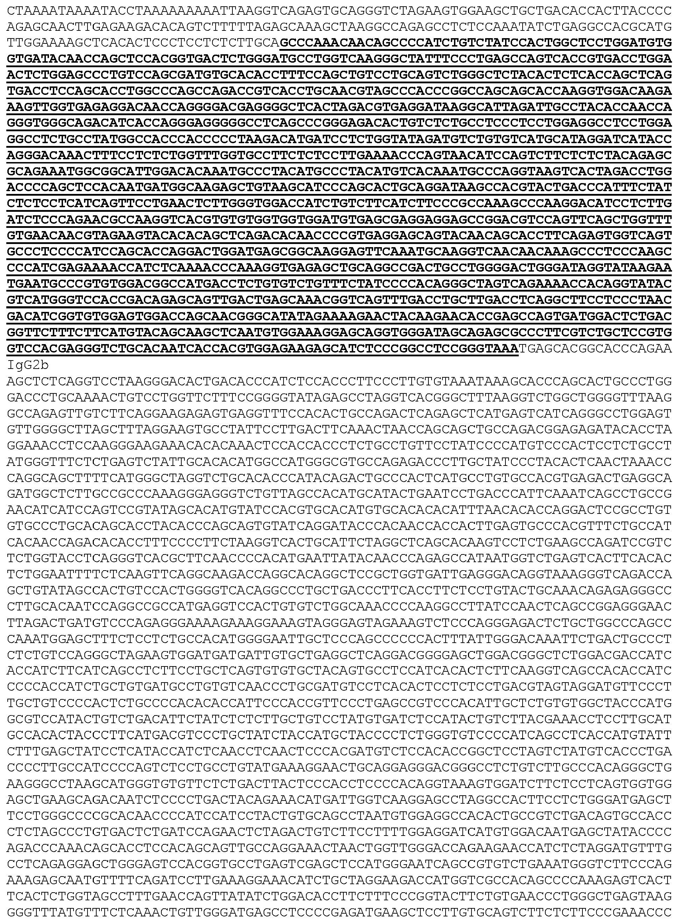

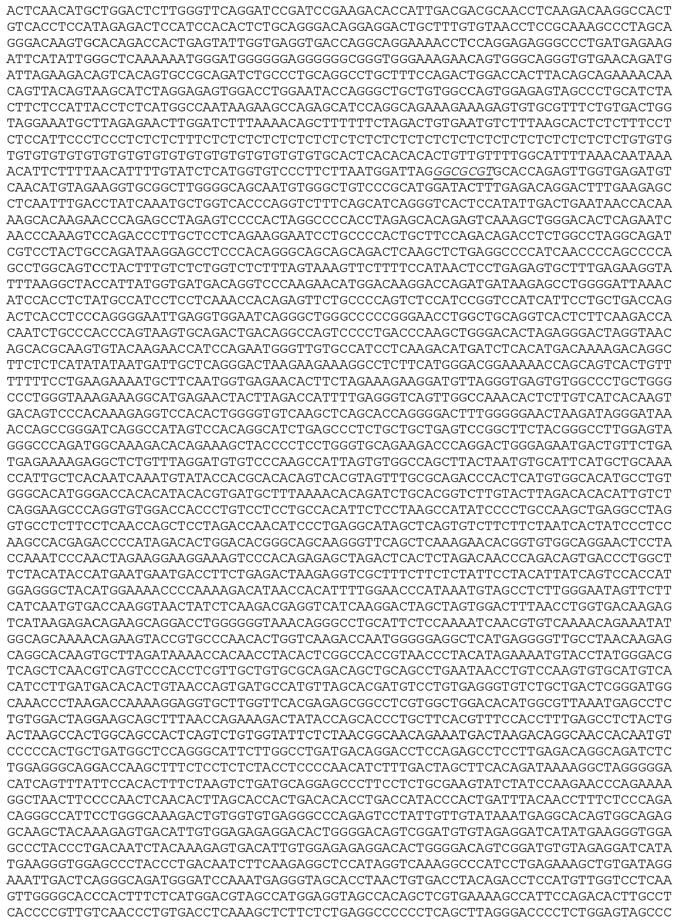

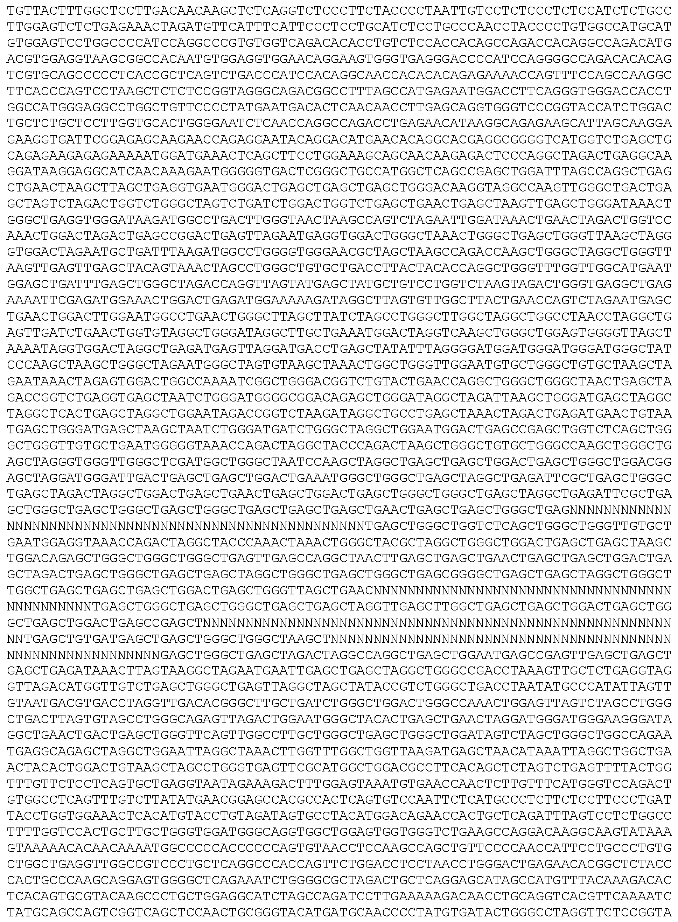

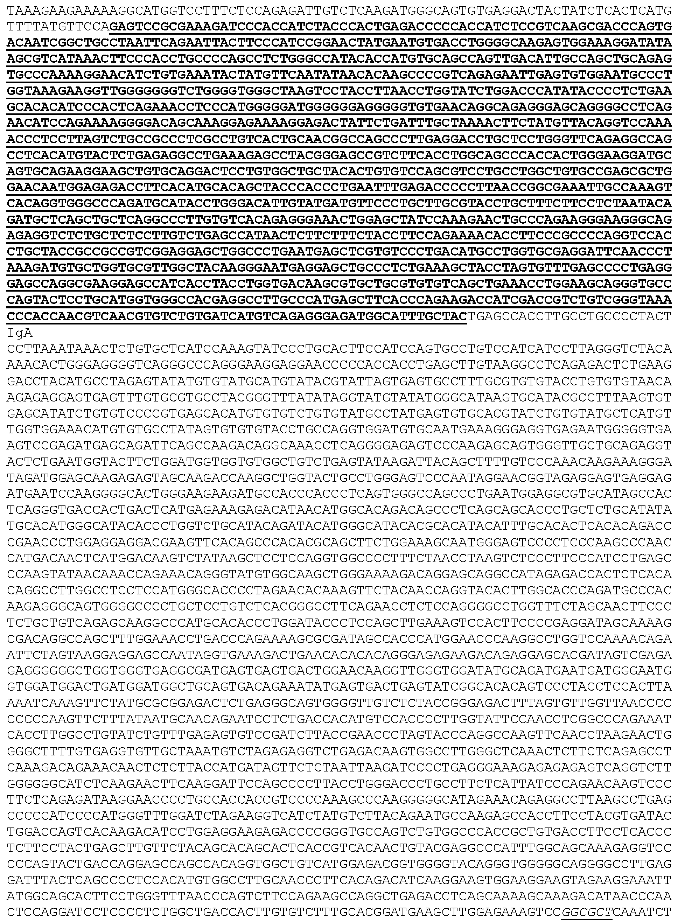

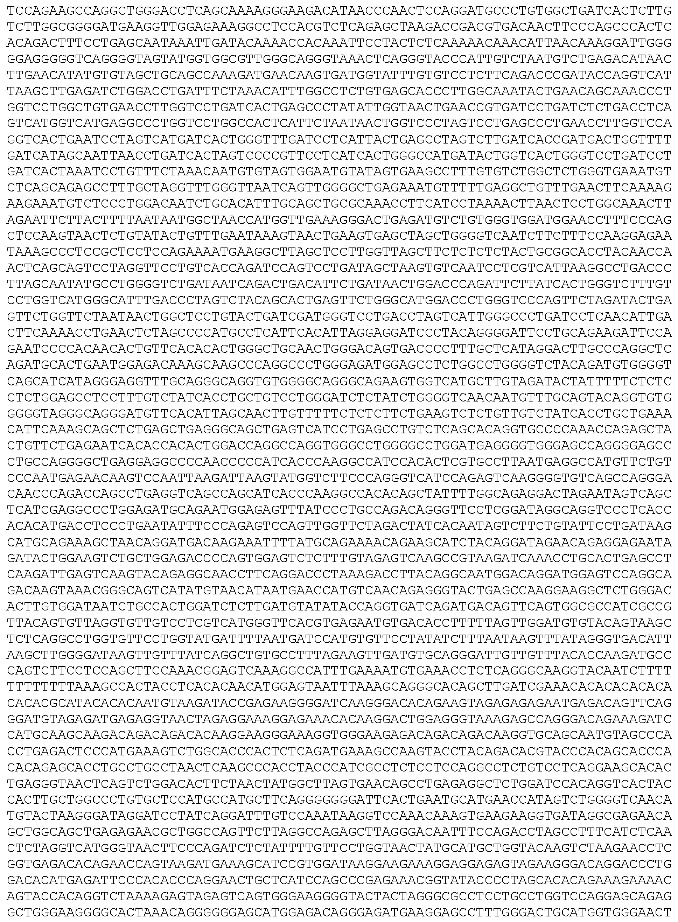

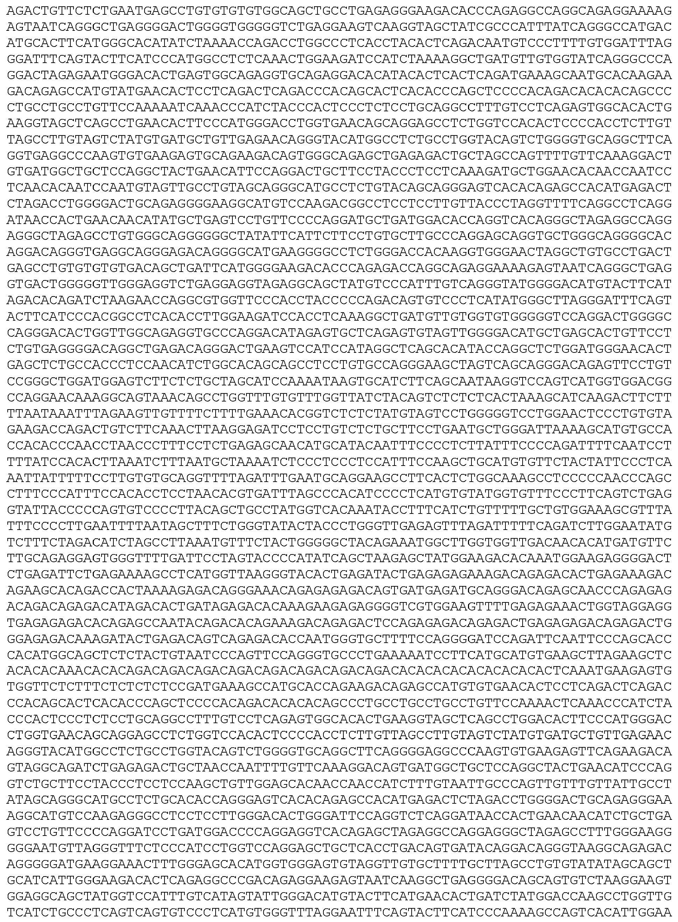

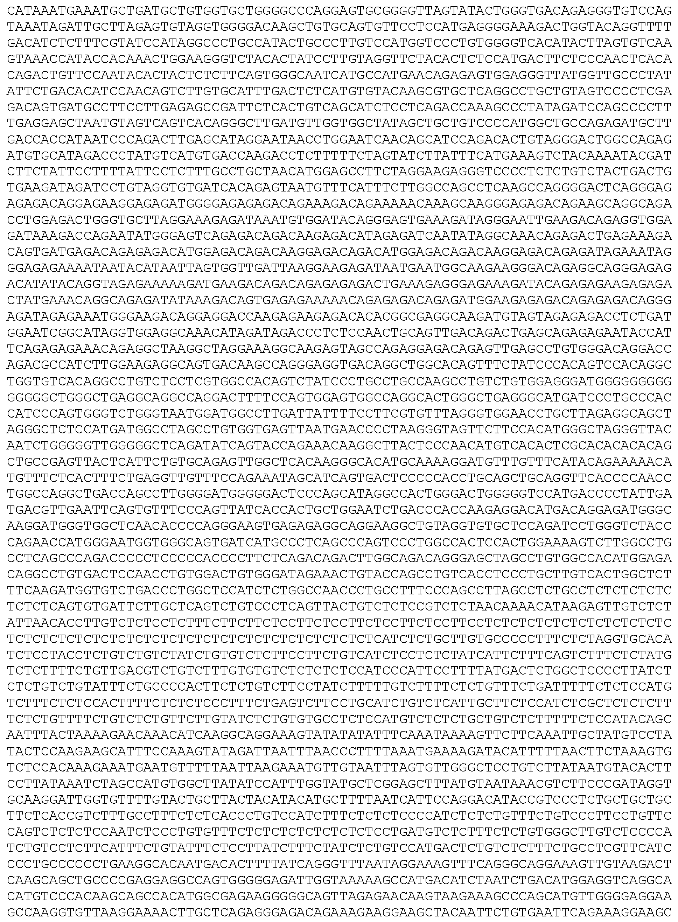

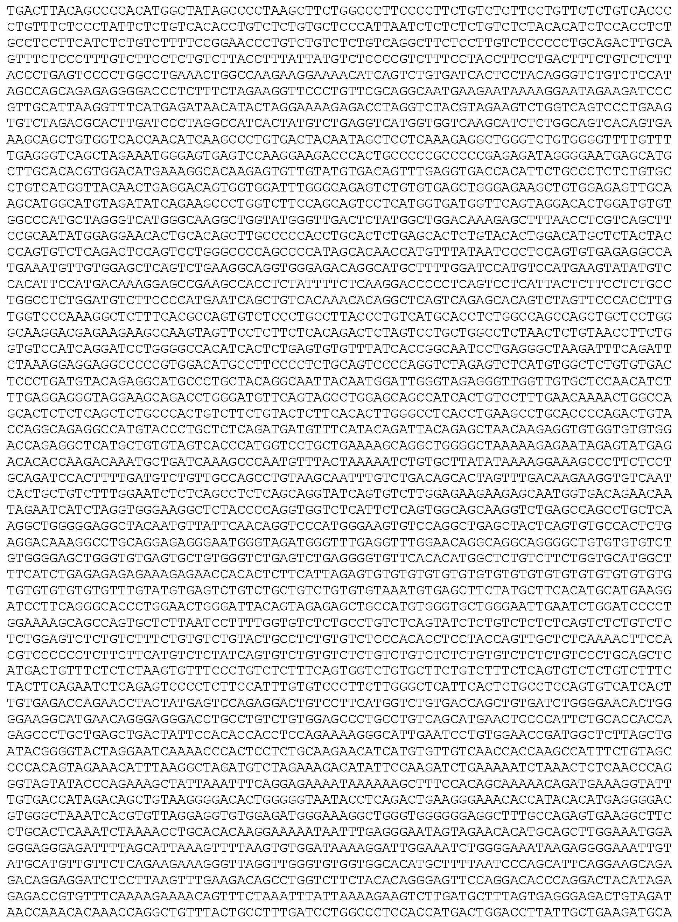

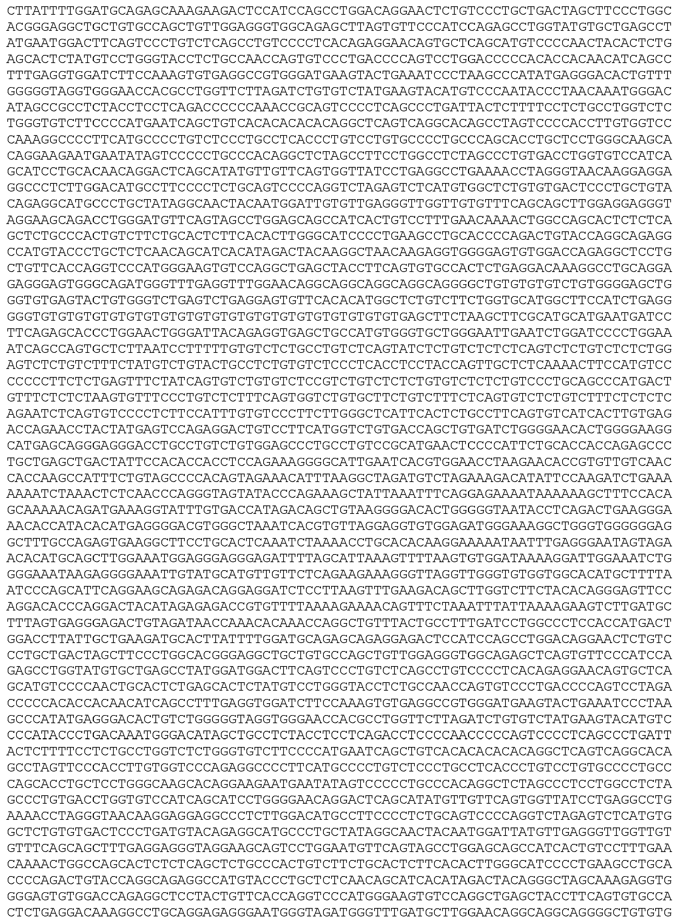

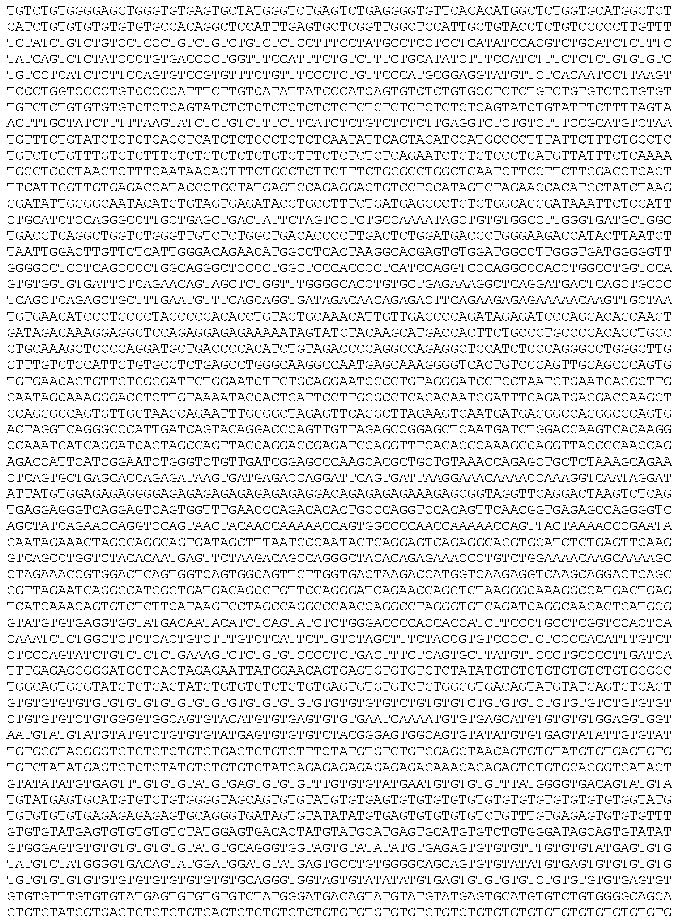

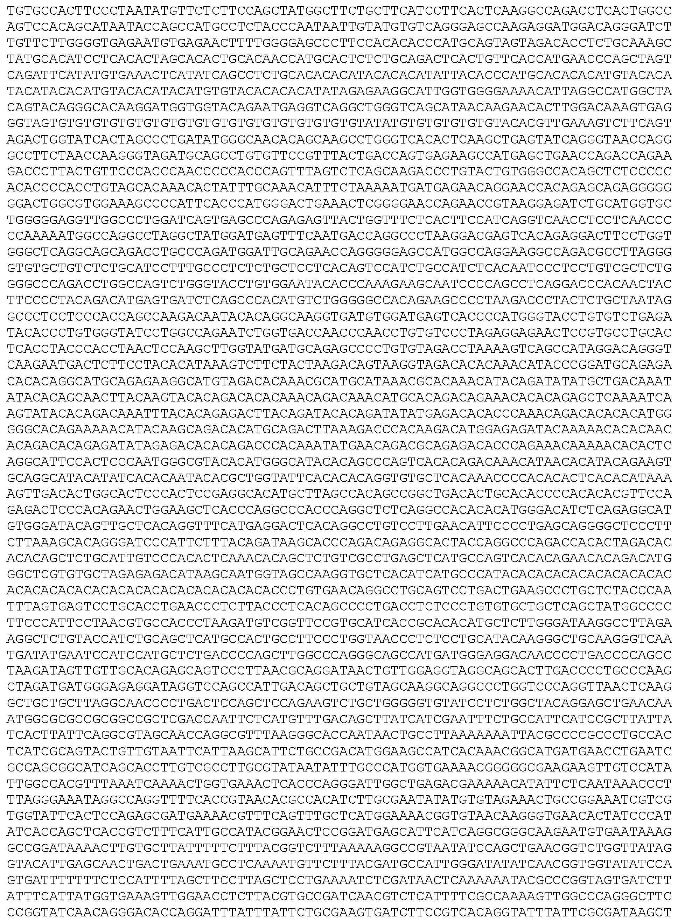

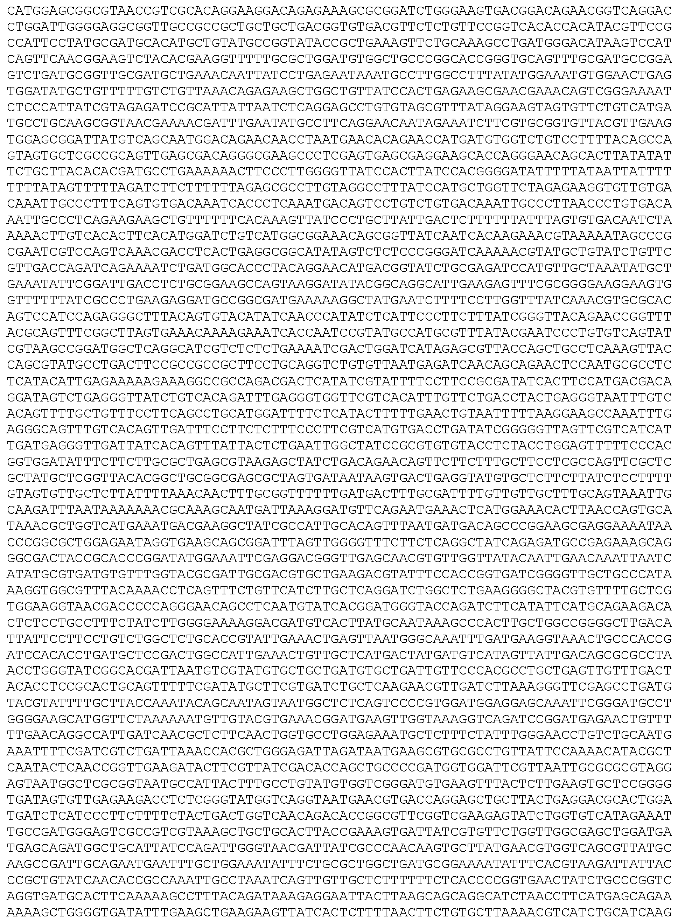

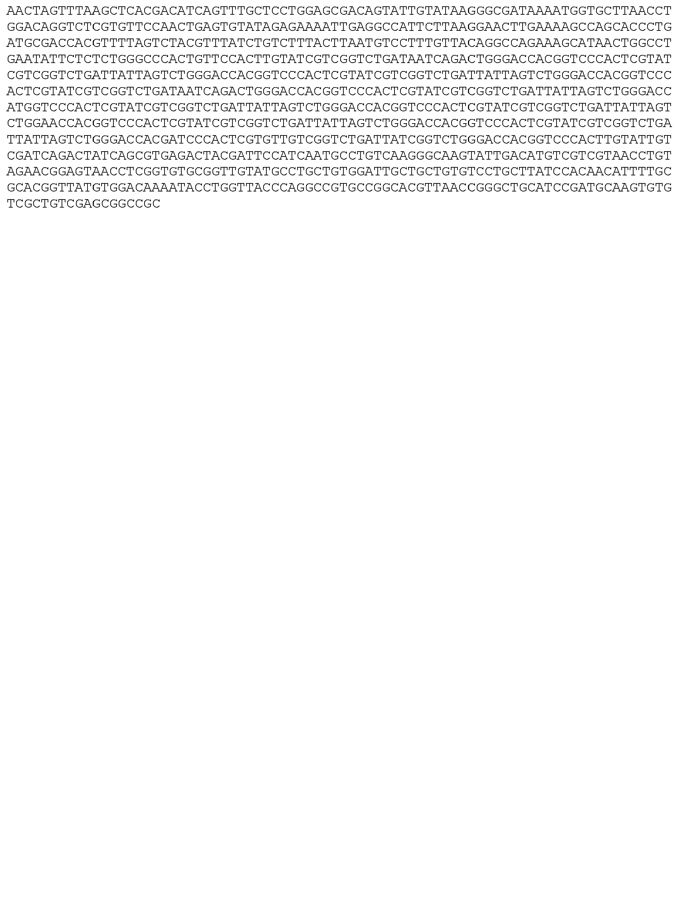

**Figure 4** depicts a Heavy chain locus sequence (SEQ ID NO: 1).
   The sequences highlighted in bold are the amino acid coding and intron sequences of the VH segments. Underlined between these are the junction sequences. Each of these is flanked by a sequence in italics that signals the beginning and end of each VH segment (coding plus flanking sequences). D segments and J segments are underlined and constant regions are in bold and underlined.

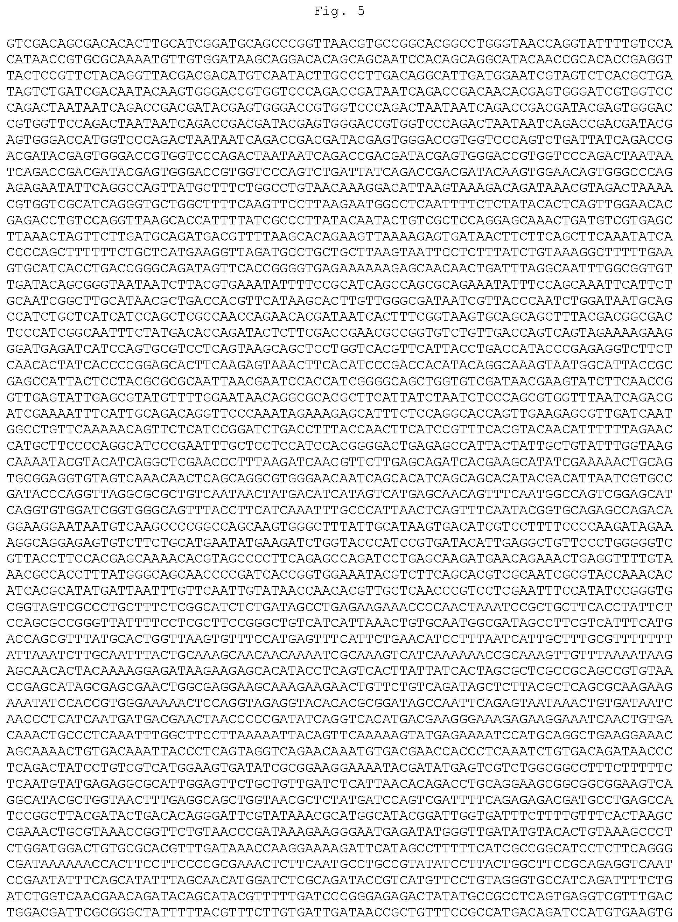

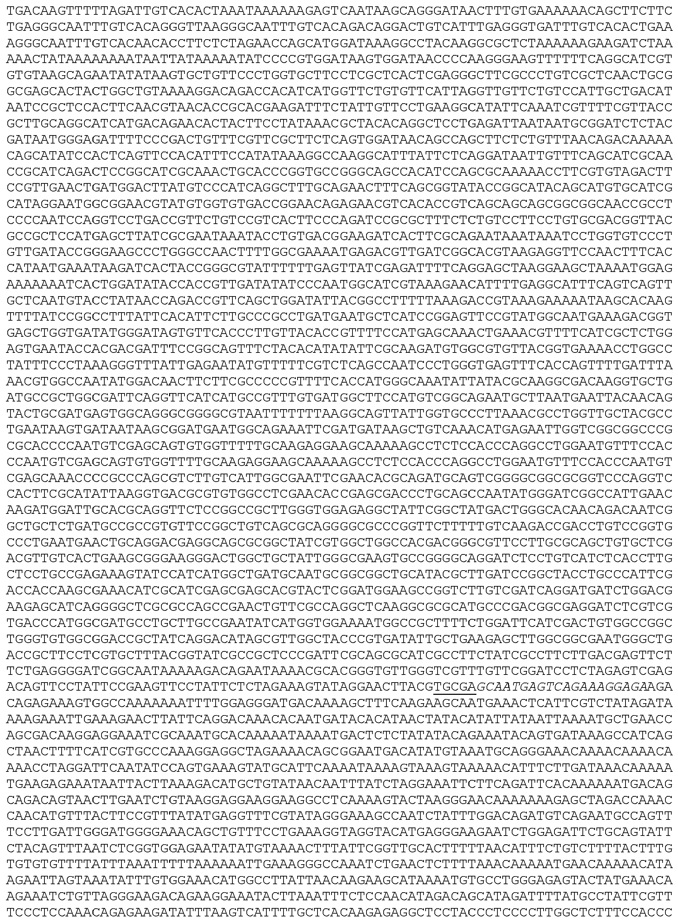

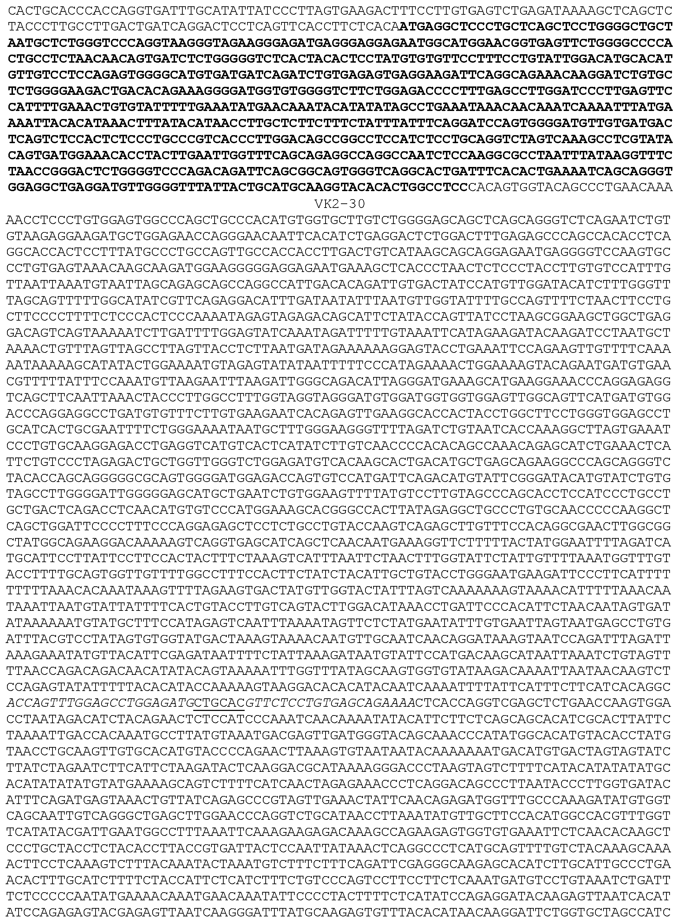

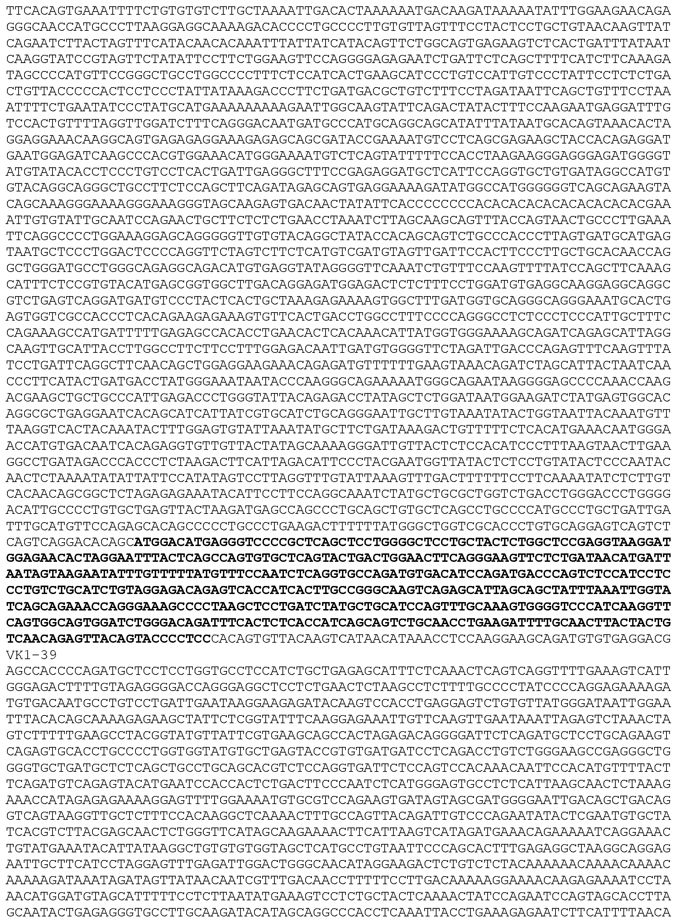

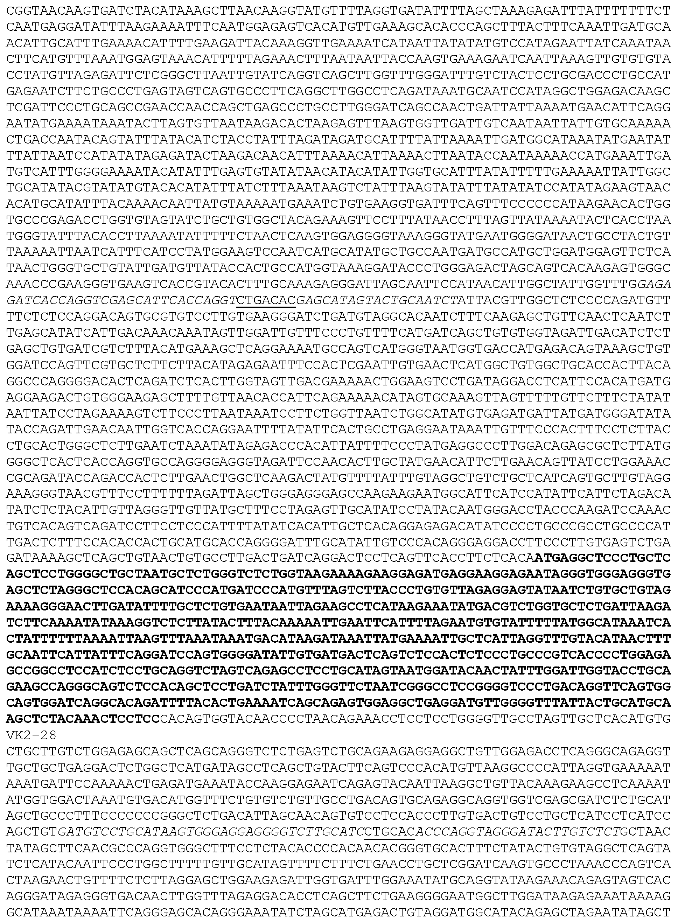

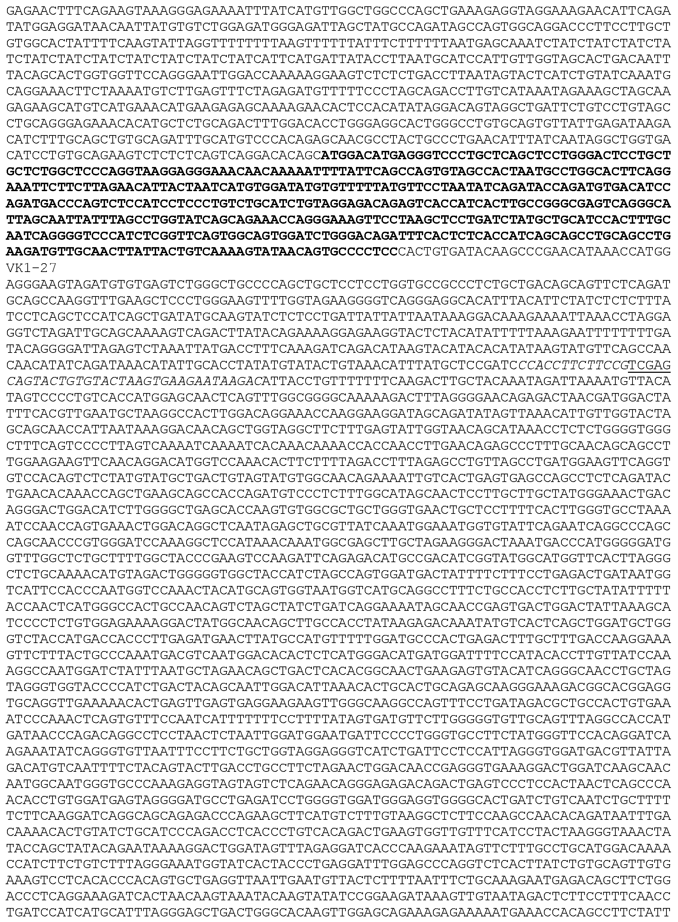

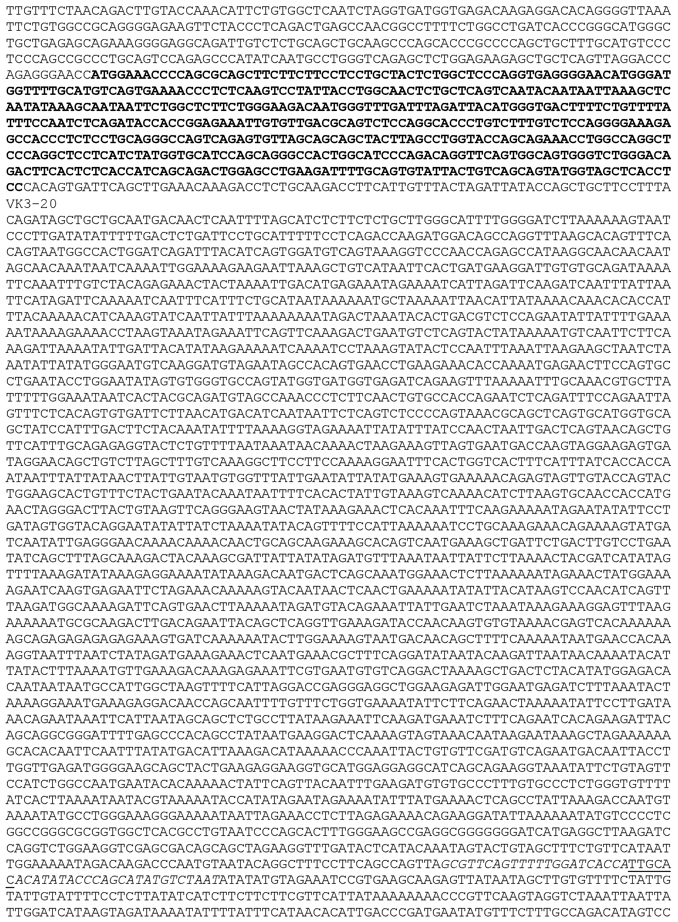

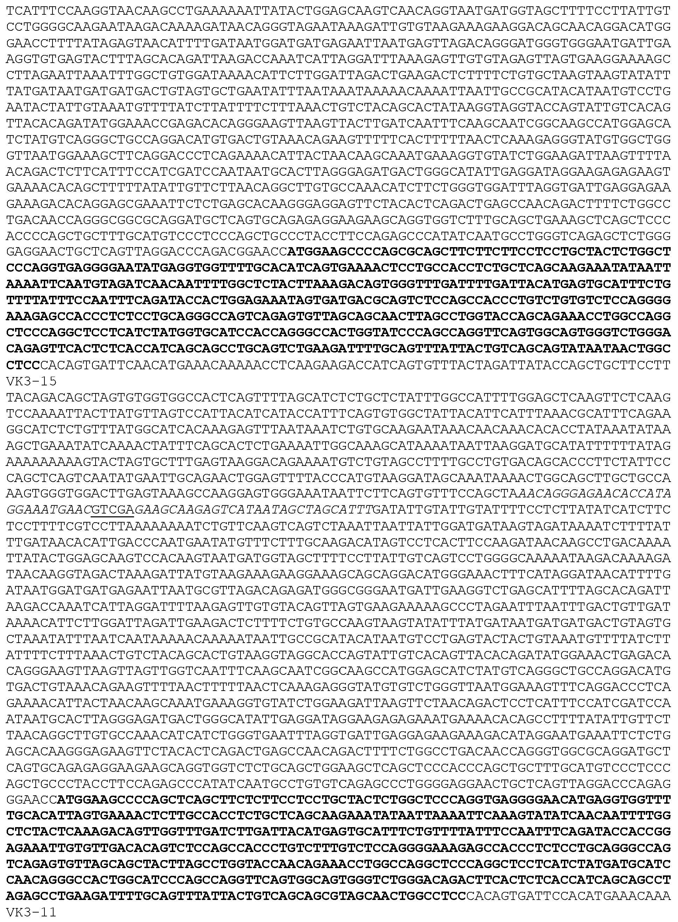

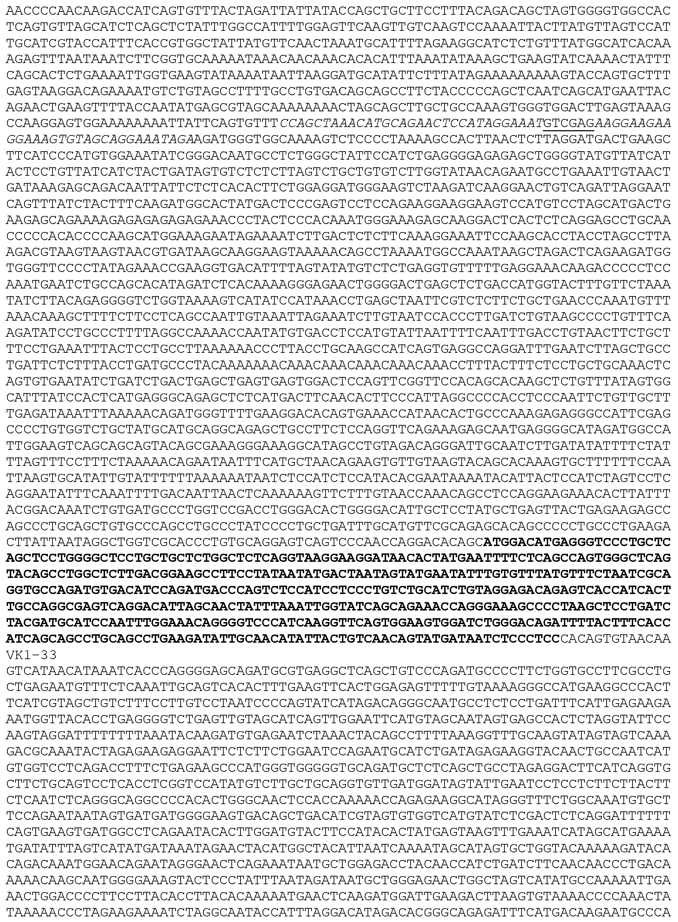

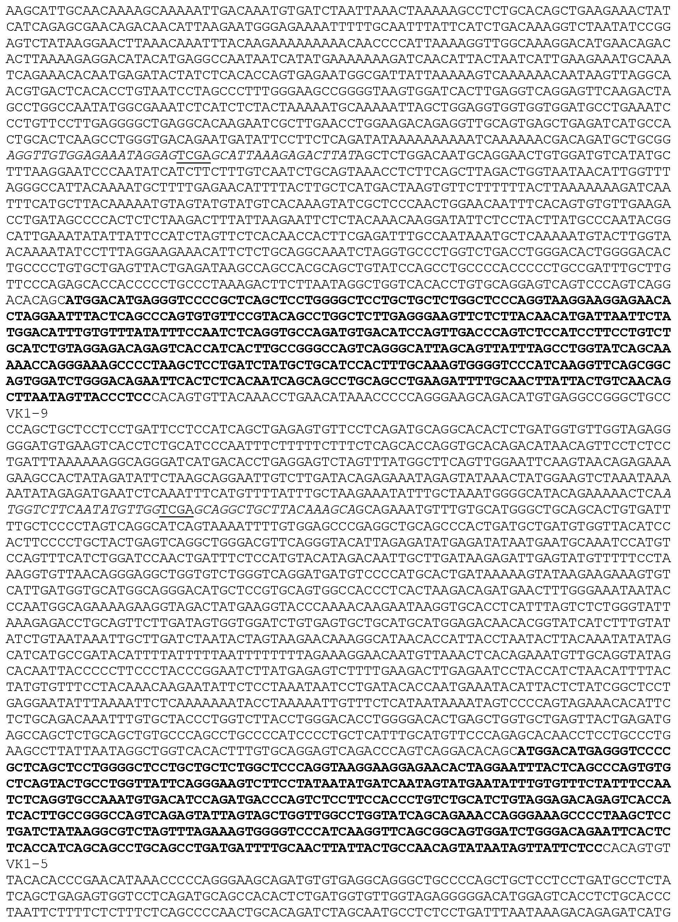

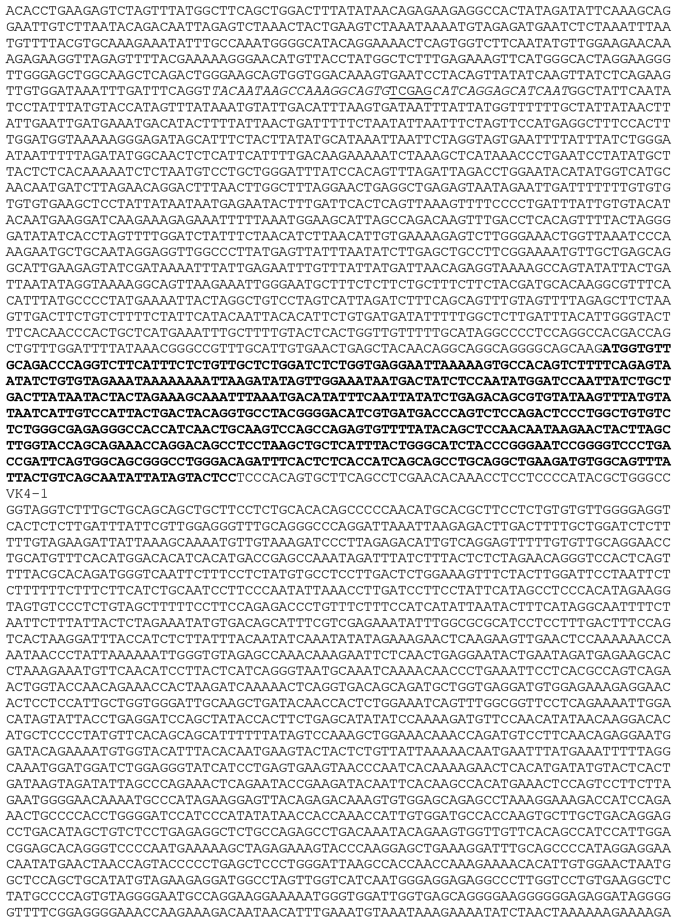

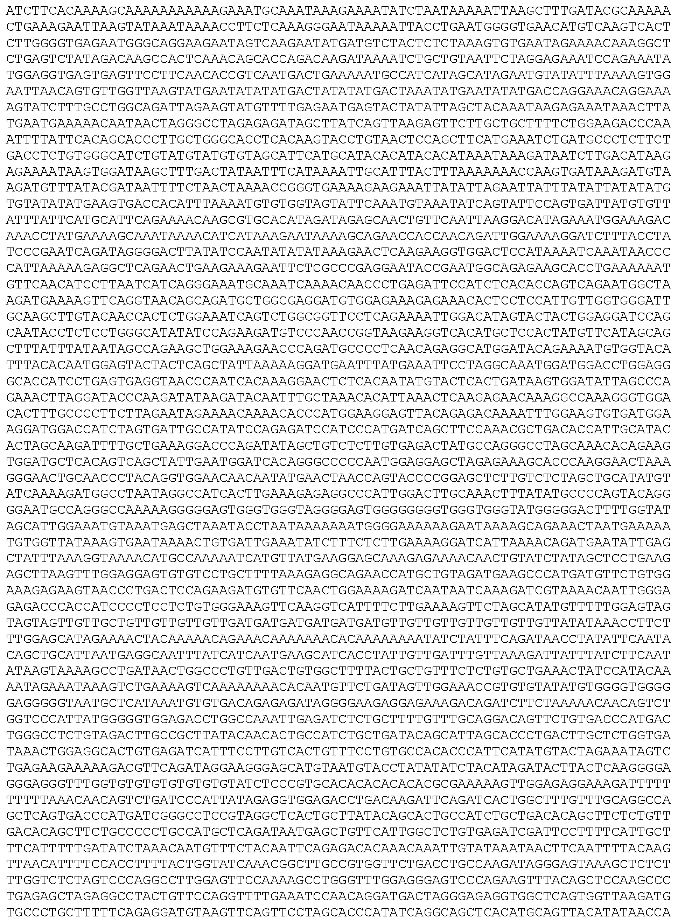

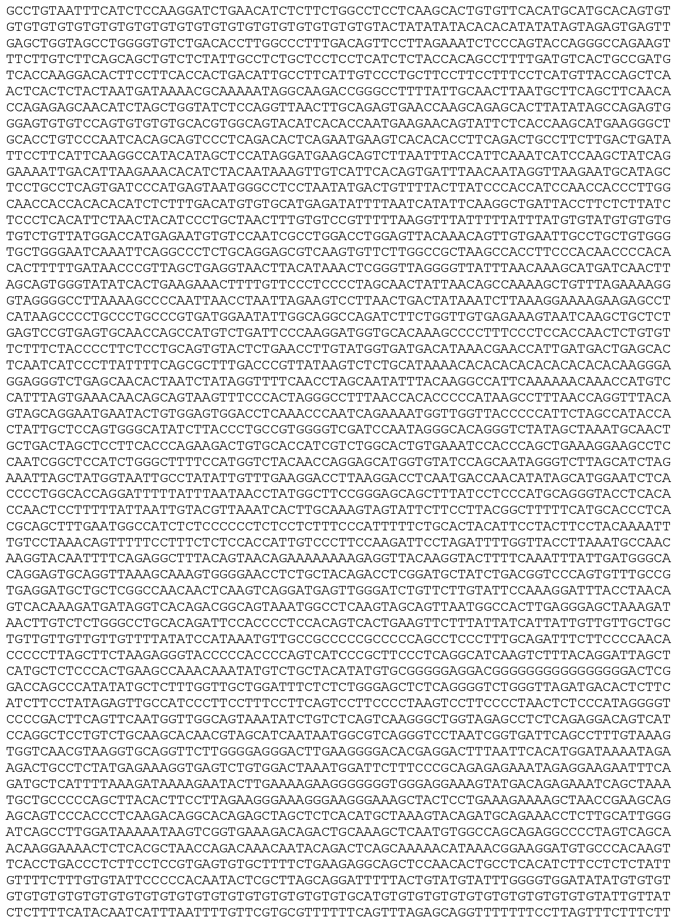

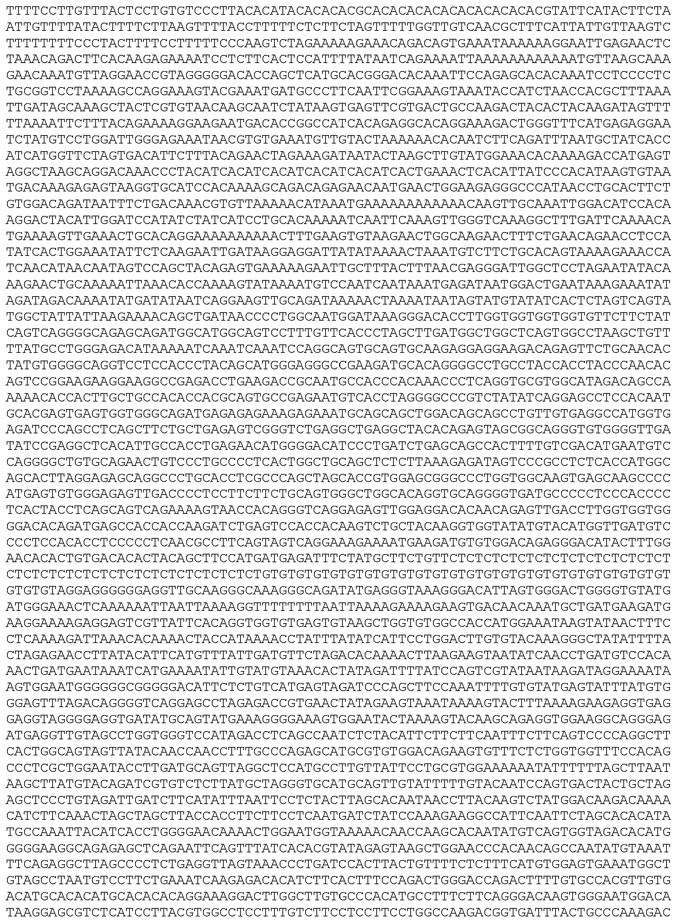

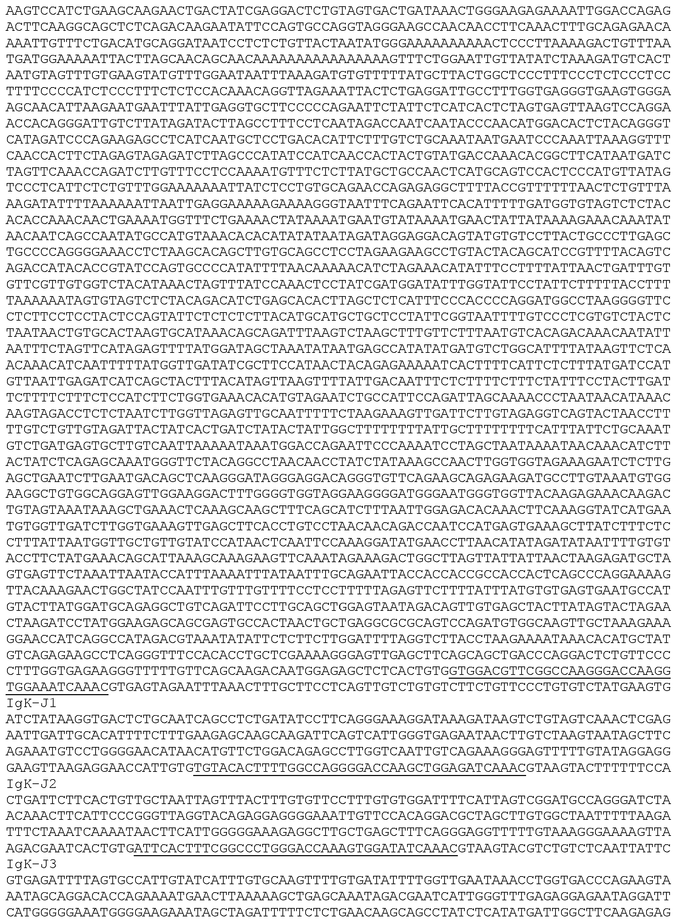

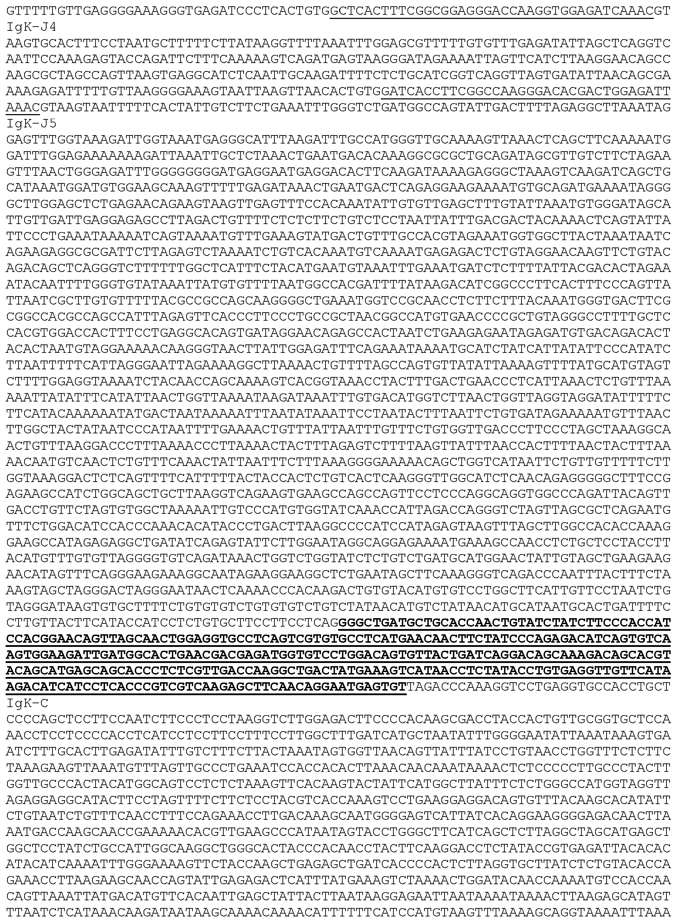

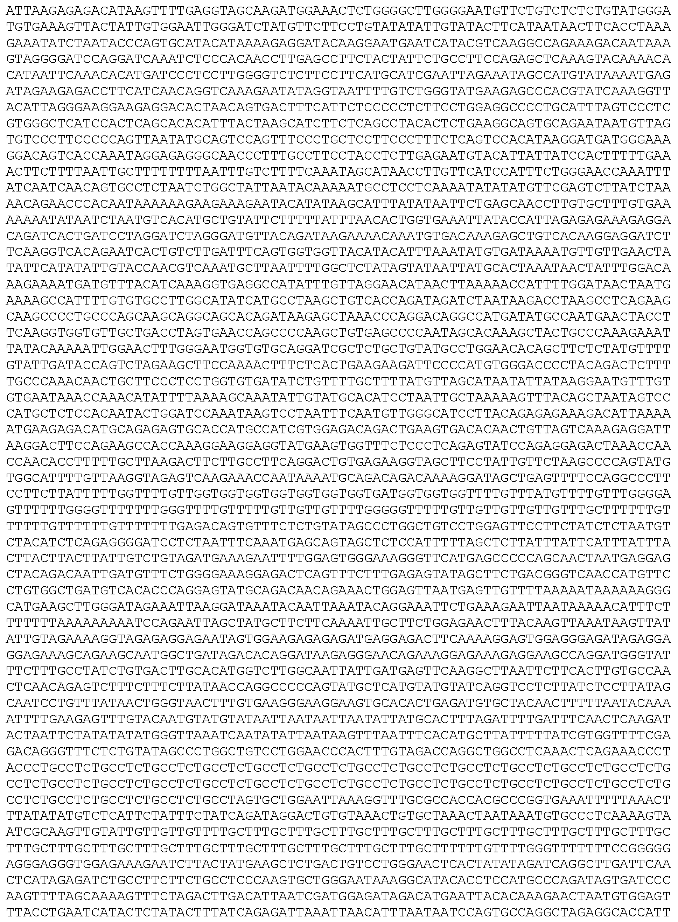

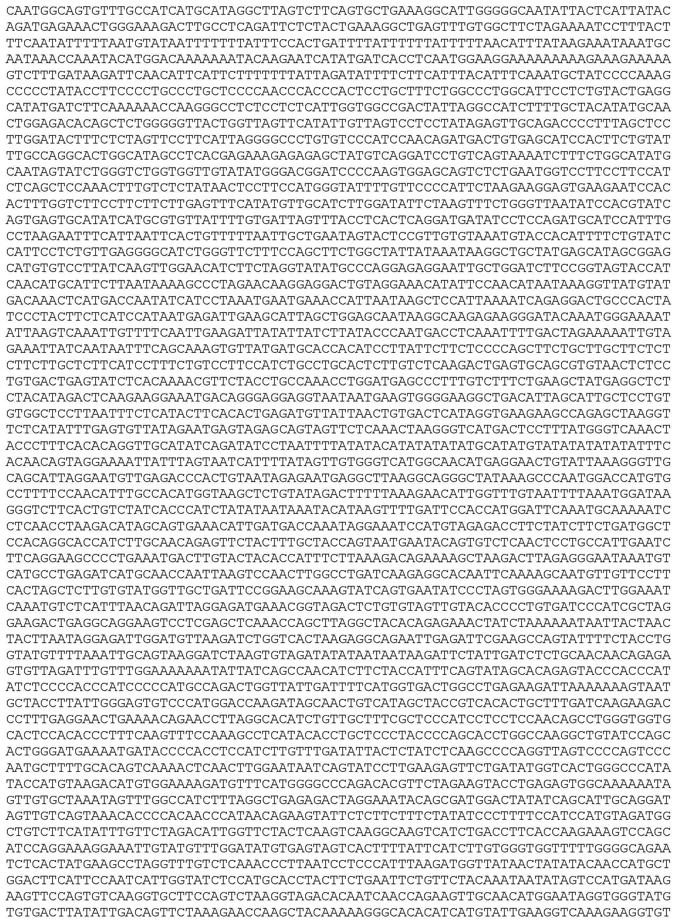

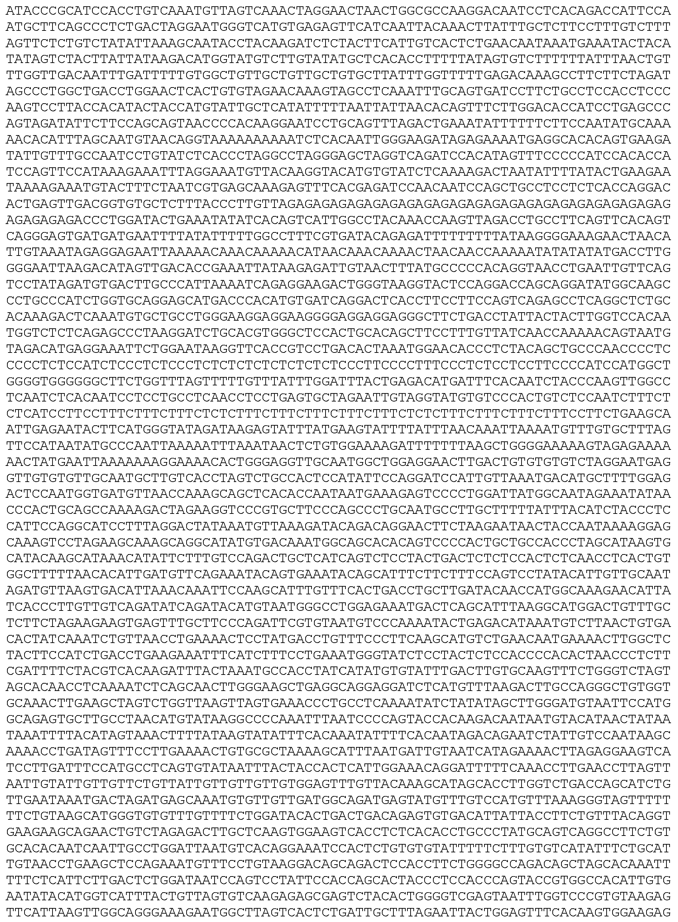

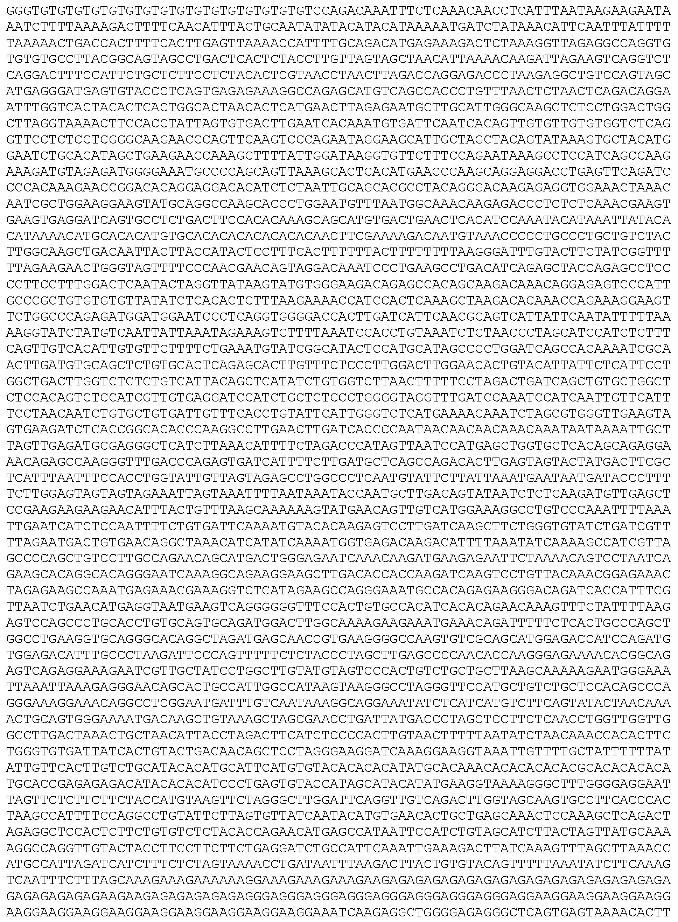

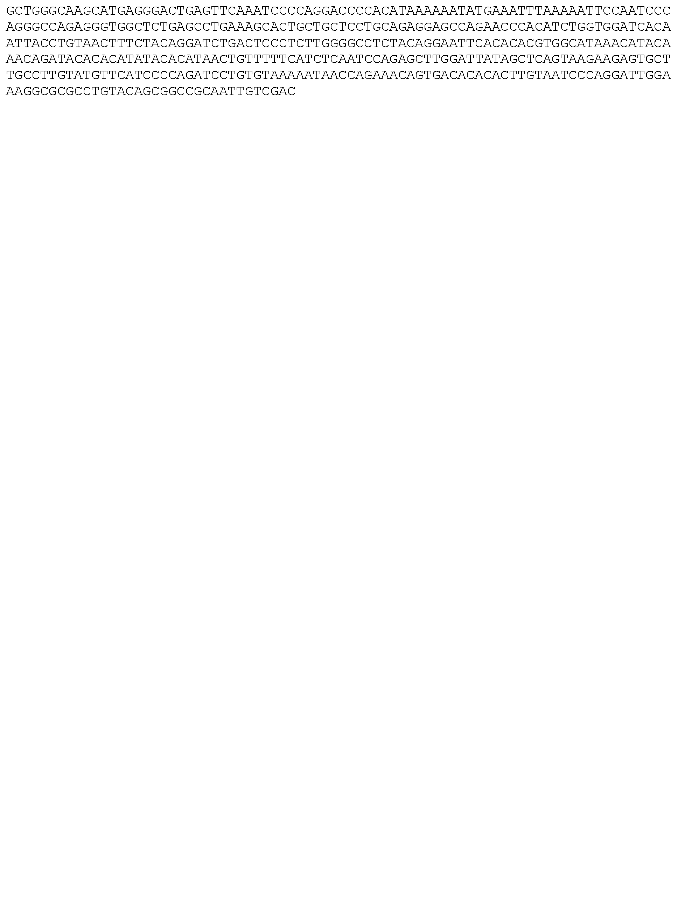

**Figure 5** depicts a Kappa light chain locus sequence (SEQ ID NO:2).
   The sequences highlighted in bold are the amino acid coding and intron sequences of the VL segments. Underlined between these are the junction sequences. Each of these is flanked by a sequence in italics that signals the beginning and end of each VL segment (coding plus flanking sequences). The J segments are underlined; the constant regions are in bold and underlined.

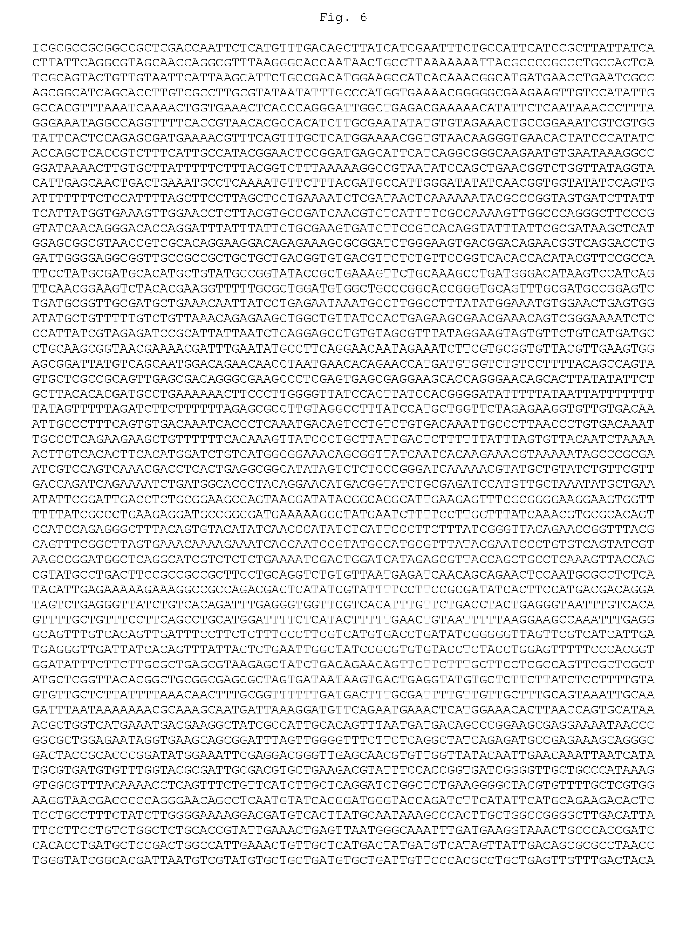

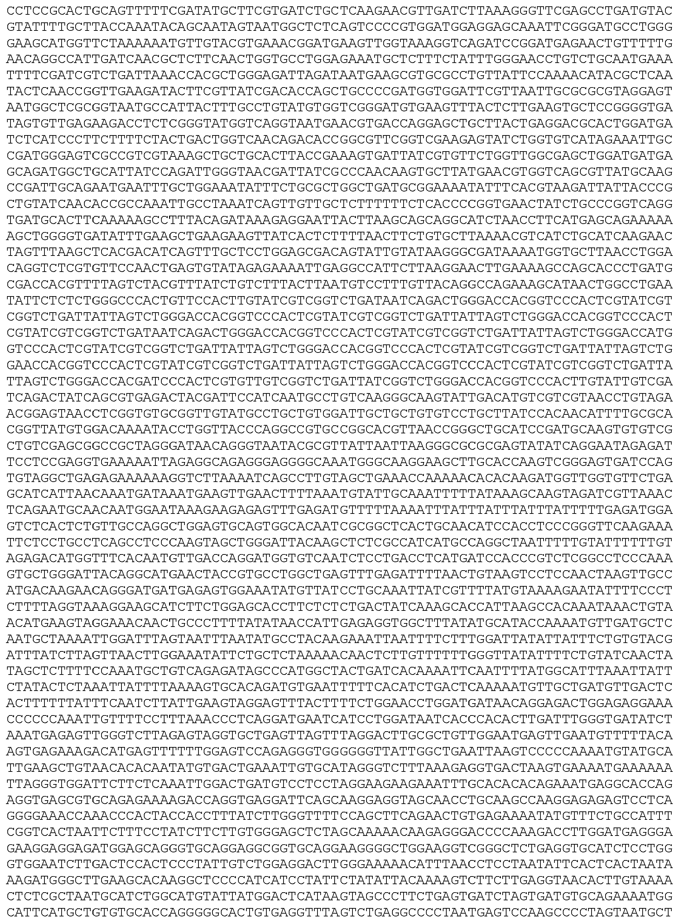

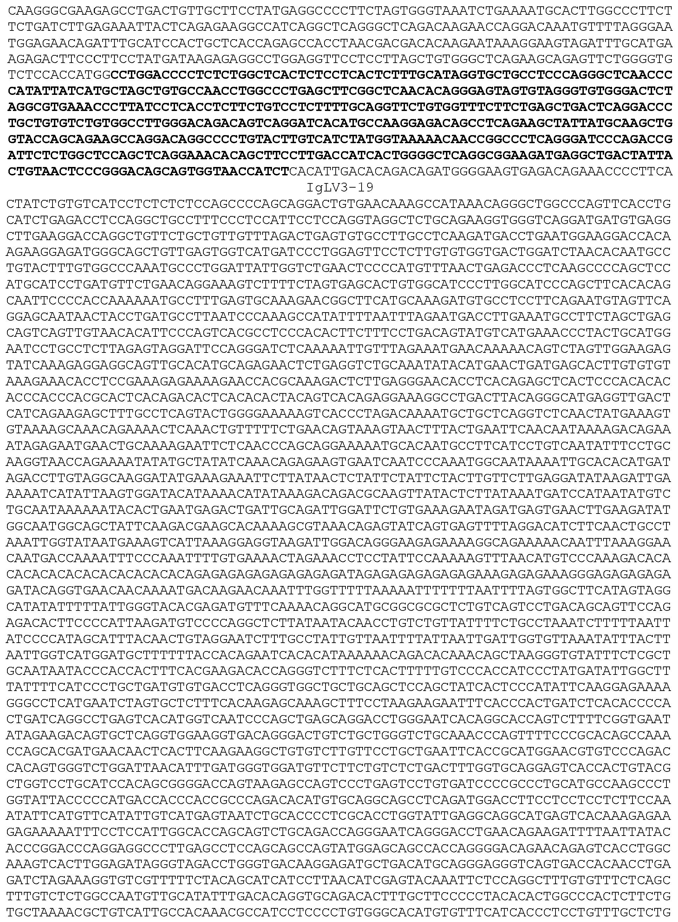

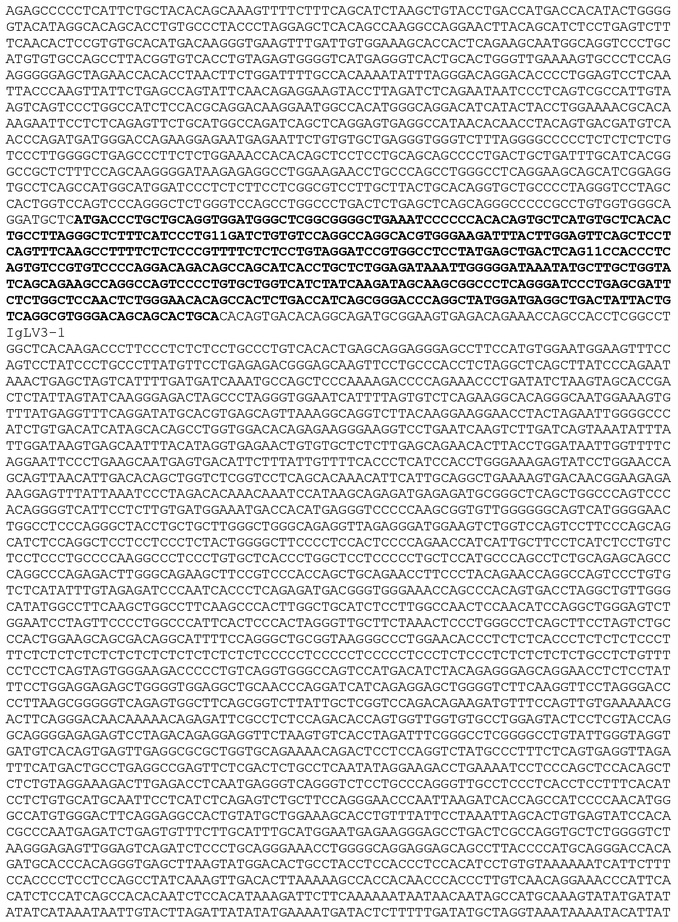

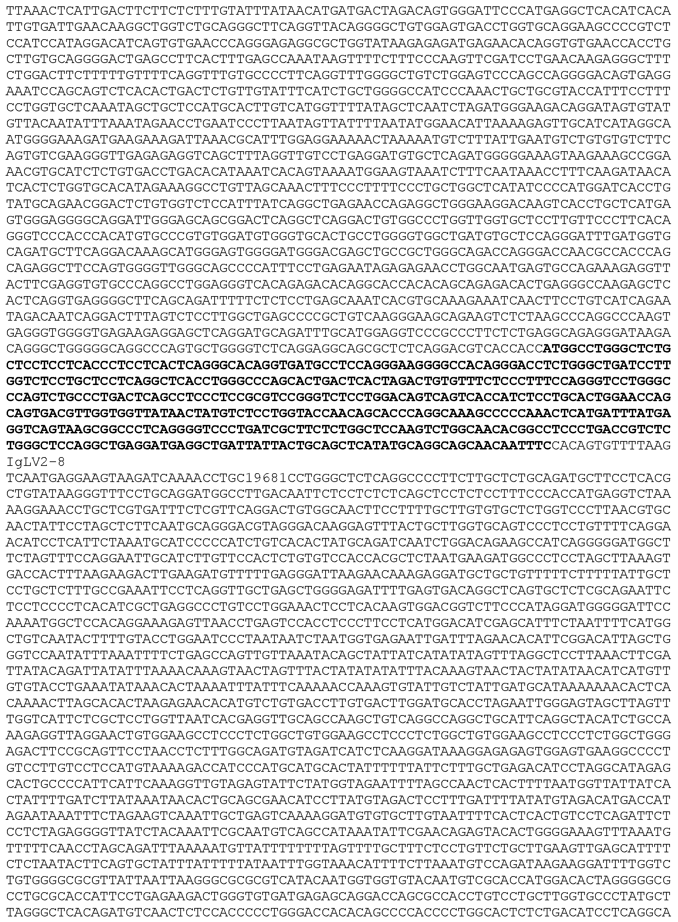

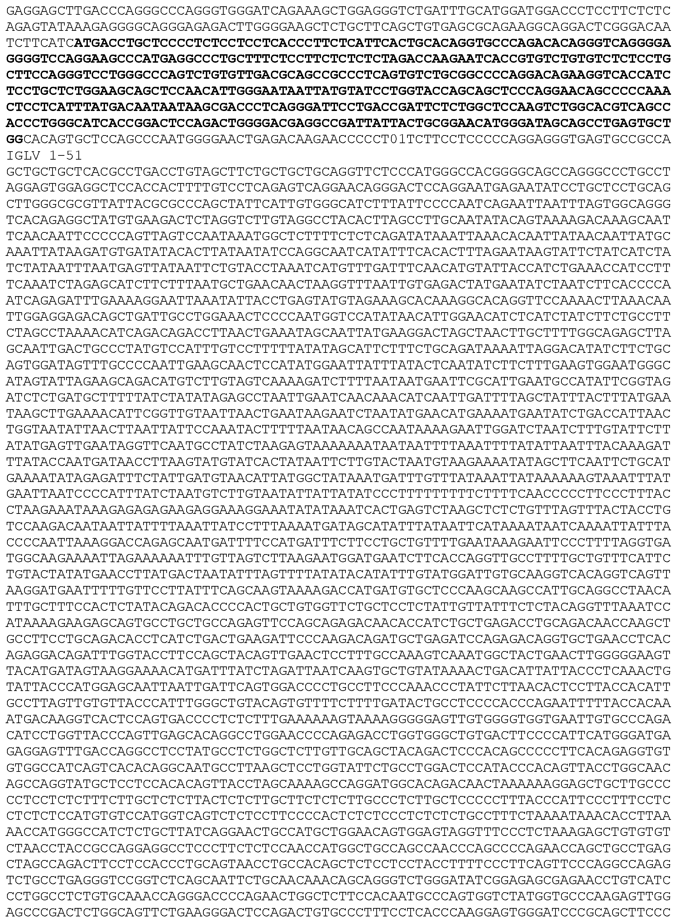

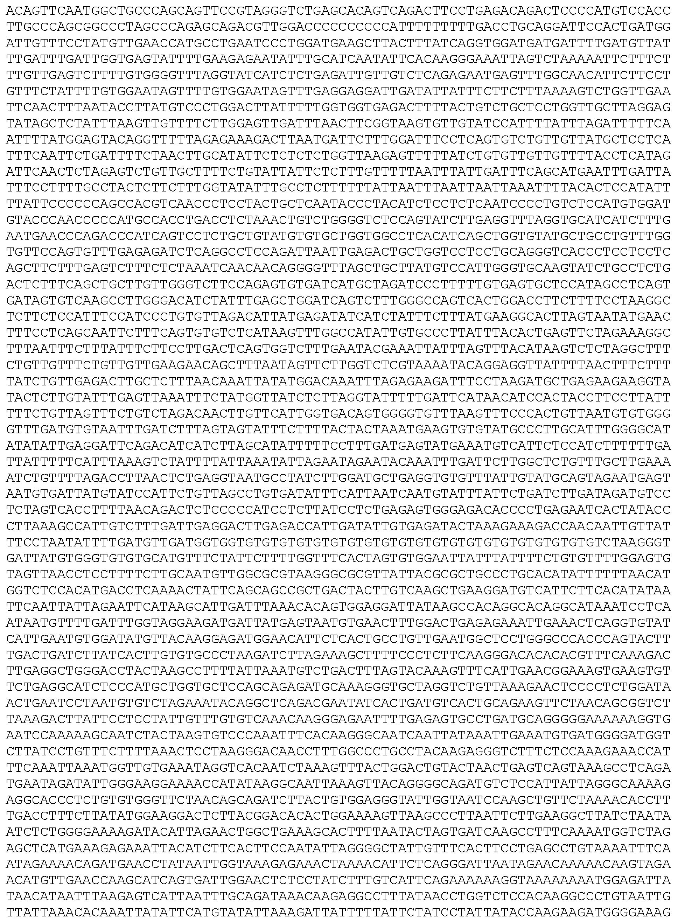

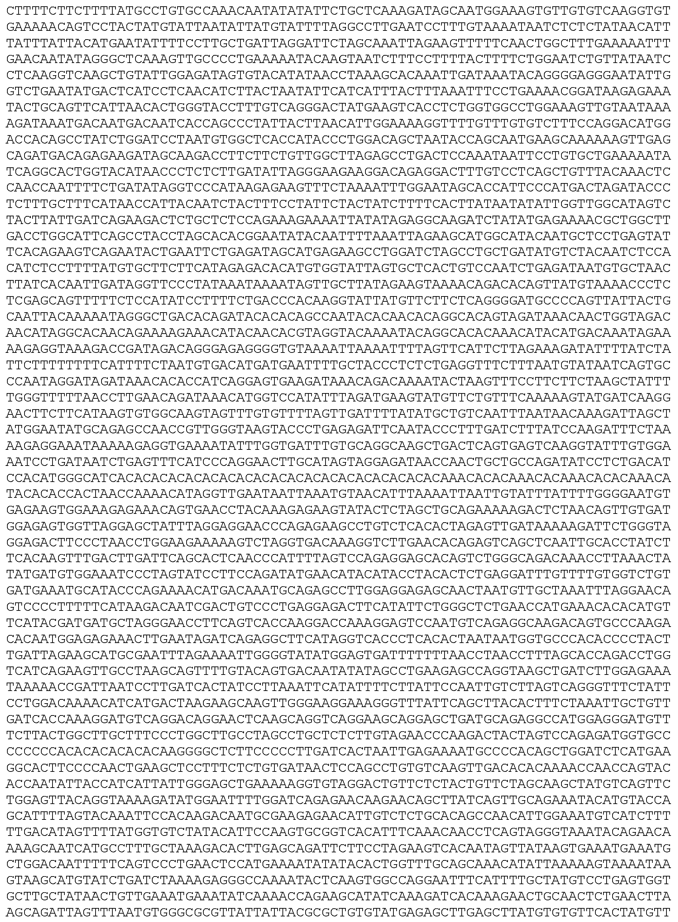

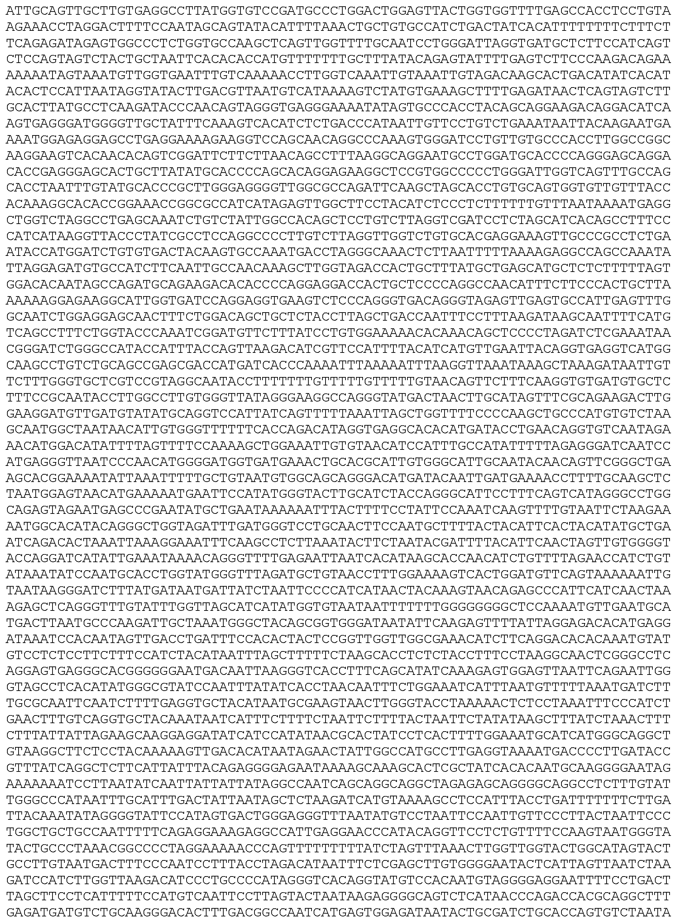

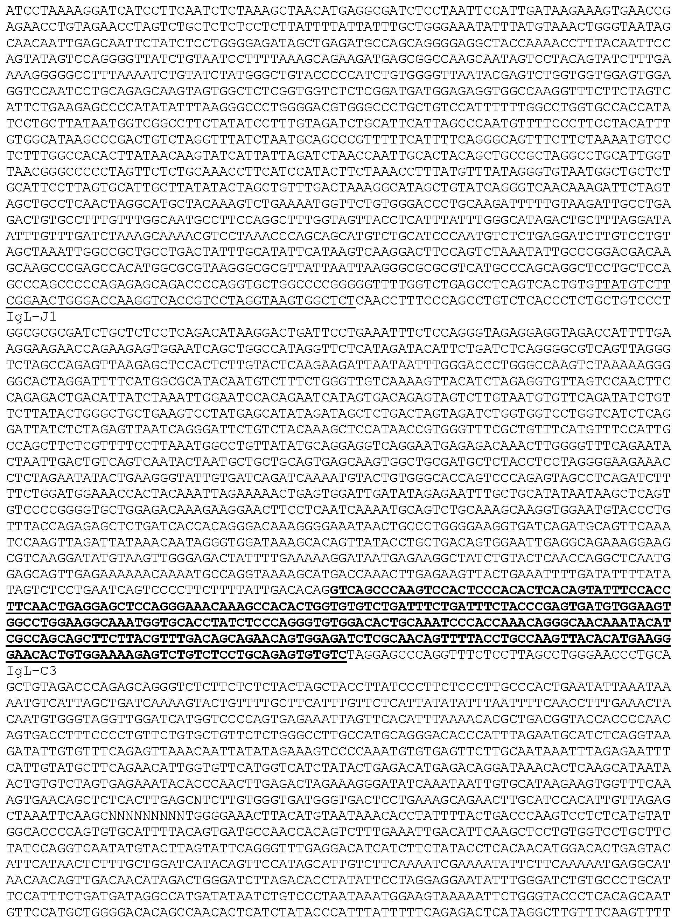

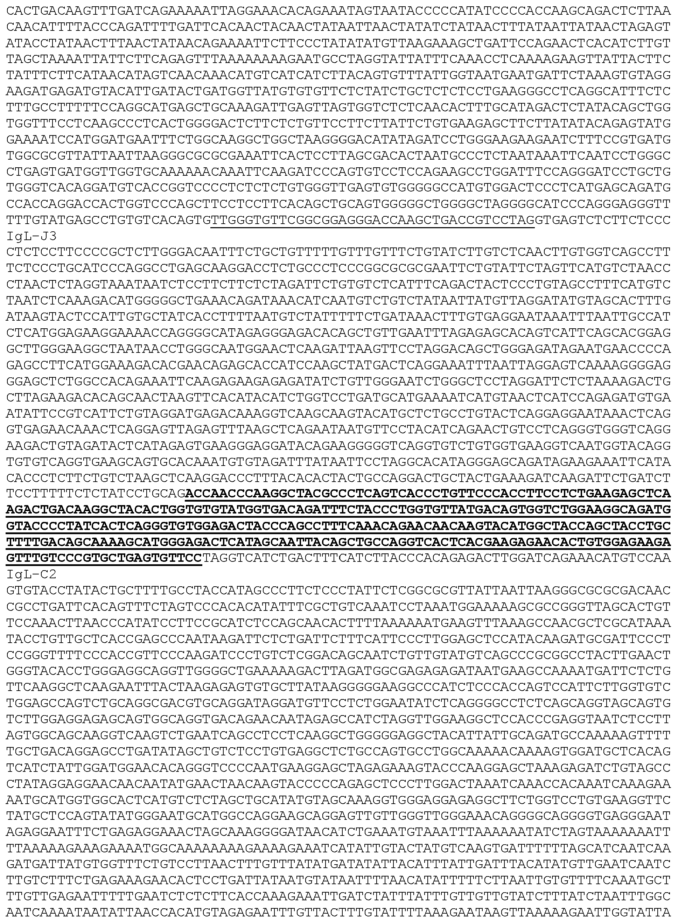

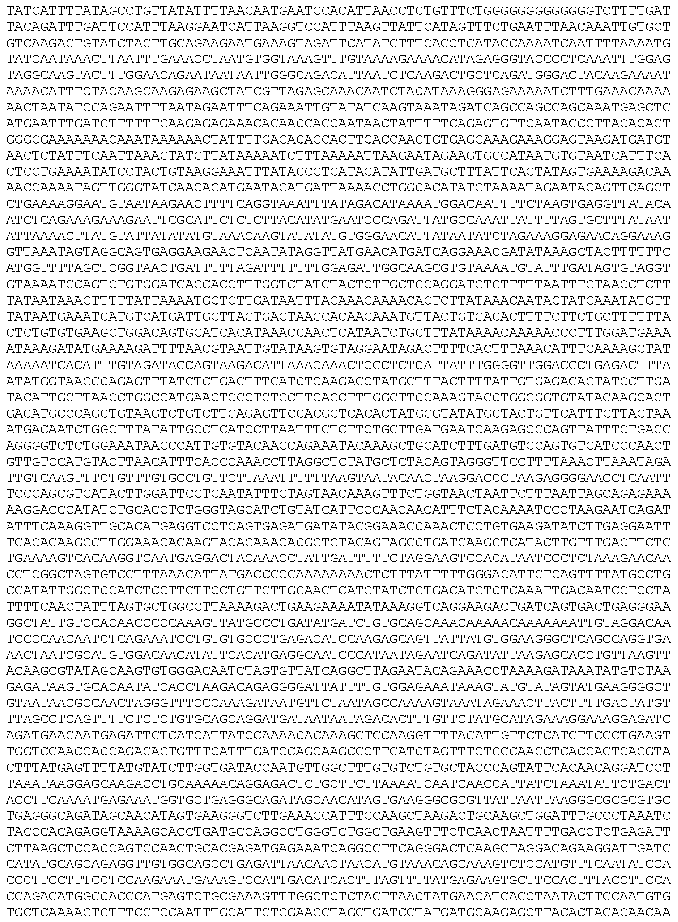

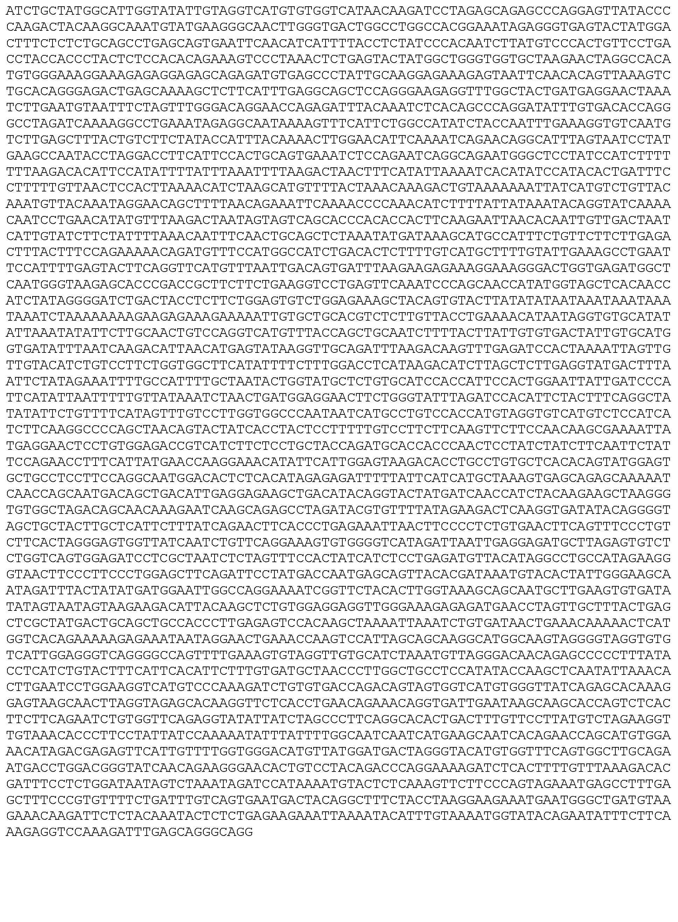

**Figure 6** depicts a Lambda light chain locus sequence (SEQ ID NO:3).
   The sequences highlighted in bold are the amino acid coding and intron sequences of the VL segments. The J segments are underlined; the constant regions are in bold and underlined.
**Figure** 7 depicts a strategy to disable mouse IgH.
   The top line shows the Cµ region of the mouse with the different exons including the two exons coding for the membrane form of IgM. To the left are the J, D and V_{H} region of the locus, to the right the other constant regions starting with Cδ. The bottom lines show part of the amino acid sequence of the normal M1 exon after recombination. The DNA sequence shows the integration sequence. The stop codon is boxed, the Spe I site is highlighted with a double line.

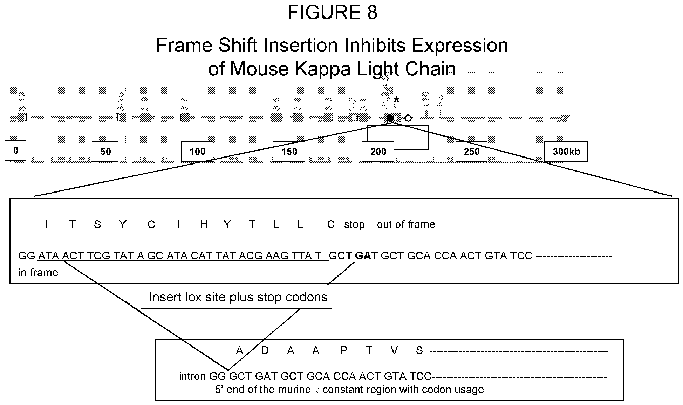

**Figure 8****:** depicts a mouse C_{κ} insertion to disable the κ locus.
   The locus is shown on the top line. The bottom shows the sequence at the 5' end of the C_{κ} exon (which is marked with an asterisk in top line) with the amino acid coding written above the bases. The GG base pair at the start is immediately flanking the splice acceptor site coding for the amino acid R after splicing. The middle line shows the insertion of a 34 basepair lox site insertion (underlined and double underlined inverted repeat sequence), which puts the codon usage of the constant region out of frame and creating downstream stop codons (e.g. TGA bold print underlined). Black circle κ-enhancer and white circle κ-LCR sequences.

### DETAILED DESCRIPTION OF THE INVENTION

In the context of the present invention, the term 'heterologous' means a nucleotide sequence or a locus as herein described which is not endogenous to the mammal in which it is located.

The transgenic mouse mammal may thus comprise a transgene comprising a heterologous immunoglobulin kappa light chain locus and associated B-cell specific regulatory elements, preferably comprising an LCR and/or a transgene comprising a heterologous lambda light chain locus and associated B-cell specific regulatory elements, preferably comprising an 89LCR.

The presence of cognate LCRs is not essential for B-cell specific expression. Their inclusion within loci ensures that high level transgene expression occurs at every site of integration and is not dependent on random integration events, only some of which fortuitously occur within chromatin regions actively transcribed in B-cells. The use of cognate LCRs significantly reduces the number of transgenic animals required to be screened for antibody expression and allows the insertion of more than one gene locus, with the certainty that all loci inserted will be expressed at essentially normal levels in a B-cell specific manner. Thus, the use of LCR technology, combined with the surprising observation that allelic exclusion mechanisms will discriminate between endogenous immunoglobulin genes and multiple competing transgenes, opens the way for the assembly of transgenic non-human mammals comprising one or more immunoglobulin heavy or light chain gene loci, each locus being of reduced V gene complexity relative to the endogenous genes and comprising a relatively manageable piece of DNA (<300Kb) to assemble *in vitro* relative to the endogenous loci (1-2Mb). For example, the 39 functional human immunoglobulin heavy chain V gene segments may be cloned into two or more immunoglobulin heavy chain loci. Each will comprise different V gene segments, but have in common D and J gene segments, and constant (effector) regions. The inclusion of the LCR ensures that each is expressed in an identical manner, irrespective of the site of integration within the genome. Thus, the inclusion of two or more small loci in this manner provides the same V gene complexity of a single, more complex gene present in a single, large gene fragment which is technically difficult to manipulate.

Each heterologous light chain locus may comprise V_{L} gene segments, J gene segments and a light chain constant region segment. Preferably, the V_{L} and J gene segments and light chain constant region segment are derived from the same mammalian source, for example rodent, pig, cow, goat or sheep. Preferably, they are of human origin.

Alternatively, the heterologous light chain loci may be hybrid loci comprising variable domains of mammalian origin, preferably of human origin, and constant (effector) regions from a different mammal, such as, but not limited to, mouse, rat, hamster, rabbit, pig, goat and cow. Where the host mammal is a mouse, preferably the constant regions are of rodent origin, more preferably mouse or rat. Such heterologous light chain loci comprise V_{L} and J segments preferably from one species only and a light chain constant region from another species.

Where hybrid kappa light chain transgenes are contemplated, the V_{L} and J gene segments are preferably from the same species, contributing the heavy chain V, D and J gene segments, and are preferably of human origin. The kappa light chain constant and heavy chain constant regions are also preferably derived from the same species but a species different from that contributing variable domains and are preferably of rodent origin, and preferably derived from rat or mouse.

A feature of all light chain transgenes contemplated is that, following antigen challenge, the light chain rearranges in a B-cell specific manner and that, following transcription and translation, the resulting light chain is capable of complexing with transgene-derived heavy chain immunoglobulin produced in the same B-cell. The productive expression of immunoglobulin tetramers gives rise to B-cell expansion and transgene-encoded, antigen-specific tetravalent immunoglobulin complexes can accumulate in serum in the absence of significant levels of endogenous immunoglobulin tetramers.

Where endogenous lambda light chain expression has not been functionally suppressed, then low levels of host or hybrid antibody comprising endogenous lambda light chains may be detectable. These may be discarded following screening of hybridoma supernatants.

In humans, there are 36 functional kappa V_{L} gene segments, five J_{L} gene segments and a single kappa light chain constant region (http://imgt.cines.fr).

The transgenic mouse may also comprise a heterologous immunoglobulin lambda light chain locus. A heterologous lambda light chain locus present in a transgene in the transgenic mouse of the invention preferably comprises a murine lambda LCR, and human lambda light chain V1 and V2 gene segments, human lambda J1, J2, J3 and J4 gene segments, and human lambda light chain C1, C2, C3 and C4 constant region segments (WO90/10077 and WO2003/000737). The human lambda light chain V1 and V2 gene segments may comprise 1, 2, 3, or 4 human V_{L} gene segments selected from Vλ3-19, Vλ3-1, Vλ2-8, and Vλ1-51. The human J gene segments comprise Jλ1 and Jλ3. The constant region gene segments comprise Cλ2 and Cλ3. Optionally, the human lambda light chain C1, C2, C3 and C4 constant region segments may be replaced by alternative lambda light chain constant regions, preferably of rat or mouse origin.

The transgenic mouse of the invention comprises a heterologous immunoglobulin heavy chain locus as defined in claim 1.

The locus also may comprise one or more human constant (effector) regions, preferably the µ and γ constant regions. Optionally, the human constant effector regions may be replaced by effector regions from other non-human mammals. Where the non-human mammalian host is a mouse or a rat, then preferably constant (effector) regions are derived from rat or mouse. The rat constant region gene segments may comprise Cµ, Cy2c, Cγ1, Cy2b, and Cα. In contrast with human, the transmembrane domains of the mouse and rat B-cell receptor complex (BCR) are 100% conserved. Thus, mice transgenic for antibody loci comprising rat constant (effector) region genes should function as well as those comprising mouse constant (effector) region genes following antigen challenge, and may be superior to those comprising human constant (effector) region genes (De Franco et al. (1995) Ann. NY Acad. Sci., 766, 195-201). The transgenes may comprise heavy chain, kappa and lambda light chain LCRs, preferably of mouse or human origin. LCRs which function across all mammalian species are known and may be substituted for human or mouse LCRs in the transgenes (Li et al. (1999) Trends Genet., 10, 403-8).

Where the generation of fully human antibodies is contemplated, cloned human antigen-specific V_{H} and V_{L} binding domains derived from hybrid antibodies expressed by hybridomas can be fused to human constant heavy and light chain constant regions, so deriving fully human tetrameric antibodies of any class.

As a further refinement, each immunoglobulin kappa and/or lambda light chain locus may also comprise a dominant selective marker gene.

The dominant selective marker genes incorporated in the loci may have the same or different mechanisms of action. For the purposes of the invention, any dominant selective marker gene can be used, provided that expression of the gene confers a selective benefit to hybridomas or transformed B-cells derived from the non-human transgenic mammal in the presence of a selective or toxic challenge. Typically, the dominant selective marker genes will be of prokaryotic origin and will be selected from a group which either confer resistance to toxic drugs, such as puromycin (Vara et al. (1986) NAR, 14, 4617-4624), hygromycin (Santerre et al. (1984) Gene, 30, 147-156) and G418 (Colbere-Garapin et al. (1981) 150, 1-14), or comprise genes which obviate certain nutritional requirements such that their expression converts a toxic substance into an essential amino acid, for example the conversion of indole to tryptophan or the conversion of histidinol to histidine (Hartmann and Mulligan, (1988) PNAS, 85, 8047-8051).

A necessary requirement of this aspect is that the dominant selective marker is incorporated within the immunoglobulin light chain transgenic locus and is co-expressed with the desired immunoglobulin light chain allele, so ensuring B-cell specific expression. Alternatively, the drug resistance gene maybe inserted into an endogenous or exogenous (transgenic) immunoglobulin locus using homologous recombination in combination with ES cells or nuclear transfer approaches (te Riele et al. (1992), PNAS, 89, 11, 5128-5132).

The transgenic mouse may also comprise a transgene or transgenes comprising a heterologous heavy chain locus and associated B-cell specific LCR and regulatory elements. More than one different transgenic heavy chain gene locus may be present, each comprising an LCR and regulatory elements.

The heavy chain gene loci, each comprising one or more V gene segments, one or more D gene segments, one or more J gene segments, and one or more constant (effector) regions are introduced as transgenes, each locus comprising a cognate LCR. Each locus comprises the 5' and 3' regulatory elements necessary to drive B-cell specific expression. Each heavy or light chain locus is expressed in an essentially identical manner to the endogenous loci in response to antigen challenge, leading to the circulation in mouse serum of transgene-encoded, antigen-specific affinitymatured, tetrameric immunoglobulins.

Preferably, each heavy chain gene locus comprises 18 V gene segments are present on any single heavy chain locus.

In one aspect, a number of V gene segments are present and each V gene segment is different from all other V gene segments. In this aspect, the V gene segments in any one locus may all be derived from an organism of the same species, e.g. all V gene segments may be of human origin. Alternatively, the V gene segments in any one locus may be derived from organisms of different species, e.g. some V gene segments from human and others from sheep, cattle, rabbits, camelids or even shark. In a second alternative, each V gene segment is identical to all the other V gene segments. Irrespective of the number and nature of the V gene segments present, the remaining D and J gene segments in each locus may be the same as or may be different from those in all the other loci.

It is thus envisaged that the transgenic mouse may contain multiple copies of a heavy chain gene locus. This has the advantage of optimising the chances that a productive re-arrangement in a B-cell will take place, thus allowing the optimal production of an immunoglobulin heavy chain for antigen recognition.

In another aspect, each locus comprises multiple V gene segments.

Preferably, the V gene segments are of human origin.

The term 'V gene segment' encompasses any naturally occurring V gene segment derived from a vertebrate, including, but not limited to, sharks, rodents, camelids and human. The V gene segment must be capable of recombining with a D gene segment, a J gene segment and a gene segment encoding a heavy chain constant (effector) region to generate an immunoglobulin heavy chain antibody capable of complexing with either a kappa or lamdba immunoglobulin light chain when the re-arranged nucleic acid is expressed in B-cells.

A V gene segment also includes within its scope any gene sequence encoding a natural or engineered homologue, derivative or protein fragment which is capable of recombining with a D gene segment, a J gene segment and a gene segment encoding a heavy chain constant region to generate an immunoglobulin heavy chain antibody capable of complexing with either a kappa or lambda immunoglobulin light chain when the re-arranged nucleic acid is expressed in B-cells. A V gene segment may, for example, be derived from a T-cell receptor locus.

Preferably, the multiple heavy chain loci comprise any number or combination of the 39 functional human V gene segments and engineered variants thereof. These may be on any number of loci, e.g. four loci comprising eight V gene segments plus one locus comprising seven V gene segments; seven loci comprising four V gene segments plus one locus comprising three V gene segments; or thirty-nine loci comprising one V gene segment each.

Human V genes are classified into seven families, V_{H}1 to V_{H}7, and the individual genes within each family numbered. The frequency at which each gene is used is dependent on the varying requirements of the particular immune response. For example, the genes of family V_{H}3 may be preferentially used in comparison to those of family V_{H}5 when responding to bacterial antigens. Therefore, in a further aspect, groups of V gene segments which have been shown to be useful for generating an antibody response against specific antigens are grouped into separate loci, each comprising a different dominant selective marker gene. The V gene segments may be grouped according to family or they may be grouped according to individual function. For example, if the V genes of family V_{H}3 are shown to be useful for generating an immune response against bacterial antigens, then these may be used to generate a locus which is particularly useful for generating heavy chain-only antibodies against bacterial antigens. Alternatively, if it is shown that several individual genes from families V_{H}3 and V_{H}5 are useful for generating an immune response against bacterial antigens, then these may be grouped together and used to generate a locus which is particularly useful for generating antibodies against bacterial antigens.

An "immunoglobulin heavy chain locus" in the context of the present disclosure relates to a minimal micro-locus encoding a V_{H} domain comprising one or more V gene segments, one or more D gene segments and one or more J gene segments, operationally linked to one or more gene segments encoding heavy chain constant (effector) regions. Preferably, the primary source of antibody repertoire variability is the CDR3 region formed by the selection of V, D and J gene segments and by the V-D and D-J junctions.

The advantage of the present invention is that antibody repertoire and diversity obtained in the rearranged V, D and J gene segments can be maximised through the use of multiple immunoglobulin heavy chain gene loci in the same transgenic non-human mammal by exploiting allelic exclusion. The process of allelic exclusion, which randomly chooses one of the loci to start recombination, followed by the next locus if the first recombination was non-productive, etc., until a productive recombination has been produced from one of the loci, would ensure that actually all the V gene segments present in the combined loci would be part of the overall recombination process.

The immunoglobulin locus in its normal configuration appears to have a three dimensional folded structure based on distance measurements made in B cells and measuring in the direction of and through the VH region (Jhunjhunwala et al. (2008) Cell, 133, 265-279). Such a folded or looped structure explains why different V_{H} region can be used equally efficiently even when they are arranged at very different distances from the D, J and constant region of the immunoglobulin heavy chain locus. It has also recently become clear that a folded structure formed by looping in a number of loci is mediated through a particular chromatin binding protein called CTCF. CTCF appears to be directly involved in the formation of chromatin looping as demonstrated by mutagenesis experiments (Splinter et al. (2006) Genes Dev., 20, 2349-2354). More recently it has been shown that cohesin, the protein complex that holds sister chromatids together, is present at CTCF binding sites (Wendt et al. (2008) Nature, 451, 796-801). The inclusion of a number of CTCF sites from the immunoglobulin V_{H} region (Kim et al. (2007) Cell, 128, 1231-1245; Denger, Wong, Jankevicius and Feeney (2009) J. Immunol., 182, 44-48) increases the probability that the V_{H} region of a transgenic immunoglobulin heavy chain locus can be folded properly and allow efficient usage of all the different V gene segments present in that locus. CTCF binding sites are present 3' to a number of the human VH gene segments used in the examples below. Thus, including the 3' sequence immediately flanking these segments in the locus also includes CTCF binding sites.

Each transgene comprising a heterologous heavy chain locus may further comprise a dominant selective marker. Preferably, the dominant selective marker is different from the dominant selective marker introduced within the kappa or lambda light chain loci.

For the purpose of the invention, any dominant selective marker gene can be used, provided that expression of the gene confers a selective benefit to hybridomas or B-cells derived from the non-human transgenic mammal in the presence of a selective or toxic challenge. Typically, the dominant selective marker genes will be of prokaryotic origin and will be selected from a group which either confer resistance to toxic drugs, such as puromycin (Vara et al. (1986) NAR, 14, 4617-4624), hygromycin (Santerre et al. (1984) Gene, 30, 147-156) and G418 (Colbere-Garapin et al. (1981) 150, 1-14), or comprise genes which obviate certain nutritional requirements such that their expression converts a toxic substance into an essential amino acid, for example the conversion of indole to tryptophan or the conversion of histidinol to histidine (see Hartmann and Mulligan (1988) PNAS, 85, 8047-8051).

A necessary requirement of the disclosure is that the dominant selective marker(s), if used, reside within the immunoglobulin heavy chain transgenic loci, so ensuring B-cell specific co-expression. Alternatively, the drug resistance gene maybe inserted into an endogenous or exogenous (transgenic) immunoglobulin locus using homologous recombination in combination with ES cells or nuclear transfer approaches (e.g. te Riele, Robanus Maandag and Berns (1992), PNAS, 89, 11, 5128-5132).

The same dominant selective marker gene may be incorporated within all heavy chain loci. Alternatively, different heavy chain loci or groups of heavy chain loci may comprise different dominant selective marker genes.

Hybridomas or B-cells, preferably long-lived plasma cells (Slifka et al (1998) Immunity, 8, 363-372), derived from transgenic mice of the invention expressing tetrameric antibodies may be selected for, free of cells expressing endogenous immunoglobulin, due to the co-expression of a functional dominant selective marker gene within the transgenic light chain loci. Furthermore, hybridomas or transformed B-cell lines or cultured B cells expressing antibodies derived from specific groups of V segments present on transgenic heavy chain loci may also be selected for due to the presence and co-expression of different dominant selective markers within heavy chain loci relative to the dominant selective markers incorporated within the light chain loci. For example, the inclusion of a puromycin resistance gene within the kappa light chain transgenic locus would allow selection of all cells expressing the kappa light chain transgene. Alternatively, the inclusion of the G418 resistance gene within a heavy chain transgenic locus comprising preferred V gene segments would allow the selection of all cells expressing the preferred V gene segments.

In the invention, the endogenous mouse heavy chain locus and the endogenous mouse kappa light chain locus are disabled. Strategies for disabling these endogenous loci are known in the art and described in the examples herein.

In particular, the invention provides a method of producing an antigen-specific heterologous hybrid monoclonal antibody comprising:
(a) immunising a transgenic mouse of the invention as described above with the antigen;
(b) preparing hybridomas or B cells, plasmablasts, memory B-cells or plasma cells each of which produces a monoclonal antibody from the B-cells of the immunised transgenic mouse;
(c) selecting at least one hybridoma or B cell, plasmablast, memory B-cell or plasma cell expressing the heterologous antibody by use of the dominant selective marker genes present in the transgenes comprising the heterologous immunoglobulin light chain and heavy chain loci; and
(d) selecting at least one hybridoma or B cell, plasmablast, memory B-cell or plasma cell which produces an antibody which binds specifically to the antigen.

The invention is now described, by way of example only, in the following detailed description with reference to the following Figures.

### EXAMPLES

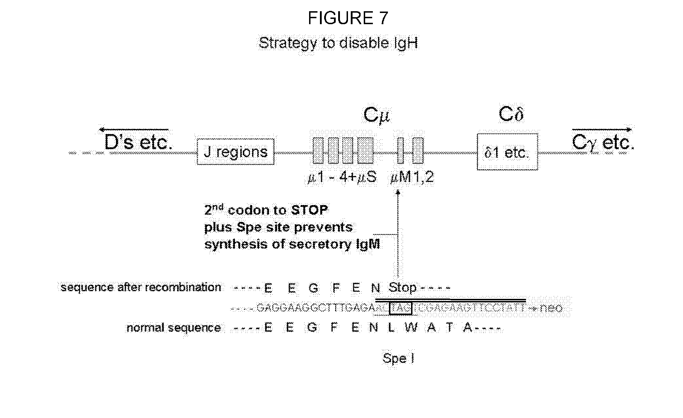

In the following examples, transgenic mice are generated that express hybrid human/rat heavy chain and light chain loci as transgenes introduced by microinjection in fertilised eggs, a routine transgenesis procedure. The egg-donating mice can be modified to have no or very low expression of the endogenous mouse heavy chain genes and mouse light chain genes. For example, the mice can be µMTE mice as depicted in Figure 7, obtained by homologous recombination of ES cells. The IgH locus can be inactivated by a strategy similar to that published by Kitamura and Rajewsky with the difference being that the stop codon is introduced into the C_{µ} regions at a position one amino acid before that described by Kitamura et al. (1991) Nature, 350, 423-426. Additionally, the mice can have the κ light chain locus inactivated as depicted in Figure 8. The lambda light chain locus can also be inactivated, although it should be noted that the mouse lambda locus is used very infrequently.

Methodology used for the construction of heavy and light chain loci, the generation and screening of transgenic mice following antigen challenge are essentially as previously described (Janssens et al. (2006) PNAS, 10, 103(41), 15130-5, WO2006/008548, WO2007/096779, , WO2009/13620, WO2010/070263 and WO2010/10965) excepting that the C_{H}1 domain is retained in all heavy chain loci. General methods for deriving vertebrates, including mammals, other than mice, which express functional heterologous immunoglobulin loci and/or have engineered endogenous loci are as described in WO2006/047367. In the examples below, recombination in ES cells is used and the modified ES cells are used to generate mice with the desired properties. However, the same procedures could be carried out in induced pluripotent stem cells (iPS cells) which are then used to generate mice (e.g. Boland, Hazen, Nazor, Rodriguez, Gifford, Martin, Kupriyanov and Baldwin (2009), 461, 7260, 91-4 and references therein). Alternatively, the modifications are carried out in somatic cells or somatic stem cells which are subsequently reprogrammed into iPS cells to generate modified mice. Also, modified hematopoietic stem cells could be transplanted into recipient mice lacking B cells to generate human or human hybrid antibodies.

### Example 1

The most frequently and moderately frequently used V_{κ} genes of the human Ig_{κ} locus (Figure 1, Vκ2-30, Vκ2-28, Vκ1-5, Vκ1-9, Vκ1-27, Vκ1-33, V_{κ}1-39, V_{κ}3-20, Vκ3-15, Vκ3-11, and Vx4-1, assessed using the Ig database; http://imgt.cines.fr/) were amplified by standard PCR and subcloned between XhoI/SalI sites, as described previously for human V_{H} segments. This allows the multimerisation of the V_{κ} regions, keeping the multimer between XhoI and SalI sites.

Also, the 3' end of the mouse κ locus, including the 3' κ enhancer, and the rat constant (C_{κ}) region plus the rat 5' enhancer were cloned together. Next, the human J_{κ} region and the region (17 kb) from between mouse V_{κ} and J_{κ} were cloned in to maintain the normal spacing between V_{κ} regions and J_{κ}. Finally, the human V_{κ} were inserted into the PAC by routine procedures (e.g. Janssens et al. (2006), *supra*).

The V_{κ} segments were multimerized and ligated into the PAC vector containing the human J regions and the mouse enhancers and rat C_{κ} regions. This results in a human-rat hybrid locus comprising human V_{κ} segments and a rat constant (C_{κ}) region (Figure 1).

The hybrid loci inserts were subsequently isolated from the plasmid as large DNA fragments and injected into fertilized mouse eggs. All of this was done by routine methods (e.g. Janssens et al. (2006), *supra*).

### Example 2

A hybrid human/rat IgH locus has been generated having 18 human V_{H} segments and 5 rat constant regions (Figure 2; human V_{H} 6-1, V_{H} 1-2, V_{H} 4-4, V_{H} 2-5, V_{H} 3-07, V_{H} 1-8, V_{H} 4-b, V_{H} 3-15, V_{H} 1-18, V_{H} 3-23, V_{H} 3-33, V_{H} 3-30, V_{H} 3-48, V_{H} 4-34, V_{H} 3-49, V_{H} 3-53, V_{H} 4-59, V_{H} 1-69). First, a central 70kb DJ region of the human locus containing 21 D gene segments and all 6 J gene segments was extended at the 5' end with 8 kb from the mouse IgH intron to maintain the proper distance between V_{H} segments and the D region. Next the first V_{H} region (6-1) of 10 kb with an artificial SceI meganuclease site was cloned at the 5' end of the mouse intron sequences. In a separate plasmid, all the remaining V_{H} regions were cloned together by slotting in Xho1/SalI V_{H} segments. The V_{H} multimer was cloned into the VH6-1DJ plasmid, after which the rat constant regions were added to complete the locus (Cµ, Cγ2χ, Cγ1, Cy2b, and Cα and switch regions). These have been amplified by standard long range PCR from rat genomic DNA. Finally, the mouse heavy chain LCR was added. This regulatory sequence was amplified from mouse genomic DNA in three parts, subcloned together to restore the complete LCR and added to the 3' side of the rat constant regions. The resulting hybrid IgH locus thus contains human V, D and J regions and rat constant regions with mouse regulatory sequences.

The hybrid loci inserts were subsequently isolated from the plasmid as large DNA fragments and injected into fertilized mouse eggs. All of this was done by routine methods (e.g. Janssens et al. (2006), *supra*).

### Example 3

The hybrid IgH and hybrid Ig_{κ} transgenic mice were subsequently bred to obtain mice that are positive for the human/rat hybrid IgH and Ig_{κ} expression. These mice were subsequently immunized to generate antigen-specific hybrid human/rat H₂L₂ antibodies by routine procedures.

### Example 4

In this example, the diversity of the human/rat hybrid antibody is increased even further by the addition of a human/rat Igλ locus through breeding to the mice that carry human/rat IgH and/or Igκ loci described in the examples above. The human/rat hybrid λ locus was generated very much as described for the human/rat Igκ locus described in the previous examples (Figure 3, Vλ3-19, Vλ3-1, Vλ2-8, and Vλ1-51). The difference is caused by the fact that Jλ and Cλ regions occur in pairs and hence 2 rat C_{λ} regions were cloned onto 2 human J_{λ} regions (Figure 3). The spacing between the V_{λ} and J_{λ} segments was maintained by cloning the normal mouse sequences that occur in that position. In this example, 2 human J_{λ} and rat C_{λ} segments were used together with four human V_{λ} segments. Together, these cover more than 80% of the human Igλ response. The regulatory sequences (LCR) were derived from the mouse to ensure optimal expression. As described above, the locus is isolated as a restriction fragment and injected into fertilised eggs to generate mice carrying the transgenic λ locus.

In all of these Examples, the complexity of the response will be enhanced even further by adding V segments as part of additional heavy or light chain transgenic loci present in the same mouse. Since all the loci are subject to allelic exclusion (see WO2007/096779), only the preferred rearrangement will be selected *in vivo* following antigen challenge, resulting in B-cell expansion and the accumulation of antibody in serum. The method could also be applied to other species using V segments specific for these species.

In all of these examples V_{H}, V_{L}, D, J and constant regions from different species can be used to generate other single species antibodies or hybrid species antibodies. It will also be apparent to one skilled in the art that, once an antigen-specific antibody has been identified, the V_{H}DJ and V_{L}J regions can be cloned onto alternative constant regions from the same species or from different species by completely routine methods.

The skilled person will appreciate that variations to this procedure may be made to generate the hybrid transgenic mice, such as the use of different vectors, different selection markers, different recombination positions to inactivate the mouse genes or variations in the actual (routine) cloning strategy of the hybrid loci. The same procedure can be used to generate any normal or hybrid locus using immunoglobulin DNA derived from any single mammalian species, or hybrid loci derived using DNA from two or more species.

### LIST OF SEQUENCES

| | |
|---|---|
| SEQ ID NO:1 | Heavy chain locus |
| SEQ ID NO:2 | Lambda light chain locus |
| SEQ ID NO:3 | Kappa light chain locus |
| SEQ ID NO:4 | VH 1-69 |
| SEQ ID NO:5 | VH 4-59 |
| SEQ ID NO:6 | VH 3-53 |
| SEQ ID NO:7 | VH 3-49 |
| SEQ ID NO:8 | VH 4-34 |
| SEQ ID NO:9 | VH 3-48 |
| SEQ ID NO:10 | VH 3-30 |
| SEQ ID NO:11 | VH 3-33 |
| SEQ ID NO:12 | VH 3-23 |
| SEQ ID NO:13 | VH 1-18 |
| SEQ ID NO:14 | VH 3-15 |
| SEQ ID NO:15 | VH 4-b |
| SEQ ID NO:16 | VH 1-8 |
| SEQ ID NO:17 | VH 3-7 |
| SEQ ID NO:18 | VH 2-5 |
| SEQ ID NO:19 | VH 4-4 |
| SEQ ID NO:20 | VH 1-2 |
| SEQ ID NO:21 | VH 6-1 |
| SEQ ID NO:22 | D1-1 |
| SEQ ID NO:23 | D2-2 |
| SEQ ID NO:24 | D3-9 |
| SEQ ID NO:25 | D3-10 |
| SEQ ID NO:26 | D4-11 |
| SEQ ID NO:27 | D5-12 |
| SEQ ID NO:28 | D6-13 |
| SEQ ID NO:29 | D1-14 |
| SEQ ID NO:30 | D2-15 |
| SEQ ID NO:31 | D3-16 |
| SEQ ID NO:32 | D3-17 |
| SEQ ID NO:33 | D5-18 |
| SEQ ID NO:34 | D6-19 |
| SEQ ID NO:35 | D1-20 |
| SEQ ID NO:36 | D2-21 |
| SEQ ID NO:37 | D3-22 |
| SEQ ID NO:38 | D4-23 |
| SEQ ID NO:39 | D5-24 |
| SEQ ID NO:40 | D6-25 |
| SEQ ID NO:41 | D1-26 |
| SEQ ID NO:42 | D7-27 |
| SEQ ID NO:43 | JH1 |
| SEQ ID NO:44 | JH2 |
| SEQ ID NO:45 | JH3 |
| SEQ ID NO:46 | JH4 |
| SEQ ID NO:47 | JH5 |
| SEQ ID NO:48 | JH6 |
| SEQ ID NO:49 | Cµ |
| SEQ ID NO:50 | Igγ2c |
| SEQ ID NO:51 | IgG1 |
| SEQ ID NO:52 | IgG2b |
| SEQ ID NO:53 | IgA |
| SEQ ID NO:54 | VK2-30 |
| SEQ ID NO:55 | VK1-39 |
| SEQ ID NO:56 | VK2-28 |
| SEQ ID NO:57 | VK1-27 |
| SEQ ID NO:58 | VK3-20 |
| SEQ ID NO:59 | VK3-15 |
| SEQ ID NO:60 | VK3-11 |
| SEQ ID NO:61 | VK1-33 |
| SEQ ID NO:62 | VK1-9 |
| SEQ ID NO:63 | VK1-5 |
| SEQ ID NO:64 | VK4-1 |
| SEQ ID NO:65 | IgK-J1 |
| SEQ ID NO:66 | IgK-J2 |
| SEQ ID NO:67 | IgK-J3 |
| SEQ ID NO:68 | IgK-J4 |
| SEQ ID NO:69 | IgK-J5 |
| SEQ ID NO:70 | IgK-C |
| SEQ ID NO:71 | IgLV3-19 |
| SEQ ID NO:72 | IgLV3-1 |
| SEQ ID NO:73 | IgLV2-8 |
| SEQ ID NO:74 | IgLV 1-51 |
| SEQ ID NO:75 | IgL-J1 |
| SEQ ID NO:76 | IgL-C3 |
| SEQ ID NO:77 | IgL-J3 |
| SEQ ID NO:78 | IgL-C2 |

## Claims

1. A transgenic mouse comprising:
(a) an immunoglobulin heavy chain locus that comprises the sequence of SEQ ID NO: 1 and a cognate Locus Control Region (LCR), and
(b)(i) a heterologous immunoglobulin kappa light chain locus comprising human Vκ gene segments, human Jκ gene segments, and a rat constant region gene segment, and a cognate LCR, wherein:
the human Vκ gene segments consist of, in the following order 5' to 3', Vκ2-30, Vκ2-28, V_{κ}1-5, Vκ1-9, Vκ1-27, Vκ1-33, V_{κ}1-39, V_{κ}3-20, Vκ3-15, Vκ3-11, and Vκ4-1,
the human Jκ gene segments comprise all five human Jκ gene segments,
and the constant region gene segment Cκ, and/or
(b)(ii) a heterologous immunoglobulin lambda light chain locus comprising four human Vλ gene segments, two human J gene segments, and two rat constant region gene segments, and a cognate LCR, wherein:
the human Vλ gene segments comprise, in the following order 5' to 3', Vλ3-19, Vλ3-1, Vλ2-8, and Vλ1-51,
the human J gene segments comprise Jλ1 and Jλ3,
and the constant region gene segments comprise Cλ2 and Cλ3,
and wherein the endogenous mouse heavy chain locus and the endogenous mouse kappa light chain locus have been disabled.

2. The transgenic mouse of claim 1, wherein said loci further comprise a selectable marker.

3. The transgenic mouse according to claim 2, wherein more than one transgene is present, and each transgene comprises a different selectable marker.

4. The transgenic mouse of any one of claims 1 to 3, wherein said immunoglobulin lambda light chain locus comprises the sequence of SEQ ID NO:3.

5. A method of producing an antigen-specific heterologous hybrid monoclonal antibody comprising:
(a) immunising a transgenic mouse of any one of claims 1 to 4 with the antigen;
(b) preparing hybridomas or B cells, plasmablasts, memory B-cells or plasma cells each of which produces a monoclonal antibody from the B-cells of the immunised transgenic mouse;
(c) optionally selecting at least one hybridoma or B cell, plasmablast, memory B-cell or plasma cell expressing the heterologous antibody by use of the dominant selective marker genes present in the transgenes comprising the heterologous immunoglobulin light chain and heavy chain loci; and
(d) selecting at least one hybridoma or B cell, plasmablast, memory B-cell or plasma cell which produces an antibody which binds specifically to the antigen.

6. A method of deriving a human antibody from a hybrid antibody comprising:
(a) carrying out the method of claim 5;
(b) selecting at least one hybridoma or B cell, plasmablast, memory B-cell or plasma cell which produces an antibody which binds specifically to the antigen and comprises V_{H} and V_{L} binding domains of the species of choice;
(c) cloning and sequencing the V_{H} and V_{L} domains;
(d) recloning selected sequences comprising the V_{H} and V_{L} binding domain coding sequences with constant effectors domains of choice from the same species; and
(e) co-expressing the recloned sequences encoding heavy and light chain polypeptides of the desired species using an expression vector in a cell type of choice.

7. A method of producing an antigen-specific monoclonal antibody having a human variable region comprising:
(a) immunising a transgenic mouse of any one of claims 1 to 4 with the antigen;
(b) preparing hybridomas from the B-cells of the immunised transgenic mouse after affinity maturation; and
(c) isolating said antibody from said hybridomas.

8. A method of producing an antigen-specific monoclonal antibody having a human variable region comprising:
(a) immunising a transgenic mouse of any one of claims 1 to 4 with the antigen;
(b) isolating cells or tissue expressing an antigen-specific heavy chain-only antibody of interest from said mouse after affinity maturation;
(c) cloning the heavy chain and light locus or loci from mRNA derived from the isolated cell or tissue; and
(d) expressing the antibody.

## Patentansprüche

1. Transgene Maus umfassend:
(a) einen Immunglobulin-Schwerkettenlocus, der die Sequenz von SEQ ID NO: 1 und eine kognate Locus-Kontrollregion (LCR) umfasst, und
(b)(i) einen heterologen Immunglobulin-kappa-Leichtketten-Locus, der humane Vκ-Gensegmente, humane Jκ-Gensegmente und ein Ratten-konstante-Region-Gensegment und eine kognate LCR umfasst, wobei:
die humanen Vκ-Gensegmente in der folgenden Reihenfolge 5' nach 3' aus Vκ2-30, Vκ2-28, Vκ1-5, Vκ1-9, Vκ1-27, Vκ1-33, Vκ1-39, Vκ3-20, Vκ3-15, Vκ3-11 und Vκ4-1 bestehen, die humanen Jκ-Gensegmente alle fünf humanen Jκ-Gensegmente umfassen,
und das konstante-Region-Gensegment Cκ, und/oder
(b)(ii) einen heterologen Immunglobulin-lambda-Leichtkettenlocus umfassend vier humane Vλ-Gensegmente, zwei humane J-Gensegmente und zwei Ratten-konstante-Region-Gensegmente und eine kognate LCR, wobei:
die humanen Vλ-Gensegmente in der folgenden Reihenfolge 5' nach 3', Vλ3-19, Vλ3-1, Vλ2-8 und Vλ1-51 umfassen, die humanen J-Gensegmente Jλ1 und Jλ3 umfassen,
und die konstante-Region-Gensegmente Cλ2 und Cλ3 umfassen,
und wobei der endogene Maus-Schwerkettenlocus und der endogene Maus-kappa-Leichtkettenlocus deaktiviert worden sind.

2. Transgene Maus nach Anspruch 1, wobei die Loci ferner einen selektierbaren Marker umfassen.

3. Transgene Maus nach Anspruch 2, wobei mehr als ein Transgen vorhanden ist und jedes Transgen einen anderen selektierbaren Marker umfasst.

4. Transgene Maus nach einem der Ansprüche 1 bis 3, wobei der Immunglobulin-lambda-Leichtkettenlocus die Sequenz von SEQ ID NO:3 umfasst.

5. Verfahren zur Herstellung eines antigenspezifischen heterologen hybriden monoklonalen Antikörpers, umfassend:
(a) Immunisieren einer transgenen Maus nach einem der Ansprüche 1 bis 4 mit dem Antigen;
(b) Herstellen von Hybridomen oder B-Zellen, Plasmablasten, Gedächtnis-B-Zellen oder Plasmazellen, von denen jede/jeder einen monoklonalen Antikörper aus den B-Zellen der immunisierten transgenen Maus produziert;
(c) gegebenenfalls Auswählen wenigstens einer Hybridomoder B-Zelle, eines Plasmablasten, einer Gedächtnis-B-Zelle oder Plasmazelle, die/der den heterologen Antikörper exprimiert, unter Verwendung der dominanten selektiven Markergene, die in den Transgenen vorhanden sind, die die heterologen Immunglobulin-Leichtkettenund -Schwerkettenloci umfassen; und
(d) Auswählen wenigstens einer Hybridom- oder B-Zelle, eines Plasmablasten, einer Gedächtnis-B-Zelle oder Plasmazelle, die/der einen Antikörper produziert, der spezifisch an das Antigen bindet.

6. Verfahren zum Ableiten eines humanen Antikörpers von einem hybriden Antikörper, umfassend:
(a) Durchführen des Verfahrens nach Anspruch 5;
(b) Auswählen wenigstens einer Hybridom- oder B-Zelle, eines Plasmablasten, einer Gedächtnis-B-Zelle oder Plasmazelle, die/der einen Antikörper produziert, der spezifisch an das Antigen bindet und V_{H}- und V_{L}-Bindungsdomänen der Spezies der Wahl umfasst;
(c) Klonieren und Sequenzieren der V_{H}- und V_{L}-Domänen;
(d) Reklonieren ausgewählter Sequenzen, die die V_{H}- und V_{L}-Bindungsdomäne-Codierungssequenzen umfassen, mit konstanten Effektordomänen der Wahl aus der gleichen Spezies; und
(e) Coexprimieren der reklonierten Sequenzen, die für Schwer- und Leichtketten-Polypeptide der gewünschten Spezies codieren, unter Verwendung eines Expressionsvektors in einem Zelltyp der Wahl.

7. Verfahren zur Herstellung eines antigenspezifischen monoklonalen Antikörpers mit einer humanen variablen Region, umfassend:
(a) Immunisieren einer transgenen Maus nach einem der Ansprüche 1 bis 4 mit dem Antigen;
(b) Herstellen von Hybridomen aus den B-Zellen der immunisierten transgenen Maus nach Affinitätsreifung; und
(c) Isolieren des Antikörpers aus den Hybridomen.

8. Verfahren zur Herstellung eines antigenspezifischen monoklonalen Antikörpers mit einer humanen variablen Region, umfassend:
(a) Immunisieren einer transgenen Maus nach einem der Ansprüche 1 bis 4 mit dem Antigen;
(b) Isolieren von Zellen oder Gewebe, die einen Antigenspezifischen Nur-Schwerkette-Antikörper von Interesse exprimieren, aus der Maus nach Affinitätsreifung;
(c) Klonieren des/der Schwerketten- und Leichtkettenlocus oder -loci aus mRNA, die von der/dem isolierten Zelle oder Gewebe abgeleitet ist; und
(d) Exprimieren des Antikörpers.

## Revendications

1. Souris transgénique comprenant :
(a) un locus de chaîne lourde d'immunoglobuline qui comprend la séquence de SEQ ID NO: 1 et une région de contrôle de locus apparentée (LCR), et
(b)(i) un locus de chaîne légère kappa d'immunoglobuline hétérologue comprenant des segments de gène Vκ humain, des segments de gène Jκ humain, et un segment de gène de région constante de rat, et un LCR apparenté, dans laquelle :
les segments de gène Vκ humain sont constitués de, dans l'ordre suivant de 5' à 3', Vκ2-30, Vκ2-28, Vκ1-5, Vκ1-9, Vκ1-27, Vκ1-33, Vκ1-39, Vκ3-20, Vκ3-15, Vκ3-11, et Vκ4-1,
les segments de gène Jκ humain comprennent l'ensemble des cinq segments de gène Jκ humain,
et le segment de gène de la région constante Cκ, et/ou
(b)(ii) un locus de chaîne légère lambda d'immunoglobuline hétérologue comprenant quatre segments de gène Vλ humain, deux segments de gène J humain, et
deux segments de gène de région constante de rat, et un LCR apparenté, dans laquelle :
les segments de gène Vλ humain comprennent, dans l'ordre suivant de 5' à 3', Vλ3-19, Vλ3-1, Vλ2-8, et Vλ1-51,
les segmente du gène J humain comprennent Jλ1 et Jλ3,
et les segments de gène de région constante comprennent Cλ2 et Cλ3,
et dans laquelle le locus de chaîne lourde de souris endogène et le locus de chaîne légère kappa de souris endogène ont été désactivés.

2. Souris transgénique selon la revendication 1, dans laquelle lesdits loci comprennent en outre un marqueur sélectionnable.

3. Souris transgénique selon la revendication 2, dans laquelle plus d'un transgène est présent, et chaque transgène comprend un marqueur sélectionnable différent.

4. Souris transgénique selon l'une quelconque des revendications 1 à 3, dans laquelle ledit locus de chaîne légère lambda d'immunoglobuline comprend la séquence de SEQ ID NO:3.

5. Procédé de production d'un anticorps monoclonal hybride hétérologue spécifique d'un antigène comprenant :
(a) immunisation d'une souris transgénique selon l'une quelconque des revendications 1 à 4 avec l'antigène ;
(b) préparation d'hybridomes ou de cellules B, de plasmablastes, de cellules B à mémoire ou de cellules plasmatiques produisant chacun un anticorps monoclonal à partir des cellules B de la souris transgénique immunisée ;
(c) facultativement la sélection d'au moins un hybridome ou d'une cellule B, d'un plasmablaste, d'une cellule B à mémoire ou d'une cellule plasmatique exprimant l'anticorps hétérologue par utilisation des gènes marqueurs sélectifs dominants présents dans les transgènes comprenant les loci de chaîne légère et de chaîne lourde hétérologue d'immunoglobuline ; et
(d) la sélection d'au moins un hybridome ou d'une cellule B, d'un plasmablaste, d'une cellule B à mémoire ou d'une cellule plasmatique qui produit un anticorps qui se lie spécifiquement à l'antigène.

6. Procédé de dérivation d'un anticorps humain à partir d'un anticorps hybride comprenant :
(a) la mise en œuvre du procédé selon la revendication 5 ;
(b) la sélection d'au moins un hybridome ou d'une cellule B, d'un plasmablaste, d'une cellule B à mémoire ou d'une cellule plasmatique qui produit un anticorps qui se lie spécifiquement à l'antigène et comprend des domaines de liaison V_{H} et V_{L} de l'espèce choisie ;
(c) le clonage et le séquençage des domaines V_{H} et V_{L} ;
(d) le reclonage de séquences sélectionnées comprenant les domaines de liaison V_{H} et V_{L} codant pour des séquences comportant des domaines effecteurs constants choisis parmi la même espèce ; et
(e) la co-expression des séquences reclonées codant pour des polypeptides de chaîne lourde et légère de l'espèce souhaitée en utilisant un vecteur d'expression dans un type de cellule choisi.

7. Procédé de production d'un anticorps monoclonal spécifique d'un antigène ayant une région variable humaine comprenant :
(a) immunisation d'une souris transgénique selon l'une quelconque des revendications 1 à 4 avec l'antigène ;
(b) préparation d'hybridomes à partir des cellules B de la souris transgénique immunisée après maturation d'affinité ; et
(c) isolement dudit anticorps à partir desdits hybridomes.

8. Procédé de production d'un anticorps monoclonal spécifique d'un antigène ayant une région variable humaine comprenant :
(a) immunisation d'une souris transgénique selon l'une quelconque des revendications 1 à 4 avec l'antigène ;
(b) isolement de cellules ou de tissu exprimant un anticorps seulement à chaîne lourde spécifique d'antigène d'intérêt à partir de ladite souris après maturation d'affinité ;
(c) clonage du locus ou des loci de chaîne lourde et légère à partir d'ARNm dérivé de la cellule ou du tissu isolé(e) ; et
(d) expression de l'anticorps.
